(19) Europäisches Patentamt
European Patent Office
Office européen des brevets

(11) **EP 2 067 501 A2**

(12) **EUROPEAN PATENT APPLICATION**

(43) Date of publication:
**10.06.2009 Bulletin 2009/24**

(51) Int Cl.:
**A61N 1/08** (2006.01)

(21) Application number: **08019275.0**

(22) Date of filing: **06.04.2007**

| | |
|---|---|
| (84) Designated Contracting States:<br>**AT BE BG CH CY CZ DE DK EE ES FI FR GB GR HU IE IS IT LI LT LU LV MC MT NL PL PT RO SE SI SK TR**<br>Designated Extension States:<br>**AL BA HR MK RS** | (71) Applicant: **Medtronic, Inc.**<br>**Minneapolis, MN 55432 (US)** |
| (30) Priority: **07.04.2006 US 744456 P**<br>**07.04.2006 US 744464 P**<br>**07.04.2006 US 744457 P**<br>**07.04.2006 US 744437 P**<br>**07.04.2006 US 744468 P**<br>**11.05.2006 US 747027 P**<br>**29.06.2006 US 806115 P** | (72) Inventors:<br>• **Gray, Robert, W.**<br>  **Rochester, NY 14620 (US)**<br>• **MacDonald, Stuart, G.**<br>  **Pultneyville, NY 14538 (US)** |
| (62) Document number(s) of the earlier application(s) in accordance with Art. 76 EPC:<br>**07760251.4 / 2 010 271** | (74) Representative: **Hughes, Andrea Michelle**<br>**Frank B. Dehn & Co.**<br>**St Bride's House**<br>**10 Salisbury Square**<br>**London**<br>**EC4Y 8JD (GB)** |

(54) **Resonance circuit for implantable devices and leads**

(57)     An implantable medical assist device includes a medical device. The medical device has a housing and electronics contained therein. A lead provides an electrical path to or from the electronics within the medical device. A resonance tuning module is located in the housing and is connected to the lead. The resonance tuning module includes a control circuit for determining a resonant frequency of the implantable medical assist device and an adjustable impedance circuit to change the combined resonant frequency of the medical device and lead. Also, a stent structure includes a stent formed of a plurality of rings to form a lumen and a plurality of struts. Each strut provides a connection between two adjacent rings. The stent structure also includes a conductive trace and a dielectric material. The conductive trace has a first end and a second end. The conductive trace is formed on a portion of a first ring, on a portion of a second ring, and on struts between the first ring and the second ring to form an inductive conductive trace loop. The first end of the conductive trace overlaps the second end of the conductive trace such that the dielectric material is located between the first end of the conductive trace and the second end of the conductive trace to form a capacitor.

*FIG. 4*

EP 2 067 501 A2

**Description**

## TECHNICAL FIELD

The present invention relates generally to a medical device that includes an anti-antenna device to prevent or significantly reduce damaging heat, created by currents or voltages induced by outside electromagnetic energy, to a tissue area. More particularly, the present invention is directed to a medical device that includes an anti-antenna device to prevent or significantly reduce damaging heat, created by currents or voltages induced by magnetic-resonance imaging, to a tissue area.

The present invention is further directed to a device for protecting a patient, physician, and/or electronic components in an electrical device implanted or partially implanted within the patient. More particularly, the present invention is directed to a device for reducing or substantially eliminating current and/or voltage surges induced by magnetic resonance imaging systems' oscillating magnetic fields.

The present invention is also directed to a circuit on a medical device. More particularly, the present invention is directed to a circuit that is a resonator or a phase shifter for magnetic resonance imaging inside stents and visual markers on catheters.

## BACKGROUND ART

Magnetic resonance imaging has been developed as an imaging technique adapted to obtain both images of anatomical features of human patients as well as some aspects of the functional activities of biological tissue. These images have medical diagnostic value in determining the state of the health of the tissue examined. Unlike the situation with fluoroscopic imaging, a patient undergoing magnetic resonance imaging procedure may remain in the active imaging system for a significant amount of time, e.g. a half-hour or more, without suffering any adverse effects.

In a magnetic-resonance imaging process, a patient is typically aligned to place the portion of the patient's anatomy to be examined in the imaging volume of the magnetic-resonance imaging apparatus. Such a magnetic-resonance imaging apparatus typically comprises a primary magnet for supplying a constant magnetic field ($B_0$) which, by convention, is along the z-axis and is substantially homogeneous over the imaging volume and secondary magnets that can provide linear magnetic field gradients along each of three principal Cartesian axes in space (generally x, y, and z, or $X_1$, $X_2$ and $X_3$, respectively).

A magnetic field gradient ($\Delta B_0/\Delta x_i$) refers to the variation of the field along the direction parallel to $B_0$ with respect to each of the three principal Cartesian axes, $x_i$. The apparatus also comprises one or more radio-frequency coils, which provide excitation signals to the patient's body, placed in the imaging volume in the form of a pulsed rotating magnetic field. This field is commonly referred to as the scanner's "B1" field and as the scanner's "RF" or "radio-frequency" field. The frequency of the excitation signals is the frequency at which this magnetic field rotates. These coils may also be used for detection of the excited patient's body material magnetic-resonance imaging response signals.

The magnetic resonance imaging apparatus also comprises one or more radio frequency coils that provide excitation and detection of the magnetic resonance imaging induced signals in the patient's body.

The gradient fields are switched ON and OFF at different rates depending on the magnetic resonance imaging scan sequence used. In some cases, this may result in a changing magnetic field on the order of dB/dt = 50 T/s. The frequency that a gradient field may be turned ON can be between 200 Hz to about 300 kHz.

For a single loop with a fixed area, Lenz's law can be stated as:

$$\text{EMF} = -\mathbf{A} \bullet d\mathbf{B}/dt$$

where $\mathbf{A}$ is the area vector, $\mathbf{B}$ is the magnetic field vector, and "•" is the vector scalar product. This equation indicates that an electro-motive-force (EMF) is developed in any loop that encircles a changing magnetic field.

In a magnetic resonance imaging system, there is applied to the biological sample (patient) a switched gradient field in all 3 coordinate directions (x-, y-, z-directions). If the patient has an implanted heart pacemaker (or other implanted devices having conductive components) the switched gradient magnetic fields (an alternating magnetic field) may cause erroneous signals to be induced/generated in a sensing lead or device or circuit; damage to electronics; and/or harmful stimulation of tissue, e.g. heart muscle, nerves, etc.

The use of the magnetic-resonance imaging process with patients who have implanted medical assist devices; such as cardiac assist devices or implanted insulin pumps; often presents problems. As is known to those skilled in the art, implantable devices (such as implantable pulse generators and cardioverter/defibrillator/pacemakers) are sensitive to a variety of forms of electromagnetic interference because these enumerated devices include sensing and logic systems that respond to low-level electrical signals emanating from the monitored tissue region of the patient. Since the sensing systems and conductive elements of these implantable devices are responsive to changes in local electromagnetic fields, the implanted devices are vulnerable to external sources of severe electromagnetic noise, and in particular, to electromagnetic fields emitted during the magnetic resonance imaging procedure. Thus, patients with implantable devices are generally advised not to undergo magnetic resonance imaging procedures.

The human heart may suffer from two classes of rhythmic disorders or arrhythmias: bradycardia and tachyarrhythmia. Bradycardia occurs when the heart beats too slowly, and may be treated by a common implantable pacemaker delivering low voltage (about 3 Volts) pacing pulses.

The common implantable pacemaker is usually contained within a hermetically sealed enclosure, in order to protect the operational components of the device from the harsh environment of the body, as well as to protect the body from the device.

The common implantable pacemaker operates in conjunction with one or more electrically conductive leads, adapted to conduct electrical stimulating pulses to sites within the patient's heart, and to communicate sensed signals from those sites back to the implanted device.

Furthermore, the common implantable pacemaker typically has a metal case and a connector block mounted to the metal case that includes receptacles for leads which may be used for electrical stimulation or which may be used for sensing of physiological signals. The battery and the circuitry associated with the common implantable pacemaker are hermetically sealed within the case. Electrical interfaces are employed to connect the leads outside the metal case with the medical device circuitry and the battery inside the metal case.

Electrical interfaces serve the purpose of providing an electrical circuit path extending from the interior of a hermetically sealed metal case to an external point outside the case while maintaining the hermetic seal of the case. A conductive path is provided through the interface by a conductive pin that is electrically insulated from the case itself.

Such interfaces typically include a ferrule that permits attachment of the interface to the case, the conductive pin, and a hermetic glass or ceramic seal that supports the pin within the ferrule and isolates the pin from the metal case.

A common implantable pacemaker can, under some circumstances, be susceptible to electrical interference such that the desired functionality of the pacemaker is impaired. For example, common implantable pacemaker requires protection against electrical interference from electromagnetic interference or insult, defibrillation pulses, electrostatic discharge, or other generally large voltages or currents generated by other devices external to the medical device. As noted above, more recently, it has become crucial that cardiac assist systems be protected from magnetic-resonance imaging sources.

Such electrical interference can damage the circuitry of the cardiac assist systems or cause interference in the proper operation or functionality of the cardiac assist systems. For example, damage may occur due to high voltages or excessive currents introduced into the cardiac assist system.

Therefore, it is required that such voltages and currents be limited at the input of such cardiac assist systems, e.g., at the interface. Protection from such voltages and currents has typically been provided at the input of a cardiac assist system by the use of one or more zener diodes and one or more filter capacitors.

For example, one or more zener diodes may be connected between the circuitry to be protected, e.g., pacemaker circuitry, and the metal case of the medical device in a manner, which grounds voltage surges and current surges through the diode(s). Such zener diodes and capacitors used for such applications may be in the form of discrete components mounted relative to circuitry at the input of a connector block where various leads are connected to the implantable medical device, e.g., at the interfaces for such leads.

However, such protection, provided by zener diodes and capacitors placed at the input of the medical device, increases the congestion of the medical device circuits, at least one zener diode and one capacitor per input/output connection or interface. This is contrary to the desire for increased miniaturization of implantable medical devices.

Further, when such protection is provided, interconnect wire length for connecting such protection circuitry and pins of the interfaces to the medical device circuitry that performs desired functions for the medical device tends to be undesirably long. The excessive wire length may lead to signal loss and undesirable inductive effects. The wire length can also act as an antenna that conducts undesirable electrical interference signals to sensitive CMOS circuits within the medical device to be protected.

Additionally, the radio-frequency energy that is inductively coupled into the wire causes intense heating along the length of the wire, and at the electrodes that are attached to the heart wall. This heating may be sufficient to ablate the interior surface of the blood vessel through which the wire lead is placed, and may be sufficient to cause scarring at the point where the electrodes contact the heart. A further result of this ablation and scarring is that the sensitive node that the electrode is intended to pace with low voltage signals becomes desensitized, so that pacing the patient's heart becomes less reliable, and in some cases fails altogether.

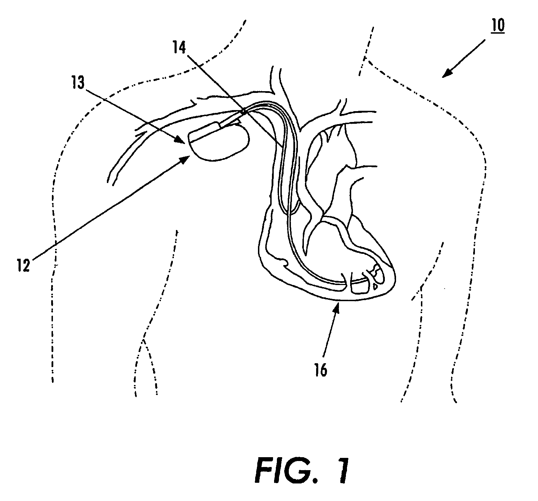

Another conventional solution for protecting the implantable medical device from electromagnetic interference is illustrated in Figure 1. Figure 1 is a schematic view of an implantable medical device **12** embodying protection against electrical interference. At least one lead **14** is connected to the implantable medical device 12 in connector block region **13** using an interface.

In the case where implantable medical device **12** is a pacemaker implanted in a body **10,** the pacemaker **12** includes at least one or both of pacing and sensing leads represented generally as leads **14** to sense electrical signals attendant to the depolarization and repolarization of the heart **16,** and to provide pacing pulses for causing depolarization of cardiac tissue in the vicinity of the distal ends thereof.

Conventionally protection circuitry is provided using a diode array component. The diode array conventionally consists of five zener diode triggered semiconductor controlled rectifiers with anti-parallel diodes arranged in an array with one common connection. This allows for a small footprint despite the large currents that may be carried through the device during defibrillation, e.g., 10 amps. The semiconductor controlled rectifiers turn ON and limit the voltage across the device when excessive voltage and current surges occur.

Each of the zener diode triggered semiconductor controlled rectifier is connected to an electrically conductive pin. Further, each electrically conductive pin is connected to a medical device contact region to be wire bonded to pads of a printed circuit board. The diode array component is connected to the electrically conductive pins via the die contact regions along with other electrical conductive traces of the printed circuit board.

Other attempts have been made to protect implantable devices from magnetic-resonance imaging fields. For example, United States Patent 5,968,083 describes a device adapted to switch between low and high impedance modes of operation in response to electromagnetic interference or insult. Furthermore, United States Patent 6,188,926 discloses a control unit for adjusting a cardiac pacing rate of a pacing unit to an interference backup rate when heart activity cannot be sensed due to electromagnetic interference or insult.

Although, conventional medical devices provide some means for protection against electromagnetic interference, these conventional devices require much circuitry and fail to provide fail-safe protection against radiation produced by magnetic-resonance imaging procedures. Moreover, the conventional devices fail to address the possible damage that can be done at the tissue interface due to radio-frequency induced heating, and the conventional devices fail to address the unwanted heart stimulation that may result from radio-frequency induced electrical currents.

Thus, it is desirable to provide devices that prevent the possible damage that can be done at the tissue interface due to induced electrical signals that may cause thermally-related tissue damage.

Moreover, problems are realized when the placement of the implant is next to particular organs. For example, when a pacemaker is placed in the upper chest and the lead tip is placed into the heart, a loop (an electrical loop) is created. A changing magnetic field (the switched gradient field) over the area of the loop (through the area of the loop) will cause an induced voltage (and current) across the heart. This induced voltage (current) can stimulate the heart inappropriately and can cause heart damage or death.

Therefore, it is desirable to provide a medical device or system that reduces or eliminates the undesirable effects of changing magnetic fields from a magnetic resonance imaging system on the medical devices and/or patients undergoing medical procedures or that have temporary or permanent implanted materials and/or devices with conducting components.

On the other hand, stents have been implanted in vessels, ducts, or channels of the human body to act as a scaffolding to maintain the patency of the vessel, duct, or channel lumen. A drawback of stenting is the body's natural defensive reaction to the implant of a foreign object. In many patients, the reaction is characterized by a traumatic proliferation of tissue as intimal hyperplasia at the implant site, and, where the stent is implanted in a blood vessel such as a coronary artery, formation of thrombi which become attached to the stent.

Each of these adverse effects contributes to restenosis--a re-narrowing of the vessel lumen--to compromise the improvements that resulted from the initial reopening of the lumen by implanting the stent. Consequently, a great number of stent implant patients must undergo another angiogram, on average about six months after the original implant procedure, to determine the status of tissue proliferation and thrombosis in the affected lumen. If re-narrowing has occurred, one or more additional procedures are required to stem or reverse its advancement.

Due to the drawbacks mentioned above, the patency of the vessel lumen and the extent of tissue growth within the lumen of the stent need to be examined and analyzed, and the blood flow therethrough needs to be measured, from time to time, as part of the patient's routine post-procedure examinations.

Current techniques employed magnetic resonance imaging (magnetic resonance imaging) to visualize internal features of the body if there is no magnetic resonance distortion. However, using magnetic resonance imaging techniques to visualize implanted stents composed of ferromagnetic or electrically conductive materials is difficult because these materials cause sufficient distortion or diminution of the magnetic resonance fields to preclude imaging the interior of the stent. This effect is attributable to their magnetic susceptibility and Faradaic physical properties in relation to the electromagnetic energy applied during the magnetic resonance imaging process.

One conventional solution to this problem is to design a stent that includes a mechanically supportive tubular structure composed primarily of metal having relatively low magnetic susceptibility, and electrically conductive layers overlying a portion of the surface of the tubular structure to enhance properties of the stent for magnetic resonance imaging of the interior of the lumen of the stent when implanted in the body. An electrically insulative layer resides between the surface of the tubular structure of the stent and the electrically conductive layers.

The tubular structure with overlying electrically conductive layers and electrically insulative layer sandwiched therebetween are arranged in a composite relationship to form an RLC circuit. The RLC circuit may either resonant at or near the desired frequency of magnetic resonance imaging frequency or produce a magnetic field approximately 180˚ out of phase to the induced current in the conductive stent, thereby substantially reducing the Faraday shielding effect of the conductive stent alone.

At least a portion of the electrically conductive layers has a geometric formation arranged on the tubular scaffolding of the stent to function as an electrical inductance element, an electrical capacitance element, and an electrical resistive element.

Therefore, it is desirable to provide a device which enables imaging and visualization of the inner lumen of an implanted stent by means of a magnetic resonance imaging technique.

## BRIEF DESCRIPTION OF THE DRAWINGS

The present invention may take form in various components and arrangements of components, and in various steps and arrangements of steps. The drawings are only for purposes of illustrating a preferred embodiment and are not to be construed as limiting the present invention, wherein:

Figure 1 is an illustration of conventional cardiac assist device;

Figure 2 shows a conventional bipolar pacing lead circuit representation;

Figure 3 is a graph illustrating the magnitude of the current, induced by magnetic-resonance imaging, flowing through the tissue at a distal end of a medical device using the bipolar pacing lead circuit of Figure 2;

Figure 4 shows a bipolar pacing lead circuit representation according to some or all of the concepts of the present invention;

Figure 5 is a graph illustrating the magnitude of the current, induced by magnetic-resonance imaging, flowing through the tissue at a distal end of a medical device using the bipolar pacing lead circuit of Figure 4;

Figure 6 is a graph illustrating the magnitude of the current, low-frequency pacing or defibrillation signals, flowing through the circuit of a medical device using the bipolar pacing lead circuit of Figure 4;

Figure 7 shows another bipolar pacing lead circuit representation according to some or all of the concepts of the present invention;

Figure 8 is a graph illustrating the magnitude of the current, induced by 64 MHz magnetic-resonance imaging, flowing through the tissue at a distal end of a medical device using the bipolar pacing lead circuit of Figure 7;

Figure 9 is a graph illustrating the magnitude of the current, induced by 128 MHz magnetic-resonance imaging, flowing through the tissue at a distal end of a medical device using the bipolar pacing lead circuit of Figure 7;

Figure 10 shows another bipolar pacing lead circuit representation according to some or all of the concepts of the present invention;

Figure 11 is a graph illustrating the magnitude of the current, induced by magnetic-resonance imaging, flowing through the tissue at a distal end of a medical device using the bipolar pacing lead circuit of Figure 10;

Figure 12 shows another bipolar pacing lead circuit representation according to some or all of the concepts of the present invention;

Figure 13 is a graph illustrating the magnitude of the current, induced by magnetic-resonance imaging, flowing through the tissue at a distal end of a medical device using the bipolar pacing lead circuit of Figure 12;

Figure 14 shows another bipolar pacing lead circuit representation according to some or all of the concepts of the present invention;

Figure 15 is a graph illustrating the magnitude of the current, induced by magnetic-resonance imaging, flowing through the tissue at a distal end of a medical device using the bipolar pacing lead circuit of Figure 14;

Figure 16 is a graph illustrating the magnitude of the current, induced by magnetic-resonance imaging, flowing through the tissue at a distal end of a medical device using the bipolar pacing lead circuit of Figure 14 using an increased resistance in the resonant circuit;

Figure 17 is a graph illustrating the magnitude of the current, induced by magnetic-resonance imaging, flowing through the tissue at a distal end of a medical device using a conventional bipolar pacing lead circuit;

Figure 18 is a graph illustrating the magnitude of the current, induced by magnetic-resonance imaging, flowing through the tissue at a distal end of a medical device using the bipolar pacing lead circuit with a resonant circuit in one lead;

Figure 19 is a graph illustrating the magnitude of the current, induced by magnetic-resonance imaging, flowing through the tissue at a distal end of a medical device using the bipolar pacing lead circuit with a resonant circuit in both leads;

Figure 20 is a graph illustrating the magnitude of the current, induced by magnetic-resonance imaging, flowing through the tissue at a distal end of a medical device using the bipolar pacing lead circuit with a resonant circuit in both leads and increased inductance;

Figure 21 is a graph illustrating the magnitude of the current, induced by magnetic-resonance imaging, flowing through the tissue at a distal end of a medical device using the bipolar pacing lead circuit with a resonant circuit in both leads and decreased inductance;

Figure 22 shows a bipolar pacing lead adaptor with a single resonant circuit according to some or all of the concepts of the present invention;

Figure 23 illustrates a resonant circuit for a bipolar pacing lead according to some or all of the concepts of the present invention;

Figure 24 is a graph illustrating the temperature at a distal end of a medical device;

Figure 25 is a graph illustrating the magnitude of the current, induced by magnetic-resonance imaging, flowing through the tissue at a distal end of a conventional medical device;

Figure 26 is a graph illustrating the magnitude of the current, induced by magnetic-resonance imaging, flowing through the tissue at a distal end of a medical device with a resonant circuit, according to the concepts of the present invention, at the proximal end thereof;

Figure 27 is a graph illustrating the temperature at a distal end of a medical device with a resonant circuit, according to the concepts of the present invention, at the distal end thereof;

Figure 28 is a graph illustrating the temperature at a distal end of a medical device with a resonant circuit, according to the concepts of the present invention, at the proximal end thereof;

Figure 29 shows another bipolar pacing lead circuit representation according to some or all of the concepts of the present invention;

Figure 30 shows a bipolar pacing device according to some or all of the concepts of the present invention;

Figure 31 is a graph illustrating the temperature at a distal end of a conventional pacing lead;

Figure 32 is a graph illustrating the temperature at a distal end of the bipolar pacing device of Figure 30 with closed windings;

Figure 33 is a graph illustrating the temperature at a distal end of the bipolar pacing device of Figure 30 with medium closed windings;

Figure 34 is a graph illustrating the temperature at a distal end of the bipolar pacing device of Figure 30 with open pitch windings;

Figure 35 is a graph illustrating the temperature at a distal end of the bipolar pacing device of Figure 30 with different states of windings;

Figure 36 shows a resonance tuning module as an integral subsystem in a device such as a pacemaker according to some or all of the concepts of the present invention;

Figure 37 shows a resonance tuning module as a secondary module that may be interposed between the device and its associated leads or leads according to some or all of the concepts of the present invention;

Figure 38 illustrates a conventional electronic device that includes a connection port, a circuit, and an electrical line;

Figure 39 illustrates an insertion of resonant circuit adaptor suitable for connecting to connector and connecting to connection port which places a resonant circuit in series with electrical wire and circuit;

Figure 40 illustrates another electronic device that includes a connection port and a circuit;

Figure 41 illustrates an electronic device having a circuit, a connection port, and a switch;

Figure 42 illustrates components of an RLC resonance circuit connected to an electrode;

Figures 43-45 illustrate embodiments of a cylinder shaped component implemented with a resonance circuit;

Figure 46 illustrates a plurality of cylinder shaped components positioned along a medical device;

Figure 47 illustrates a cross-sectional view of an embodiment of a cylinder shaped component implemented with a resonance circuit;

Figure 48 illustrates a cross-sectional view of another embodiment of a cylinder shaped component implemented with a resonance circuit;

Figure 49 illustrates a lead with an RLC circuit at distal tip tuned to desired resonant frequency;

Figure 50 illustrates a lead with multiple RLC circuits at distal end of lead;

Figure 51 illustrates a lead with multiple RLC circuits positioned along the leads length;

Figure 52 illustrates a diode is placed at the distal end of the lead;

Figure 53 illustrates an RLC circuit, as illustrated in Figure 49, wherein two diodes are connected back to back across an inductor;

Figure 54 illustrates an RLC circuit, as illustrated in Figure 49, wherein two diodes are connected back to back across a capacitor;

Figure 55 illustrates the impedance of a coiled wire of the lead being divided into two sections;

Figure 56 is a schematic of an implanted pacemaker arrangement in a body;

Figure 57 is a schematic of a pacemaker lead comprising three conductive strands;

Figure 58 is a schematic of a sensing system used with a pacemaker;

Figure 59 illustrates an embodiment of a pacemaker canister according to the concepts of the present invention;

Figure 60 illustrates another embodiment of a pacemaker canister according to the concepts of the present invention;

Figure 61 illustrates a further embodiment of a pacemaker canister according to the concepts of the present invention;

Figure 62 is an illustration of inductive currents in conductor loops;

Figure 63 is an illustration of canceling inductive currents in conductor loops according to the concepts of the present invention;

Figure 64 is a schematic of an embodiment of a pacemaker lead utilizing inductive loops according to the concepts of the present invention;

Figure 65 is a schematic of an embodiment of inductive loops in a pacemaker canister according to the concepts of the present invention;

Figure 66 is a schematic of an embodiment of inductive loops with a pacemaker canister according to the concepts of the present invention;

Figure 67 illustrates of an embodiment of a medical device with an external voltage cancellation unit according to the concepts of the present invention;

Figure 68 illustrates of another embodiment of a medical device with an external voltage cancellation unit according to the concepts of the present invention;

Figure 69 illustrates a portion of coiled leads used in a medial device according to the concepts of the present invention;

Figure 70 illustrates another embodiment of a portion of coiled leads used in a medial device according to the concepts of the present invention;

Figure 71 illustrates a further embodiment of a portion of coiled leads used in a medial device according to the concepts of the present invention;

Figure 72 illustrates another embodiment of a portion of coiled leads used in a medial device according to the concepts of the present invention;

Figure 73 illustrates a circuit diagram representing a guide wire with an unbalancing impedance circuit according to the concepts of the present invention;

Figure 74 illustrates another embodiment of a circuit diagram representing a guide wire with an unbalancing impedance circuit according to the concepts of the present invention;

Figure 75 illustrates a balun used in conjunction with a guide wire according to the concepts of the present invention;

Figure 76 is a circuit diagram representing a capacitance unbalanced balun unit according to the concepts of the present invention;

Figure 77 illustrates another embodiment of a portion of coiled leads used in a medial device according to the concepts of the present invention;

Figures 78 through 81 illustrate embodiments of a portion of coiled wires of a multi-wire medical lead according to the concepts of the present invention;

Figure 82 illustrates a pacing system having a lead and a pulse generator according to the concepts of the present invention;

Figure 83 illustrates a bipolar pacing system in magnetic resonance imaging scanner environment according to the concepts of the present invention;

Figure 84 illustrates a pacing system with a lead including two conductors with a low pass filter being implemented by the insertion of a capacitor between the two conductors according to the concepts of the present invention;

Figure 85 illustrates a pacing system comprising a lead, a pulse generator, and a voltage compensation component according to the concepts of the present invention;

Figure 86 illustrates another implementation of a voltage compensation device utilized with a lead in which an additional conductor is inserted into the lead together with the two conductors according to the concepts of the present invention;

Figure 87 illustrates another implementation of a voltage compensation device according to the concepts of the present invention;

Figure 88 illustrates a coiled conductive wire according to the concepts of the present invention;

Figure 89 illustrates a coiled conductive wire according to the concepts of the present invention;

Figure 90 shows a stent structure;

Figure 91 shows an circuit with inductor path along some of the stent's struts;

Figures 92-95 show various inductor paths around a stent;

Figure 96 shows the formation of a capacitor element on a portion of a stent strut;

Figure 97 shows a circuit on a medical device;

Figure 98 shows a schematic circuit model of a stent with an RLC circuit in a magnetic resonance imaging scanner;

Figure 99 is a frequency sweep plot of induced summed currents; and

Figure 100 is a frequency sweep plot of induced summed currents when the RLC circuit is tuned according to this invention.

## BEST MODE FOR CARRYING OUT THE INVENTION

As noted above, a medical device includes an anti-antenna device to prevent or significantly reduce damaging heat, created by currents or voltages induced by outside electromagnetic energy (namely magnetic-resonance imaging), to a tissue area.

More specifically, the present invention is directed to a medical device that includes anti-antenna device, which significantly reduces the induced current on the "signal" wire of a pacing lead when the pacing lead is subjected to the excitation signal's frequency of a magnetic-resonance imaging scanner without significantly altering a low frequency pacing signal. The low frequency pacing signal may be generated by an implantable pulse generator or other pulse generator source outside the body.

To provide an anti-antenna device, in one embodiment of the present invention utilizes a resonant circuit or circuits in line with a lead. The lead may be a signal wire of the pacing lead. Although the following descriptions of the various embodiments of the present invention, as well as the attached claims may utilize, the term pacing lead or lead, the term pacing lead or lead may generically refer to a unipolar pacing lead having one conductor; a bipolar pacing lead having two conductors; an implantable cardiac defibrillator lead; a deep brain stimulating lead having multiple conductors; a nerve stimulating lead; and/or any other medical lead used to deliver an electrical signal to or from a tissue area of a body. The resonant circuit or circuits provide a blocking quality with respect to the currents induced by the excitation signal's frequency of the magnetic-resonance imaging scanner. The excitation signal's frequency of the magnetic-resonance imaging scanner is commonly defined as the rotational frequency of the scanner's excitation magnetic field, commonly known as the scanner's B1 field.

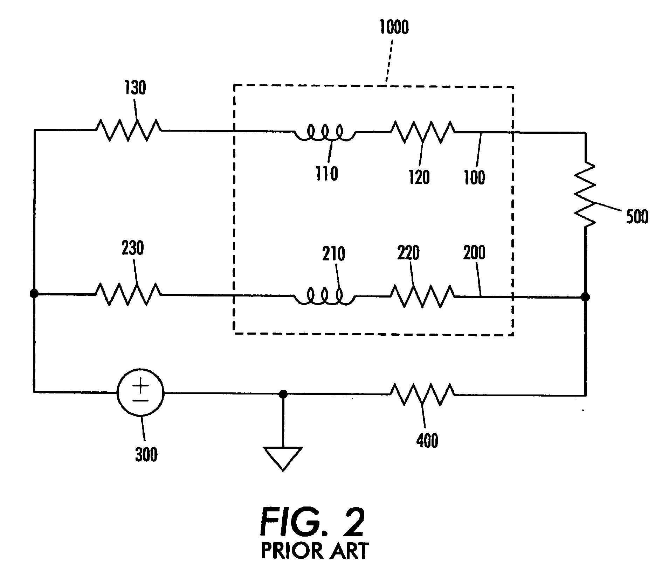

Figure 2 provides a conventional circuit representation of a bipolar pacing lead. As illustrated in Figure 2, the bipolar pacing lead **1000** includes two leads (**100** and **200**). A first pacing lead **100** includes resistance and inductance represented by a first resistor **120** and a first inductor **110,** respectively. A second pacing lead **200** includes resistance and inductance represented by a second resistor **220** and a second inductor **210,** respectively. At a distal end of each lead, the leads (**100** and **200**) come in contact with tissue.

As illustrated in Figure 2, the circuit paths from the distal ends of the leads (**100** and **200**) include a first tissue resistance, represented by first tissue modeled resistor **130,** and a second tissue resistance, represented by second tissue modeled resistor **230.**

The conventional circuit representation of a bipolar pacing lead, as illustrated in Figure 2, further includes a voltage source **300** that represents the induced electromagnetic energy (voltage or current) from magnetic resonance imaging, a body modeled resistor **400** that represents the resistance of the body, and a differential resistor **500** that represents a resistance between the leads.

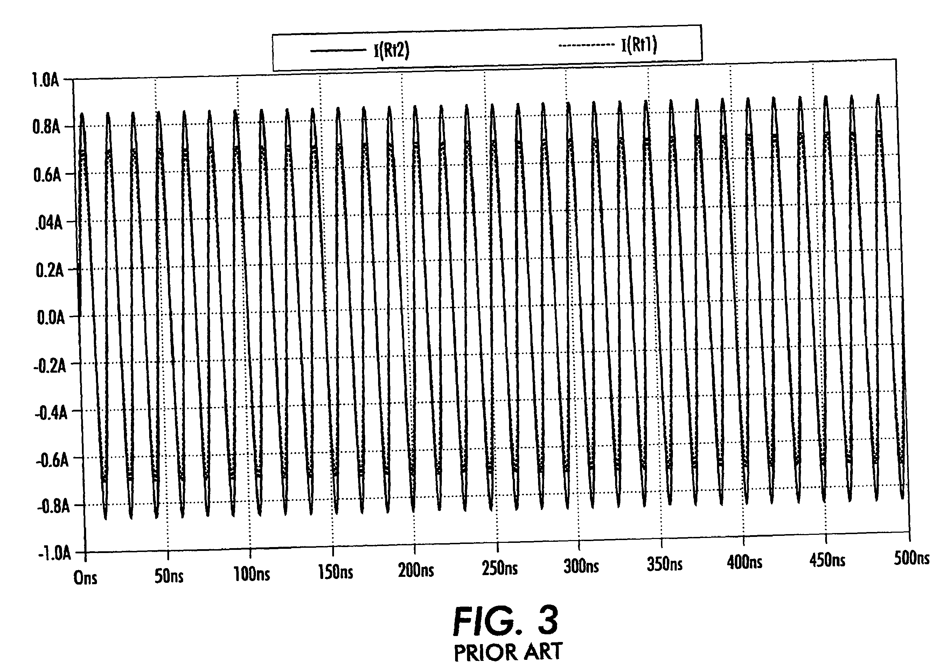

In Figure 3, it is assumed that the bipolar pacing leads of Figure 2 are subjected to a 64 MHz magnetic resonance imaging environment. As demonstrated in Figure 3, the current induced by the 64 MHz magnetic resonance imaging environment and flowing through the tissue at the distal end of the bipolar pacing leads can have a magnitude between 0.85 and -0.85 amps. This magnitude of current (**IRt2,** which represents the current flowing through first tissue modeled resistors **130** and **IRt1,** which represents the current flowing through second tissue modeled resistors **230**) at the distal end of the bipolar pacing leads can lead to serious damage to the tissue due to heat generated by the current flowing to the tissue.

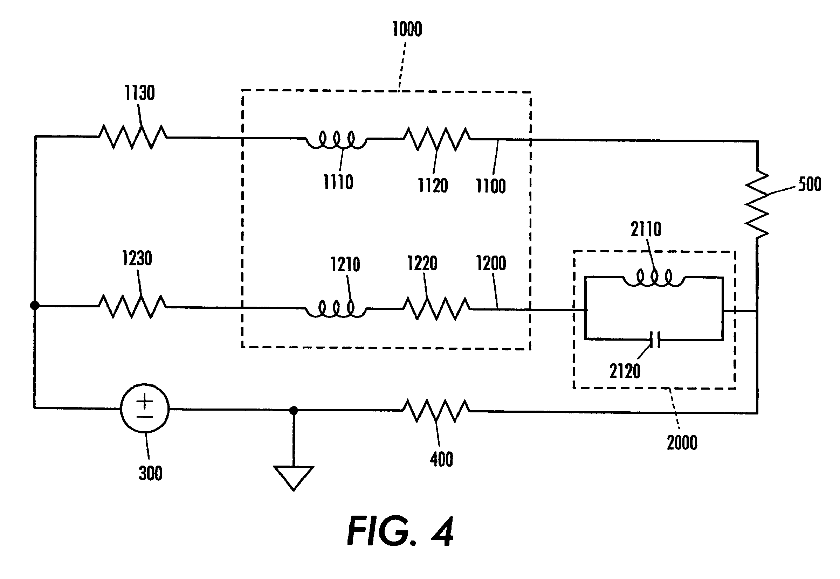

To reduce the heat generated by the induced current in the tissue, Figure 4 provides a circuit representation of a bipolar pacing lead according to the concepts of the present invention. As illustrated in Figure 4, the bipolar pacing lead **1000** includes two leads (**1100** and **1200**). A first pacing lead **1100** includes resistance and inductance represented by a first resistor **1120** and a first inductor **1110,** respectively. A second pacing lead **1200** includes resistance and inductance represented by a second resistor **1220** and a second inductor **1210,** respectively. At a distal end of each lead, the leads (**1100** and **1200**) come in contact with tissue.

As illustrated in Figure 4, the circuit paths from the distal ends of the leads (**1100** and **1200**) include a first tissue resistance, represented by first tissue modeled resistor **1130,** and a second tissue resistance, represented by second tissue modeled resistor **1230.**

The circuit representation of a bipolar pacing lead, as illustrated in Figure 4, further includes a voltage source **300** that represents the induced electromagnetic energy (voltage or current) from magnetic resonance imaging, a body modeled resistor **400** that represents the resistance of the body, and a differential resistor **500** that represents a resistance between the leads.

In addition to the elements discussed above, the circuit representation of a bipolar pacing lead, as illustrated in Figure 4, includes a resonant circuit **2000** in series or inline with one of the pacing leads, namely the second lead **1200.** The resonant circuit **2000** includes a LC circuit having an inductor **2110** in parallel to a capacitor **2120.** The resonant circuit **2000,** together with the second lead **1200,** acts as an anti-antenna device, thereby reducing the magnitude of the current induced through the tissue at the distal end of the pacing leads (**1100** and **1200**).

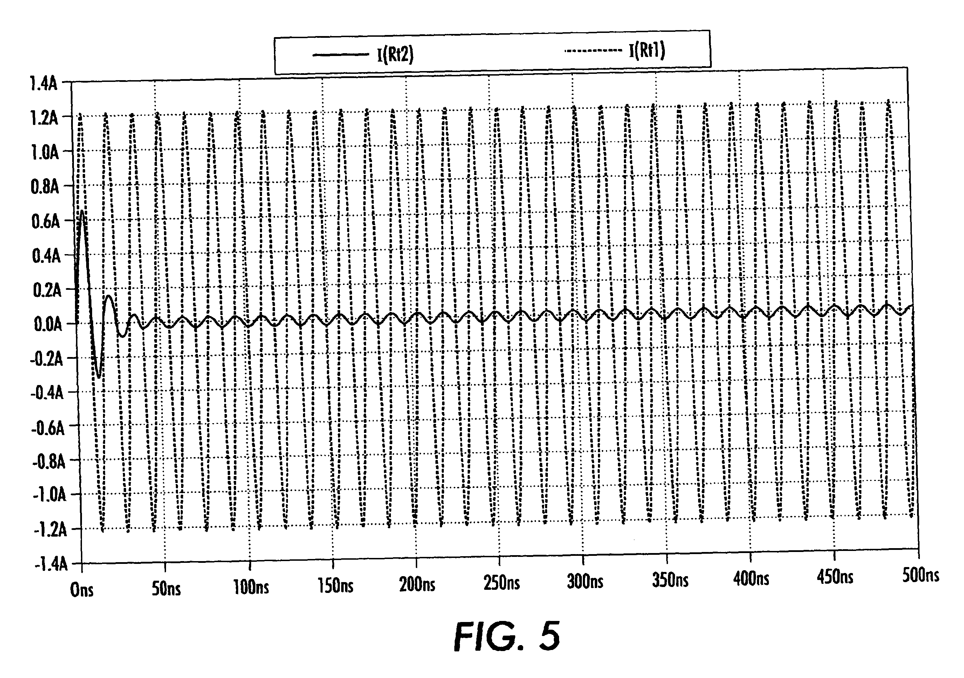

In Figure 5, it is assumed that the bipolar pacing leads of Figure 4 are subjected to a 64 MHz magnetic resonance imaging environment. As demonstrated in Figure 5, the current (**IRt2,** which represents the current flowing through tissue modeled resistor **1230** of Figure 4) induced by the 64 MHz magnetic resonance imaging environment and flowing through the tissue at the distal end of the second bipolar pacing lead **1200** can be greatly reduced. It is noted that the current (**IRt1,** which represents the current flowing through tissue modeled resistor **1130** of Figure 4) induced by the 64 MHz magnetic resonance imaging environment and flowing through the tissue at the distal end of the second bipolar pacing lead **1100,** according to this simulated model, can have a magnitude between 1.21 and -1.21 amps. This reduced magnitude of current (**IRt2,** which represents the current flowing through tissue modeled resistor **1230** of Figure 4) at the distal end of the bipolar pacing lead can significantly reduce the damage to the tissue due to heat generated by the current flowing to the tissue.

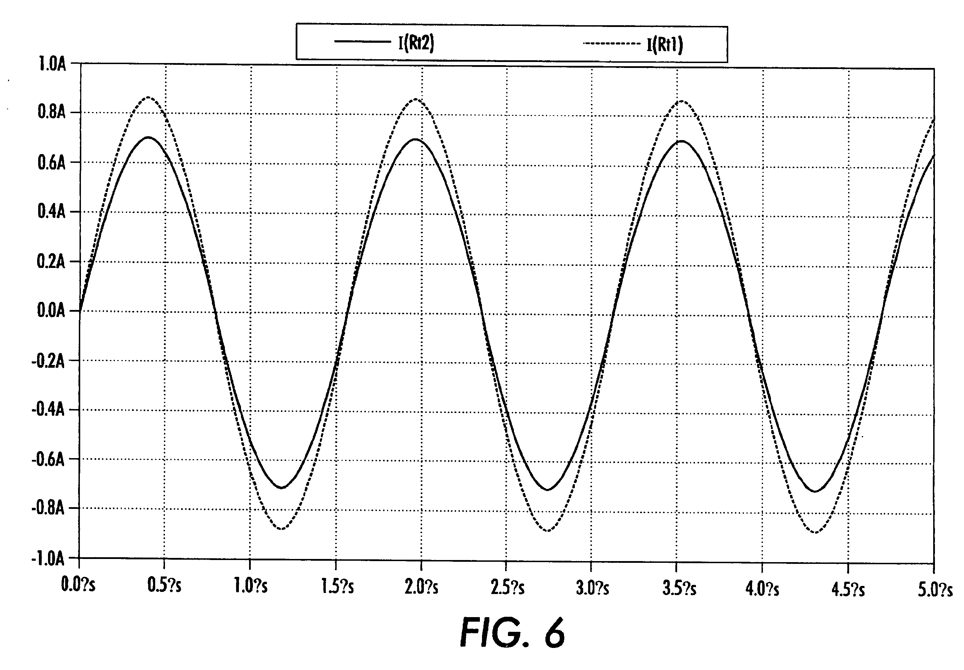

Notwithstanding the inclusion of the resonant circuit **2000,** the bipolar pacing leads can still provide an efficient pathway for the pacing signals, as illustrated by Figure 6. As can be seen when compared to Figure 3, the current magnitudes shown in Figure 6 through tissue resistors **1130** and **1230,** shown in Figure 4, are approximately the same as the magnitudes of the currents passing through tissue resistors **130** and **230,** shown in Figure 2. Thus, with the resonant circuit **2000** inserted into the circuit of Figure 4, the low frequency pacing signals are not significantly altered.

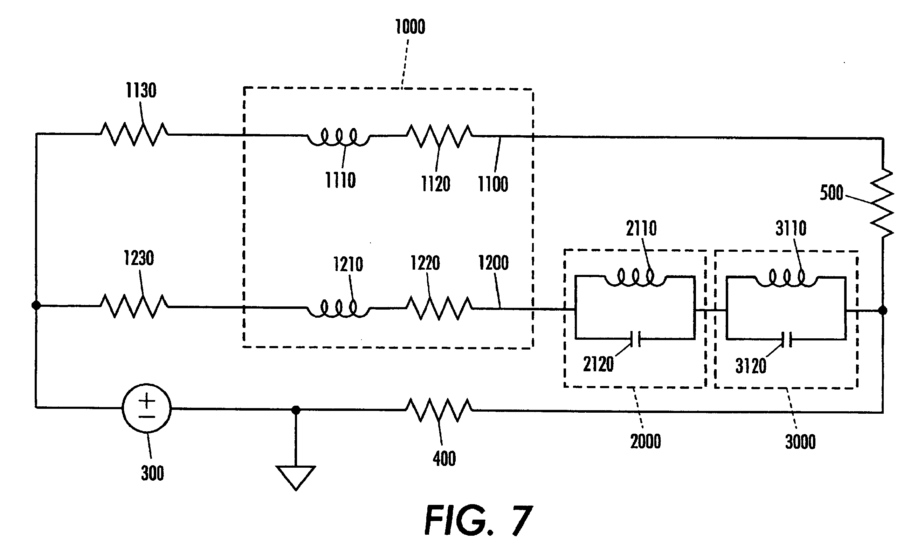

To provide a further reduction of the heat generated by the induced current in the tissue, Figure 7 provides a circuit representation of a bipolar pacing lead according to the concepts of the present invention. As illustrated in Figure 7, the bipolar pacing lead **1000** includes two leads (**1100** and **1200**). A first pacing lead **1100** includes resistance and inductance represented by a first resistor **1120** and a first inductor **1110,** respectively. A second pacing lead **1200** includes resistance and inductance represented by a second resistor **1220** and a second inductor **1210,** respectively. At a distal end of each lead, the leads (**1100** and **1200**) come in contact with tissue.

As illustrated in Figure 7, the circuit paths from the distal ends of the leads (**1100** and **1200**) include a first tissue resistance, represented by first tissue modeled resistor **1130,** and a second tissue resistance, represented by second tissue modeled resistor **1230.**

The circuit representation of a bipolar pacing lead, as illustrated in Figure 7, further includes a voltage source **300** that represents the induced electromagnetic energy (voltage or current) from magnetic resonance imaging, a body resistor **400** that represents the resistance of the body, and a differential resistor **500** that represents a resistance between the leads.

In addition to the elements discussed above, the circuit representation of a bipolar pacing lead, as illustrated in Figure 7, includes two resonant circuits (**2000** and **3000**) in series or inline with one of the pacing leads, namely the second lead **1200.** The first resonant circuit **2000** includes a LC circuit, tuned to about 64 MHz, having an inductor **2110** in parallel to a capacitor **2120.** The second resonant circuit **3000** includes a LC circuit, tuned to about 128 MHz, having an inductor **3110** in parallel to a capacitor **3120.**

The lead **1200** together with the inline resonant circuits (**2000** and **3000**) act as an anti-antenna device, thereby reducing the magnitude of the current induced through the tissue at the distal end of the pacing lead (**1200**).

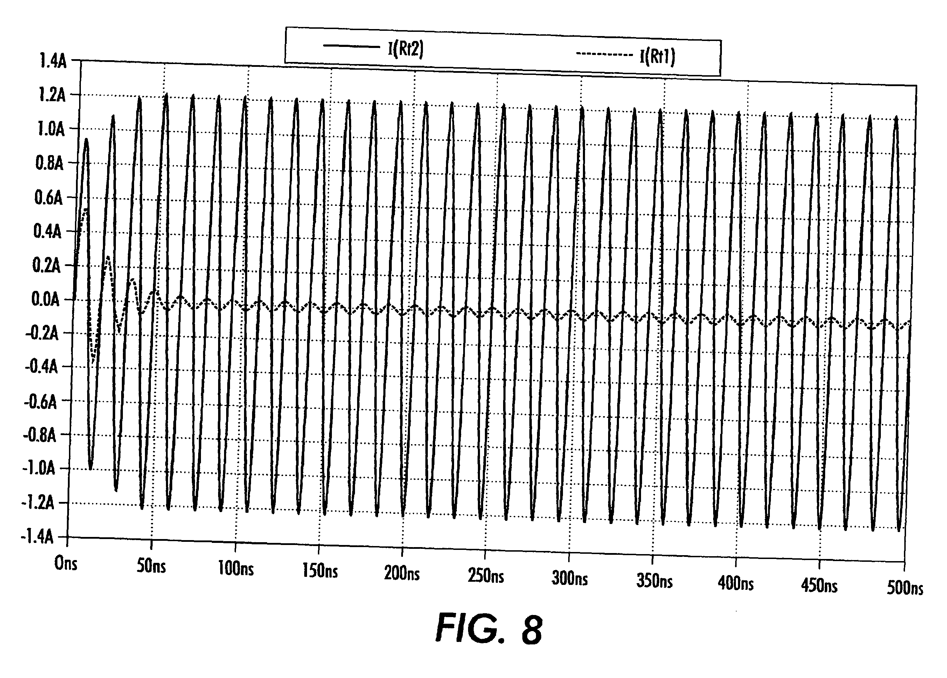

In Figure 8, it is assumed that the bipolar pacing leads of Figure 7 are subjected to a 64 MHz magnetic resonance imaging environment. As demonstrated in Figure 8, the current (**IRt1,** which represents the current flowing through tissue modeled resistor **1230** of Figure 7) induced by the 64 MHz magnetic resonance imaging environment and flowing through the tissue at the distal end of the second bipolar pacing lead **1200** can be greatly reduced. It is noted that the current (**IRt2,** which represents the current flowing through tissue modeled resistor **1130** of Figure 7) induced by the 64 MHz magnetic resonance imaging environment and flowing through the tissue at the distal end of the first bipolar pacing lead **1100** can have a magnitude between 1.21 and -1.21 amps. This reduced magnitude of current (**IRt1,** which represents the current flowing through tissue modeled resistor **1230** of Figure 7) at the distal end of the bipolar pacing lead can significantly reduce the damage to the tissue due to heat generated by the current flowing to the tissue.

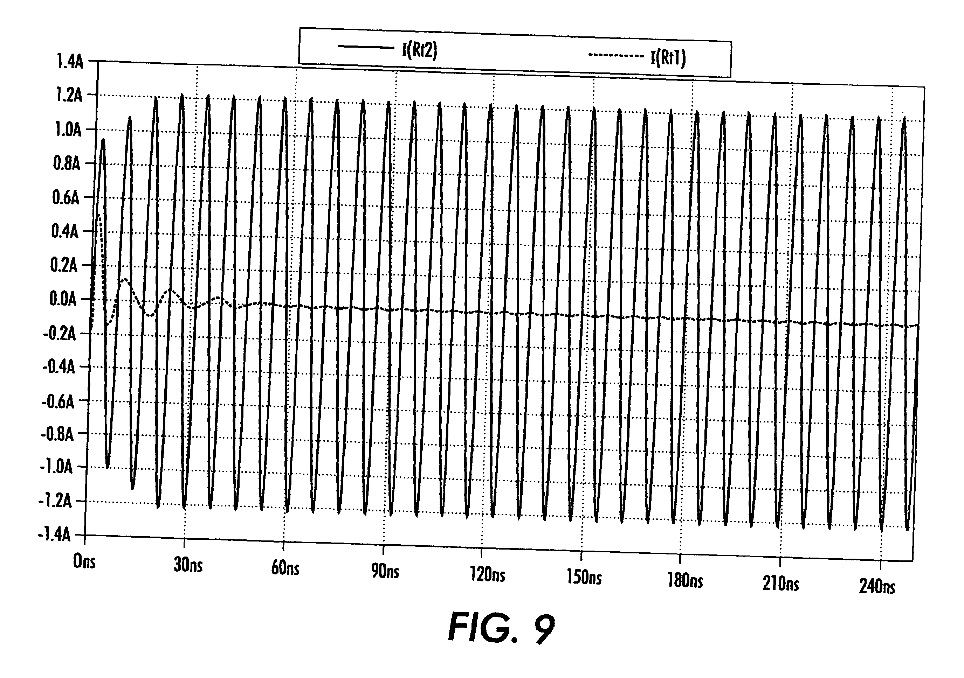

In Figure 9, it is assumed that the bipolar pacing leads of Figure 7 are subjected to a 128 MHz magnetic resonance imaging environment. As demonstrated in Figure 9, the current (**IRt1,** which represents the current flowing through tissue modeled resistor **1230** of Figure 7) induced by the 128 MHz magnetic resonance imaging environment and flowing through the tissue at the distal end of the second bipolar pacing lead **1200** can be greatly reduced. It is noted that the current (**IRt2,** which represents the current flowing through tissue modeled resistor **1130** of Figure 7) induced by the 128 MHz magnetic resonance imaging environment and flowing through the tissue at the distal end of the first bipolar pacing lead **1100** can have a magnitude between 1.21 and -1.21 amps. This reduced magnitude of current (**IRt1,** which represents the current flowing through tissue modeled resistor **1230** of Figure 7) at the distal end of the bipolar pacing lead can significantly reduce the damage to the tissue due to heat generated by the current flowing to the tissue.

It is noted that by including the two resonant circuits (**2000** and **3000**), the bipolar pacing leads can reduce heat generation, notwithstanding the operational frequency of the magnetic resonance imaging scanner. It is noted that further resonant circuits may be added, each tuned to a particular operational frequency of a magnetic resonance imaging scanner.

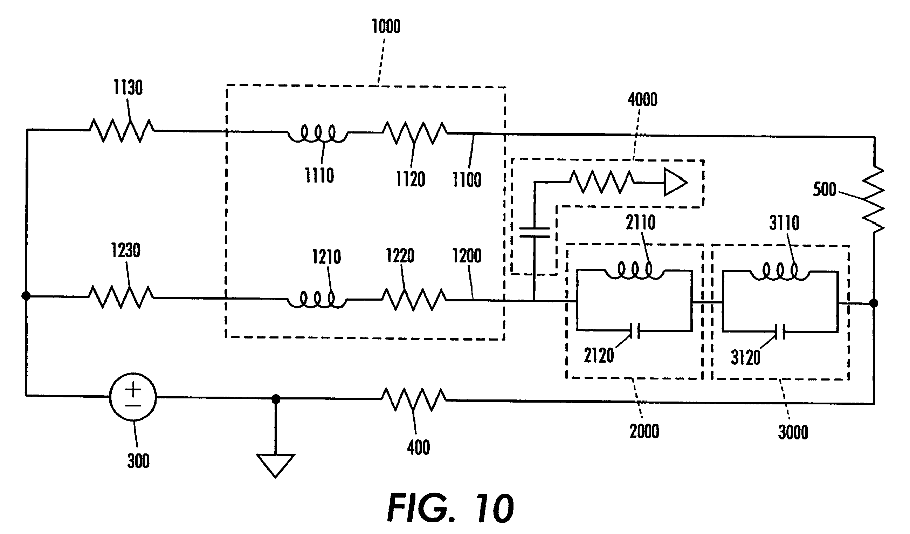

To reduction of the heat generated by the induced current in the tissue, Figure 10 provides a circuit representation of a bipolar pacing lead according to the concepts of the present invention. As illustrated in Figure 10, the bipolar pacing lead **1000** includes two leads (**1100** and **1200**). A first pacing lead **1100** includes resistance and inductance represented by a first resistor **1120** and a first inductor **1110,** respectively. A second pacing lead **1200** includes resistance and inductance represented by a second resistor **1220** and a second inductor **1210,** respectively. At a distal end of each lead, the leads (**1100** and **1200**) come in contact with tissue.

As illustrated in Figure 10, the circuit paths from the distal ends of the leads (**1100** and **1200**) include a first tissue resistance, represented by first tissue modeled resistor **1130,** and a second tissue resistance, represented by second tissue modeled resistor **1230.**

The circuit representation of a bipolar pacing lead, as illustrated in Figure 10, further includes a voltage source **300** that represents the induced electromagnetic energy (voltage or current) from magnetic resonance imaging, a body resistor **400** that represents the resistance of the body, and a differential resistor **500** that represents a resistance between the leads.

In addition to the elements discussed above, the circuit representation of a bipolar pacing lead, as illustrated in Figure 10, includes two resonant circuits (**2000** and **3000**) in series or inline with one of the pacing leads, namely the second lead **1200.** The first resonant circuit **2000** includes a LC circuit, tuned to about 64 MHz, having an inductor **2110** in parallel to a capacitor **2120.** The second resonant circuit **3000** includes a LC circuit, tuned to about 128 MHz, having an inductor **3110** in parallel to a capacitor **3120.**

The lead **1200** together with the inline resonant circuits (**2000** and **3000**) act as an anti-antenna device, thereby reducing the magnitude of the current induced through the tissue at the distal end of the pacing lead (**1200**).

Lastly, the circuit representation of a bipolar pacing lead, as illustrated in Figure 10, includes a capacitance circuit **4000** (a capacitor and resistor), which may represent parasitic capacitance or distributive capacitance in the second pacing lead (1200) or additional capacitance added to the pacing lead. It is noted that the parasitic capacitance or distributive capacitance is the inherent capacitance in a pacing lead along its length. Moreover, it is noted that the parasitic capacitance or distributive capacitance may be the inter-loop capacitance in a coiled wire pacing lead. The location of the capacitance circuit **4000** positions the resonant circuits (**2000** and **3000**) at the proximal end of the pacing lead (**1200**).

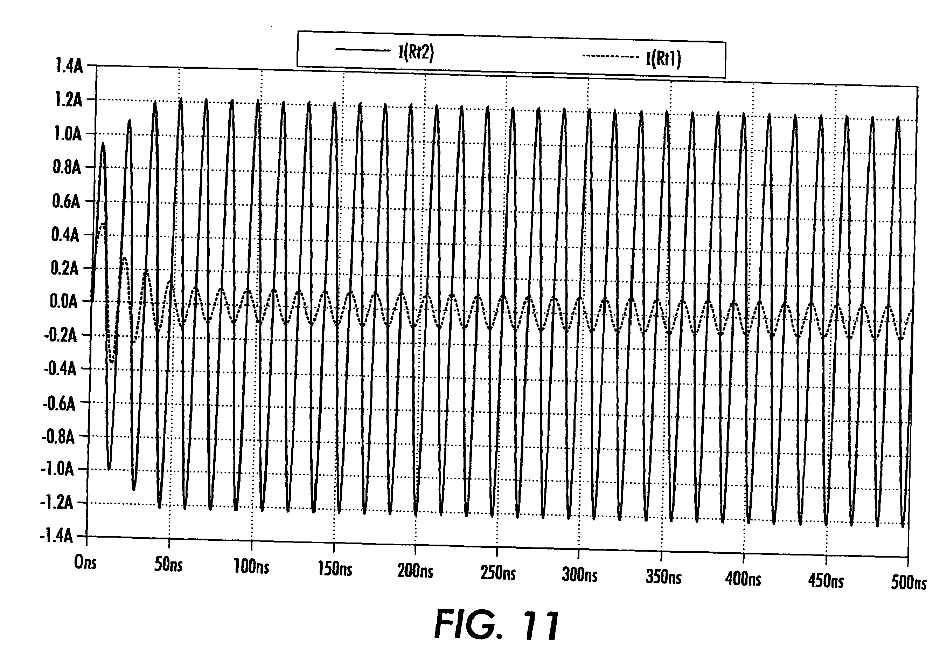

In Figure 11, it is assumed that the bipolar pacing leads of Figure 10 are subjected to a magnetic resonance imaging environment having an operating radio frequency of approximately 64 MHz. As demonstrated in Figure 11, the current (**IRt1,** which represents the current flowing through tissue modeled resistor **1230** of Figure 10) induced by the magnetic resonance imaging environment and flowing through the tissue at the distal end of the second bipolar pacing lead **1200** is not reduced by the same amount as the previous circuits. In other words, the capacitance circuit **4000** lowers the effectiveness of the resonant circuits (**2000** and **3000**), located at the proximal end of the lead, to block the magnetic resonance imaging induced currents.

It is noted that the current (**IRt2,** which represents the current flowing through tissue modeled resistor **1130** of Figure 10) induced by the magnetic resonance imaging environment and flowing through the tissue at the distal end of the first bipolar pacing lead **1100** can have a magnitude between 1.21 and -1.21 amps.

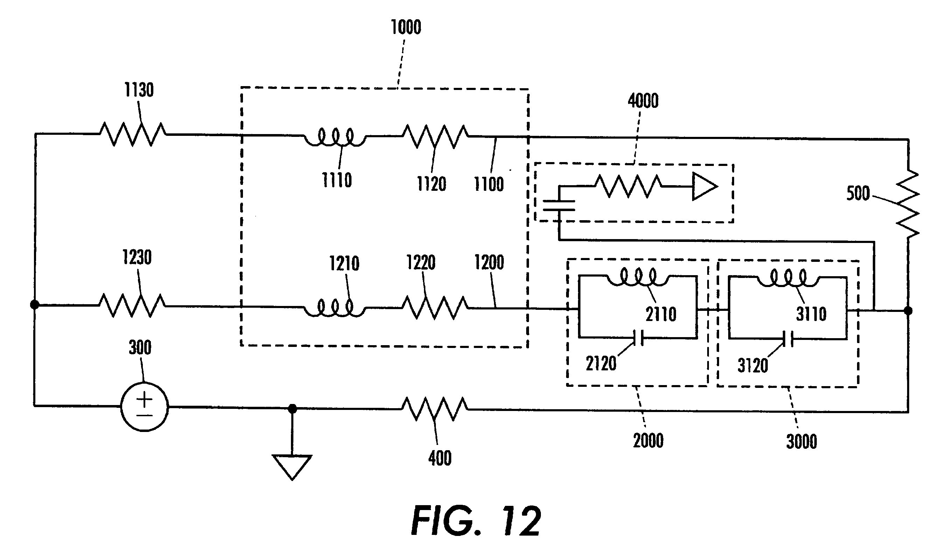

Although the resonant circuits (**2000** and **3000**) still reduce the induced current, the capacitance circuit **4000** reduces the effectiveness of the resonant circuits (**2000** and **3000**). To increase the effectiveness of the resonant circuits (**2000** and **3000**), the resonant circuits (**2000** and **3000**) are moved to the distal end of the pacing lead, as illustrated in Figure 12.

To reduction of the heat generated by the induced current in the tissue, Figure 12 provides a circuit representation of a bipolar pacing lead according to the concepts of the present invention. As illustrated in Figure 12, the bipolar pacing lead **1000** includes two leads (**1100** and **1200**). A first pacing lead **1100** includes resistance and inductance represented by a first resistor **1120** and a first inductor **1110,** respectively. A second pacing lead **1200** includes resistance and inductance represented by a second resistor **1220** and a second inductor **1210,** respectively. At a distal end of each lead, the leads (**1100** and **1200**) come in contact with tissue.

As illustrated in Figure 12, the circuit paths from the distal ends of the leads (**1100** and **1200**) include a first tissue resistance, represented by first tissue modeled resistor **1130,** and a second tissue resistance, represented by second tissue modeled resistor **1230.**

The circuit representation of a bipolar pacing lead, as illustrated in Figure 12, further includes a voltage source **300** that represents the induced electromagnetic energy (voltage or current) from magnetic resonance imaging, a body resistor **400** that represents the resistance of the body, and a differential resistor **500** that represents a resistance between the leads.

In addition to the elements discussed above, the circuit representation of a bipolar pacing lead, as illustrated in Figure 12, includes two resonant circuits (**2000** and **3000**) in series or inline with one of the pacing leads, namely the second lead **1200.** The first resonant circuit **2000** includes a LC circuit, tuned to about 64 MHz, having an inductor **2110** in parallel to a capacitor **2120.** The second resonant circuit **3000** includes a LC circuit, tuned to about 128 MHz, having an inductor 3110 in parallel to a capacitor **3120.**

The lead **1200** together with the inline resonant circuits (**2000** and **3000**) act as an anti-antenna device, thereby reducing the magnitude of the current induced through the tissue at the distal end of the pacing leads (**1100** and **1200**).

Lastly, the circuit representation of a bipolar pacing lead, as illustrated in Figure 12, includes a capacitance circuit **4000** (a capacitor and resistor), which may represent parasitic capacitance in the second pacing lead (**1200**) or additional capacitance added to the pacing lead. The location of the capacitance circuit **4000** positions the resonant circuits (**2000** and **3000**) at the distal end of the pacing lead (**1000**).

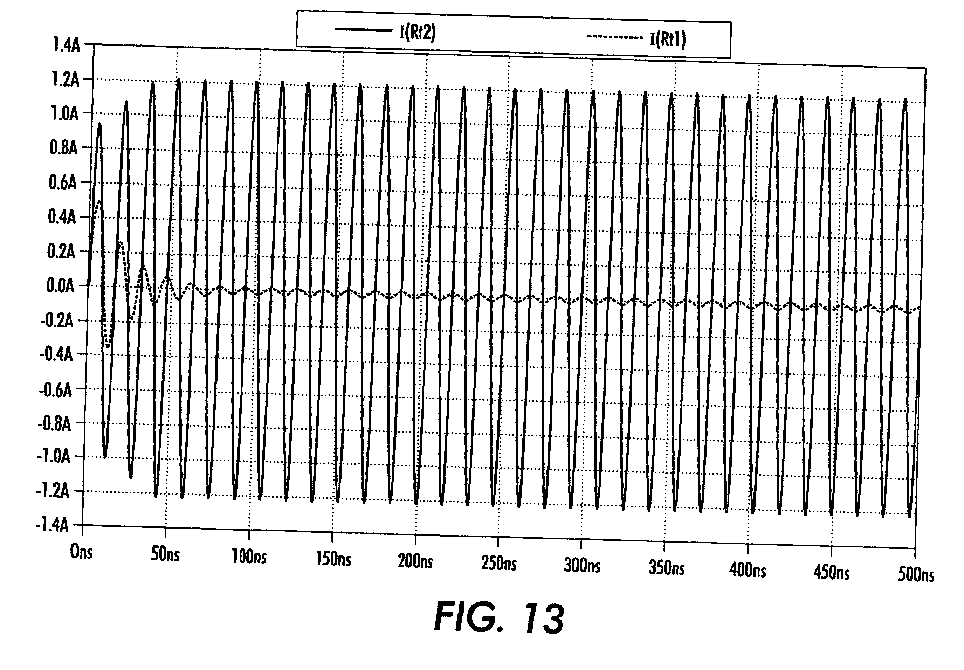

In Figure 13, it is assumed that the bipolar pacing leads of Figure 12 are subjected to a magnetic resonance imaging environment having an operating radio frequency of approximately 64 MHz. As demonstrated in Figure 13, the current (**IRt1,** which represents the current flowing through tissue modeled resistor **1230** of Figure 12) induced by the magnetic resonance imaging environment and flowing through the tissue at the distal end of the second bipolar pacing lead **1200** is reduced. In other words, the moving of the resonant circuits (**2000** and **3000**) to the distal end increases the effectiveness of the resonant circuits (**2000** and **3000**), when a parasitic or distributive capacitance of the lead is involved.

It is noted that the current (IRt2, which represents the current flowing through tissue modeled resistor **1130** of Figure 12) induced by the magnetic resonance imaging environment and flowing through the tissue at the distal end of the first bipolar pacing lead **1100** can have a magnitude between 1.21 and -1.21 amps.

However, space is very limited at the distal end of the lead. It is noted that the inductor and capacitor values of the resonant circuits (**2000** and **3000**) can be adjusted which may help reduce the space requirement when implementing the resonant circuit.

The resonance frequency of the circuit is calculated by using the formula

$$f_{RES} = \frac{1}{2\pi\sqrt{LC}}$$

The required inductance (L) can be reduced (thereby reducing the physical size required), by increasing the capacitance (C). So, for example, if L=50nH and C=123.7pF, and if there is no room in the distal end of the pacing lead for an inductor L having the inductance L = 50nH, an inductor having an inductance of L=25nH could be used if the capacitor used has a capacitance of 247.4pF. The resonance frequency remains the same.

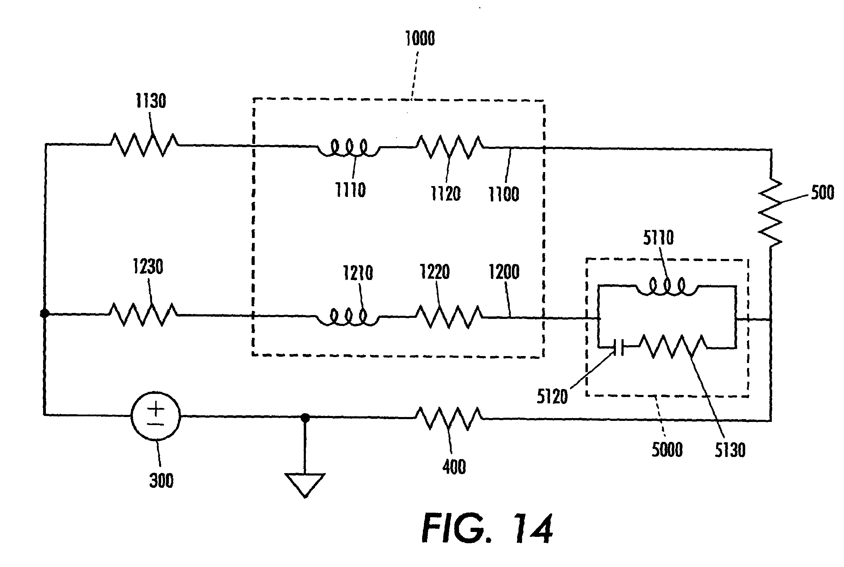

To reduction of the heat generated by the induced current in the tissue, Figure 14 provides a circuit representation of a bipolar pacing lead according to the concepts of the present invention. As illustrated in Figure 14, the bipolar pacing lead **1000** includes two leads (**1100** and **1200**). A first pacing lead **1100** includes resistance and inductance represented by a first resistor **1120** and a first inductor **1110,** respectively. A second pacing lead **1200** includes resistance and inductance represented by a second resistor **1220** and a second inductor **1210,** respectively. At a distal end of each lead, the leads (**1100** and **1200**) come in contact with tissue.

As illustrated in Figure 14, the circuit paths from the distal ends of the leads (**1100** and **1200**) include a first tissue resistance, represented by first tissue modeled resistor **1130,** and a second tissue resistance, represented by second tissue modeled resistor **1230.**

The circuit representation of a bipolar pacing lead, as illustrated in Figure 14, further includes a voltage source **300** that represents the induced electromagnetic energy (voltage or current) from magnetic resonance imaging, a body resistor **400** that represents the resistance of the body, and a differential resistor **500** that represents a resistance between the leads.

In addition to the elements discussed above, the circuit representation of a bipolar pacing lead, as illustrated in Figure 14, includes a resonant circuit (**5000**) in series or inline with one of the pacing leads, namely the second lead **1200.** The resonant circuit **5000** includes a RLC circuit having an inductor **5110** in parallel with a current limiting resistor **5130** and a capacitor **5120.**

The lead **1200** together with the inline resonant circuit (**5000**) acts as an anti-antenna device, thereby reducing the magnitude of the current induced through the tissue at the distal end of the pacing lead (**1200**).

The current limiting resistor **5130** reduces the current in the resonant circuit **5000** to make sure that the inductor **5110** is not damaged by too much current passing through it.

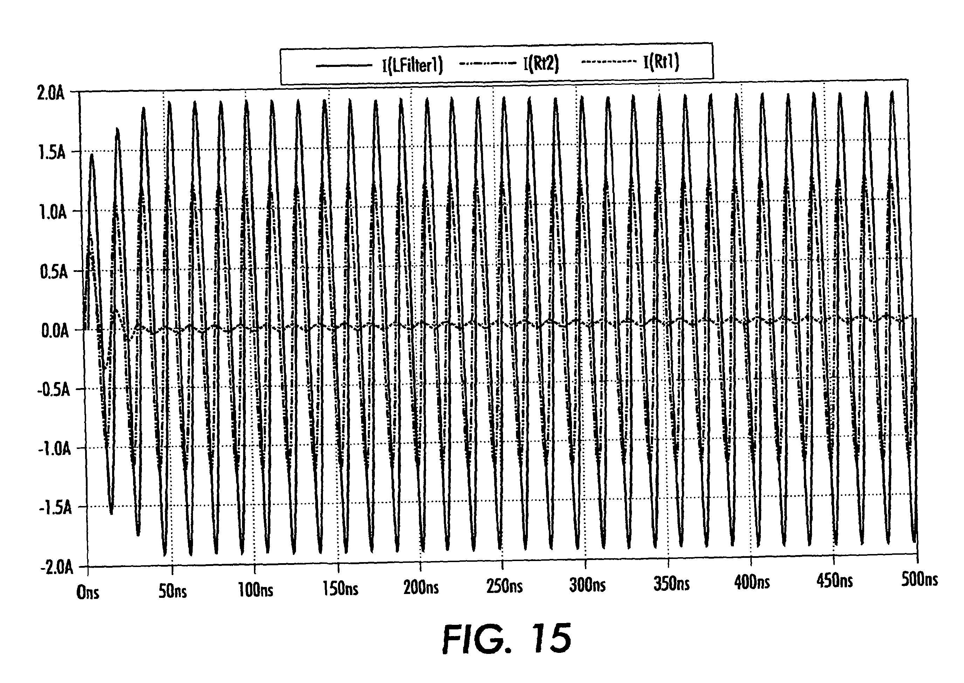

Figure 15, it is assumed that the bipolar pacing leads of Figure 14 are subjected to a magnetic resonance imaging environment having an operating radio frequency of approximately the resonance frequency of the resonant circuit **5000.** As demonstrated in Figure 15, the current (**IRt1,** which represents the current flowing through tissue modeled resistor **1230** of Figure 14) induced by the magnetic resonance imaging environment and flowing through the tissue at the distal end of the second bipolar pacing lead **1200** can be greatly reduced, notwithstanding the addition of the current limiting resistor **5130.** It is noted that the current (**IRt2,** which represents the current flowing through tissue modeled resistor **1130** of Figure 14) induced by the magnetic resonance imaging environment and flowing through the tissue at the distal end of the first bipolar pacing lead **1100** can have a magnitude between 1.21 and -1.21 amps. This reduced magnitude of current (**IRt1,** which represents the current flowing through tissue modeled resistor **1230** of Figure 14) at the distal end of the bipolar pacing lead can significantly reduce the damage to the tissue due to heat generated by the current flowing to the tissue. It is noted that the current **ILFilter1** is the current through the inductor **5110** when the resistor **5130** is a small value.

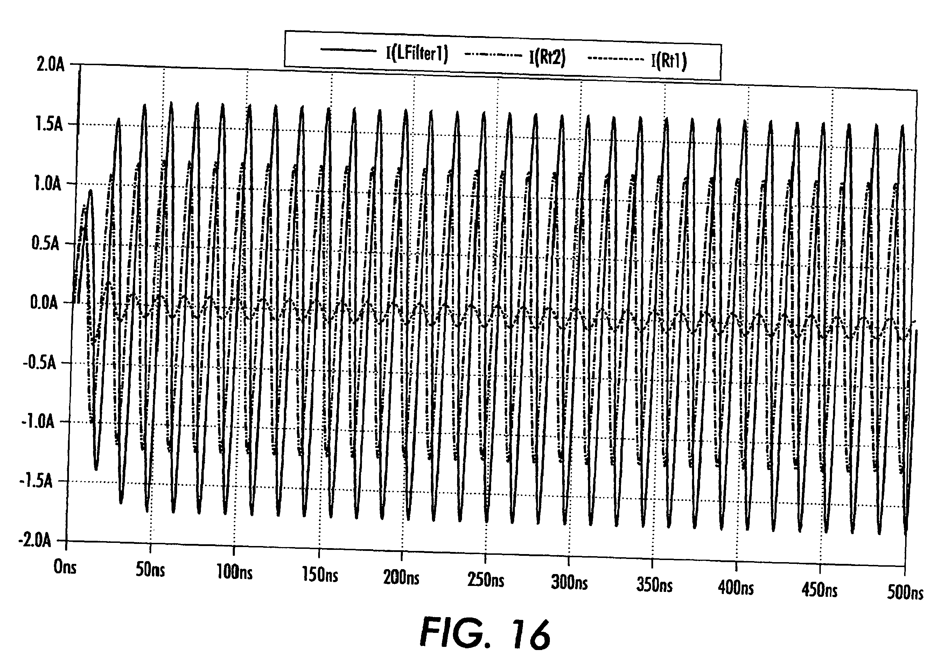

Figure 16, it is assumed that the bipolar pacing leads of Figure 14 are subjected to a magnetic resonance imaging environment wherein the resistance of the current limiting resistor **5130** is increased. As demonstrated in Figure 16, the current (**IRt1,** which represents the current flowing through tissue modeled resistor **1230** of Figure 14) induced by the magnetic resonance imaging environment and flowing through the tissue at the distal end of the second bipolar pacing lead **1200** can be reduced, but the increased resistance of the current limiting resistor **5130** has a slight negative impact on the effectiveness of the resonant circuit **5000.**

It is noted that the current (**IRt2,** which represents the current flowing through tissue modeled resistor **1130** of Figure 14) induced by the magnetic resonance imaging environment and flowing through the tissue at the distal end of the first bipolar pacing lead **1100** can have a magnitude between 1.21 and -1.21 amps. This reduced magnitude of current (**IRt1,** which represents the current flowing through tissue modeled resistor **1230** of Figure 14) at the distal end of the bipolar pacing lead can significantly reduce the damage to the tissue due to heat generated by the current flowing to the tissue. It is noted that the current **ILFilter1** through the inductor **5110** has decreased with the increase in the resistance of resistor **5130,** thereby illustrating controlling the current through the inductor **5110** of the resonant circuit.

It is noted that the frequencies used in generating the various graphs are examples and do not represent the exact frequencies to be used in the design and manufacturing of these circuits. More specifically, the exact frequencies to be used are governed by the Larmor frequency of the proton in the Hydrogen atom and the frequency of the radio frequency of the magnetic resonance imaging scanner.

The gyromagnetic ratio for the proton in the Hydrogen atom is $\gamma = 42.57$ MHz/T or $\gamma = 42.58$ MHz/T, depending on the reference used. In the following discussion $\gamma = 42.57$ MHz/T will be used.

Given that the Larmor equation is $f = B_0 \times \gamma$, the frequency to which the resonant circuit is to be tuned, for example, in a 1.5T magnetic resonance imaging scanner, is $f = (1.5 \text{ T})(42.57 \text{ MHz/T}) = 63.855$ MHz.

The following table gives the resonance frequency for several cases along with example circuit parameter values for the inductor and capacitor to form the resonance circuit.

**Table 1**

| $B_0$ (Tesla) | Circuit Resonance Frequency (MHz) | Example Circuit Parameters | |
| --- | --- | --- | --- |
| | | Inductor (nH) | Capacitor (pF) |
| 0.5 | 21.285 | 50 | 1118.2 |
| 1.0 | 42.57 | 50 | 279.55 |
| 1.5 | 63.855 | 50 | 124.245 |
| 3.0 | 127.71 | 50 | 31.06 |

These circuit parameter values are for the ideal case. It is expected that the actual values used in a real circuit could be different. That is, in the excitation signal's frequency environment of the magnetic resonance imaging scanner, there are other effects (like parasitic capacitance in the inductor) that may affect the circuit, requiring the circuit parameters to be adjusted. In one embodiment, the resonant circuits are tuned to a frequency close to the ideal values given in Table 1. In another embodiment, at least one resonant circuit is tuned to within 5 MHz of the ideal resonant frequency given in Table 1. In still another embodiment, at least one resonant circuit is tuned to within 10 MHz of the resonant frequency given in Table 1.

It is noted that introducing the resonant circuit only into one of the two bipolar pacing wires may result in an increase in the current through the other wire.

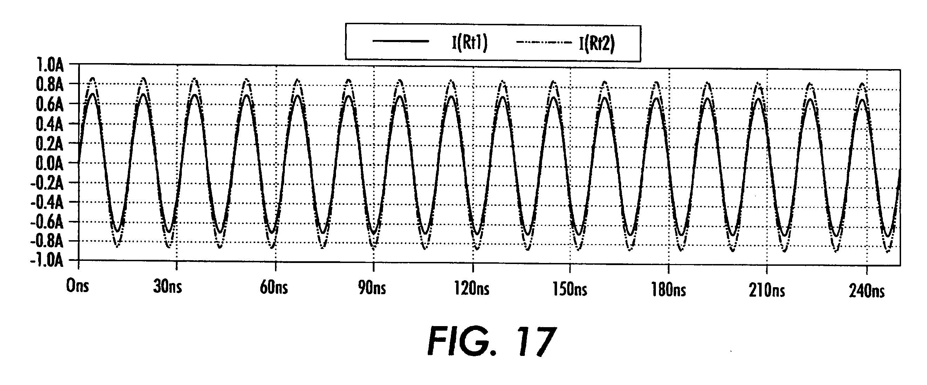

For example, as illustrated in Figure 17, when no resonant circuits are included with the bipolar pacing leads (Figure 2), the current flowing through the first tissue modeled resistor **130** and second tissue modeled resistor **230** of Figure 2 is significant, thereby generating heat to possibly damage the tissue.

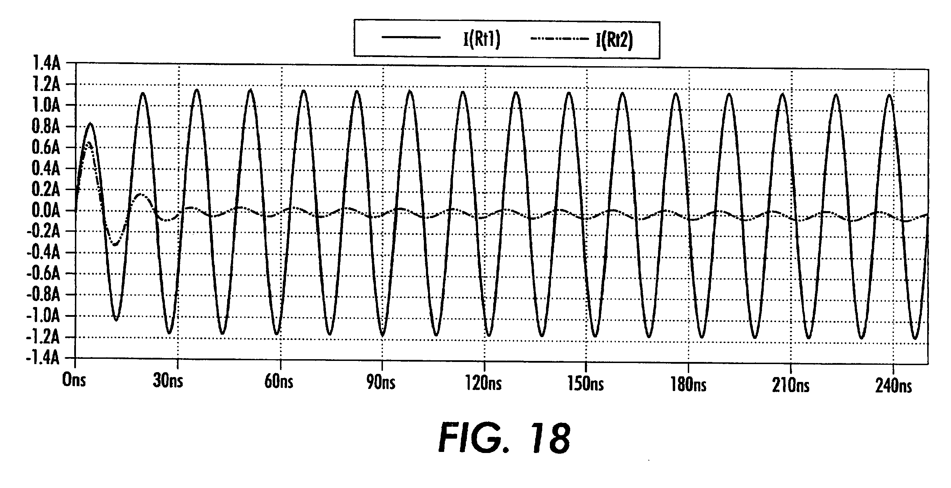

On the other hand, as illustrated in Figure 18, when a resonant circuit or resonant circuits are included in only one of the bipolar pacing leads (Figures 4, 7, 10 and 14), the current (**IRt1**) flowing through the second tissue modeled resistor **1230** is significantly reduced in the one lead, but the current (**IRt2**) slightly increases in the first tissue modeled resistor **1130.** It is noted that these behaviors are dependent on the characteristics of the implemented pacing lead and pulse generator system.

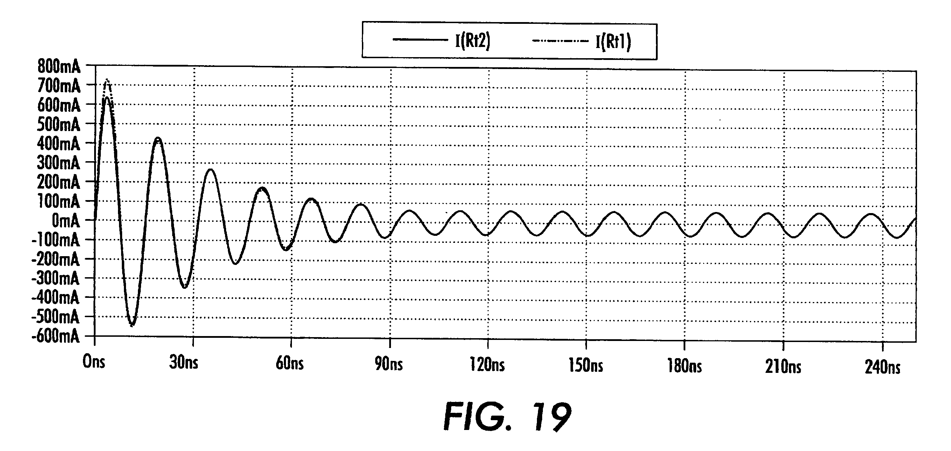

On the other hand, as illustrated in Figure 19, when a resonant circuit or resonant circuits are included in both bipolar leads (not shown), the current (**IRt1** and **IRt2**) flowing through the tissue modeled resistors **130** and **1130** and second tissue modeled resistor **230** and **1230** is significantly reduced.

It is noted that even if the resonant circuits of the present invention are tuned, for example to 63.86 MHz on the bench top, when the resonant circuits of the present invention are placed in the patient's body, the resonant circuits of the present invention may shift resonance a little because of inductive and capacitive coupling to the surrounding environment.

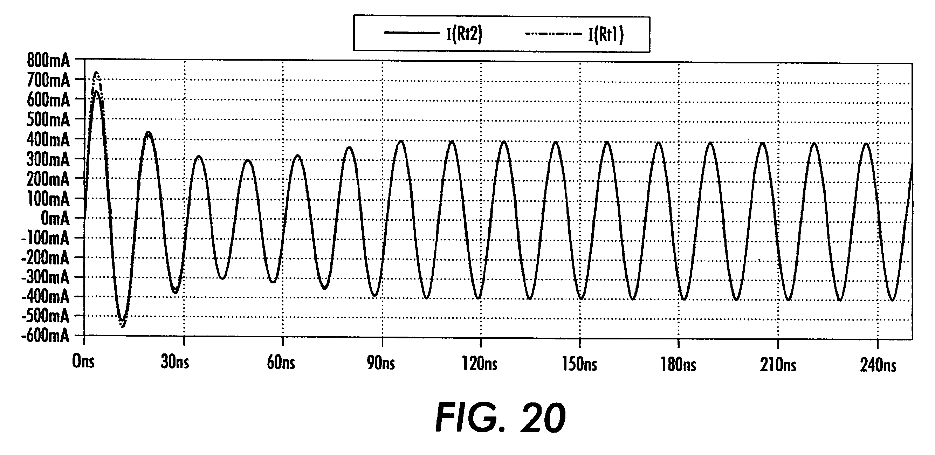

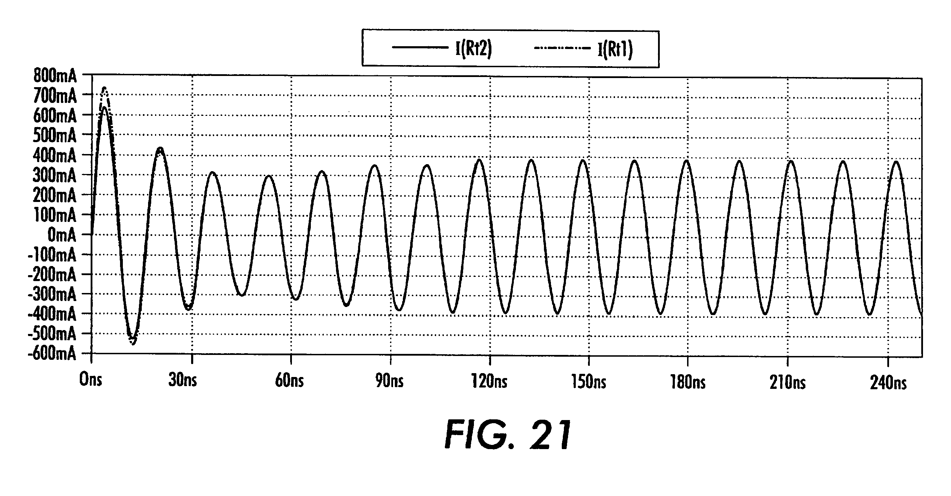

Notwithstanding the potential shift, the concepts of the present invention still significantly reduce the heat generated current in the tissue at the distal end of the bipolar pacing leads, as illustrated in Figures 20 and 21. Figures 20 and 21 provide a graphical representation of the effectiveness of the resonant circuits of the present invention as the circuits are tuned away from the ideal resonance of 63.86 MHz (for the 1.5 T case).

In Figure 20, the inductance of the resonant circuit is increased by 10%. In this instance, the resonant circuits of the present invention significantly reduce the heat generated by currents (**IRt1** and **IRt2**) in the tissue at the distal end of the bipolar pacing leads.

Moreover, in Figure 21, the inductance of the resonant circuit is decreased by 10%. In this instance, the resonant circuits of the present invention significantly reduce the heat generated by currents (**IRt1** and **IRt2**) in the tissue at the distal end of the bipolar pacing leads.

Therefore, the resonant circuits of the present invention need not be perfectly tuned to be effective. As mentioned above, even if the resonant circuits of the present invention were perfectly tuned, once implanted into a patient, the circuits are expected to shift resonance frequency.

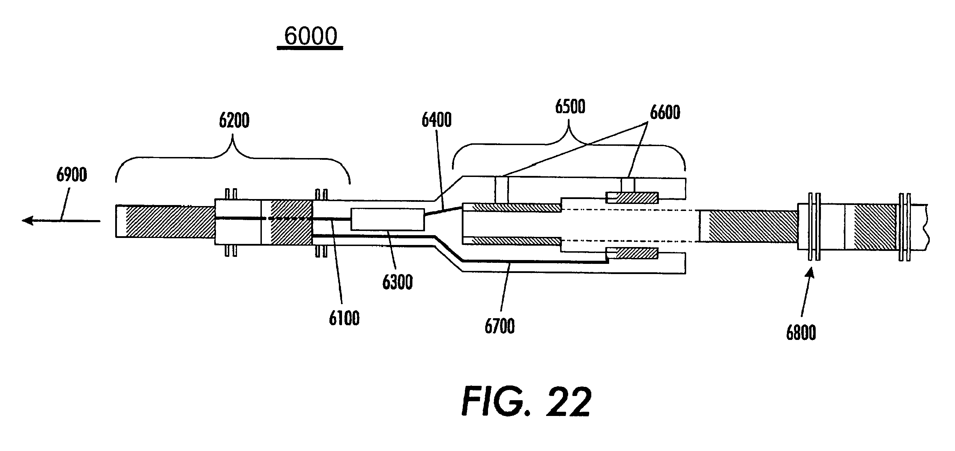

Figure 22 illustrates an adapter, which can be utilized with an existing conventional bipolar pacing lead system. As illustrated in Figure 22, an adapter **6000** includes a male IS-1-BI connector **6200** for providing a connection to an implantable pulse generator **6900**. The adapter **6000** includes a female IS-1-BI connector **6500** for providing a connection to bipolar pacing lead **6800**. The female IS-1-BI connector **6500** includes locations **6600** for utilizing set screws to hold the adapter **6000** to the bipolar pacing lead **6800.**

The adapter **6000** further includes connection wire **6700** to connect the outer ring of the bipolar pacing lead **6800** to the outer ring of the implantable pulse generator **6900**. The adapter **6000** includes a wire **6400** to connect an inner ring of the bipolar pacing lead **6800** to a resonant circuit **6300** and a wire **6100** to the resonant circuit **6300** to an inner ring of the implantable pulse generator **6900**. It is noted that an additional resonant circuit could be placed between the outer ring of the bipolar pacing lead **6800** and the outer ring of the implantable pulse generator 6900.

It is noted that the resonant circuit **6300** in Figure 22 can be multiple resonant circuits in series. It is also noted that the adaptor **6000** can be manufactured with resonant circuits in series with both wires of the bipolar pacing lead. It is further noted that this adapter is connected to the proximal end of the bipolar pacing lead.

Additionally, the adapter of the present invention may include enough mass in the housing to dissipate the heat generated by the resonant circuits. Alternatively, the adapter may be constructed from special materials; e.g., materials having a thermal transfer high efficiency, etc.; and/or structures; e.g., cooling fins, etc.; to more effectively dissipate the heat generated by the resonant circuits. Furthermore, the adapter may include, within the housing, special material; e.g., materials having a thermal transfer high efficiency, etc.; and/or structures; e.g., cooling fins, etc.; around the resonant circuits to more effectively dissipate the heat generated by the resonant circuits.

The concepts of the adapter of Figure 22 can be utilized in a different manner with an existing conventional bipolar pacing lead system. For example, an adapter may include a connector for providing a connection to an implantable electrode or sensor. On the other hand, the adapter may include an implantable electrode or sensor instead of a connection therefor.

The adapter may also include a connector for providing a connection to bipolar pacing lead. The connector may include locations for utilizing set screws or other means for holding the adapter to the bipolar pacing lead.

As in Figure 22, this modified adapter would include a connection wire to connect one conductor of the bipolar pacing lead to the electrode or sensor. The modified adapter would include a wire to connect the other conductor of the bipolar pacing lead to a resonant circuit and a wire to the resonant circuit to ring associated with the electrode of other device associated with the sensor, such as a ground. It is noted that an additional resonant circuit could be placed between the one conductor of the bipolar pacing lead and the electrode or sensor.

It is noted that the resonant circuit can be multiple resonant circuits in series. It is also noted that the modified adaptor can be manufactured with resonant circuits in series with both wires of the bipolar pacing lead. It is further noted that this modified adapter is connected to the distal end of the bipolar pacing lead.

Additionally, the modified adapter of the present invention may include enough mass in the housing to dissipate the heat generated by the resonant circuits. Alternatively, the modified adapter may be constructed from special materials; e.g., materials having a thermal transfer high efficiency, etc.; and/or structures; e.g., cooling fins, etc.; to more effectively dissipate the heat generated by the resonant circuits. Furthermore, the modified adapter may include, within the housing, special material; e.g., materials having a thermal transfer high efficiency, etc.; and/or structures; e.g., cooling fins, etc.; around the resonant circuits to more effectively dissipate the heat generated by the resonant circuits.

In one embodiment, all other wires and electrodes, which go into a magnetic resonance imaging environment, (and not necessarily implanted into the patient's body) can be augmented with a resonant circuit. Any wires to sensors or electrodes, like the electrodes of EEG and EKG sensor pads, can be augmented with a resonant circuit in series with their wires. Even power cables can be augmented with resonant circuits.

Other implanted wires, e.g. deep brain stimulators, pain reduction stimulators, etc. can be augmented with a resonant circuit to block the induced currents caused by the excitation signal's frequency of the magnetic resonance imaging scanner.

Additionally, the adapter of the present invention, when used within implanted devices, may contain means for communicating an identification code to some interrogation equipments external to the patient's body. That is, once the implantable pulse generator, adapter, and pacing lead are implanted into the patient's body, the adapter has means to communicate and identify itself to an external receiver. In this way, the make, model, year, and the number of series resonance circuits can be identified after it has been implanted into the body. In this way, physicians can interrogate the adapter to determine if there is a resonance circuit in the adapter which will block the excitation signal's frequency induced currents caused by the magnetic resonance imaging scanner the patient is about to be placed into.

Furthermore, the adapter of the present invention has the capability of being tested after implantation to insure that the resonance circuit is functioning properly.

Since the present invention is intended to be used in a magnetic resonance imaging scanner, care needs to be taken when selecting the inductor to be used to build the resonant circuit. The preferred inductor should not contain a ferromagnetic or ferrite core. That is, the inductor needs to be insensitive to the magnetic resonance imaging scanner's $B_0$ field. The inductor should also be insensitive to the excitation signal's frequency field (B1) of the magnetic resonance imaging scanner. The inductor should function the same in any orientation within the magnetic resonance imaging scanner. This might be accomplished putting the inductor (for the entire resonant circuit) in a Faraday cage.

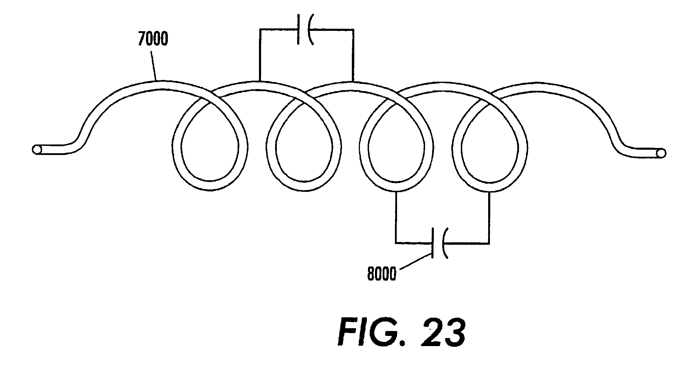

The resonant circuit of the present invention could also be realized by adding capacitance along the bipolar pacing lead, as illustrated in Figure 23. In Figure 23, capacitors **8000** are added across the coils of the pacing lead **7000.**

With respect to Figure 23, the adjacent coiled loops of the coiled wire provide the capacitors for an RLC parallel resonant circuit. The adjacent coiled loops can be adjusted by various means (coiling pitch, wire cross section geometry (square, rectangular, circular), dielectric material between adjacent loops) to tune the coiled wire to have a self-resonance at or near or harmonic of the radio-frequency of the magnetic resonance imaging scanner.

As illustrated in Figure 23, an oxidation layer or an insulating material is formed on the wire resulting in essentially a resistive coating over the wire form. Thus, the current does not flow through adjacent coil loop contact points, but the current instead follows the curvature of the wire. The parasitic capacitance enables electrical current to flow into and out of the wire form due to several mechanisms, including the oscillating electrical field set up in the body by the magnetic resonance imaging unit.

In pacing leads and some other leads, a coiled wire is used. A thin insulative film (polymer, enamel, etc.) is coated over the wire used to electrically insulate one coiled loop from its neighboring loops. This forms an inductor. By inserting an appropriate sized capacitor **8000** across multiple loops of the coiled wire, a parallel resonance circuit suitable for reducing the induced current, in accordance with the concepts of the present invention, can be formed.

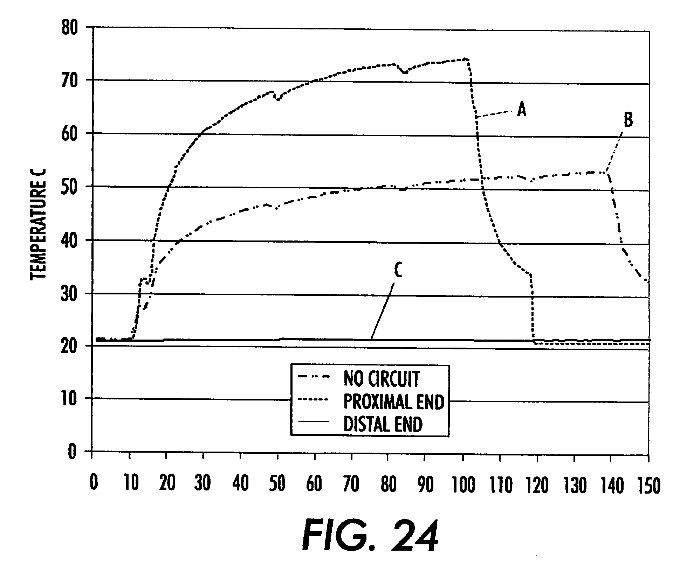

Figure 24 shows the temperature of the tissue at the distal end of a wire wherein the wire includes a resonant circuit at the proximal end (**A**); the wire does not include a resonant circuit (**B**); and the wire includes a resonant circuit at the distal end (**C**).

As illustrated in Figure 24, the "Proximal End" case (**A**) (resonant circuit at proximal end) results in a higher temperature increase at the distal end than when the resonant circuit is located at the distal end (**C**). In the demonstration used to generate the results of Figure 24, a wire of 52 cm in length and having a cap at one end was utilized, resulting in a distributive capacitive coupling to the semi-conductive fluid into which the wires were placed for these magnetic resonance imaging heating experiments.

For the "Proximal End" case (**A**) (resonant circuit at proximal end) only, the resonant circuit was inserted 46.5 cm along the wire's length. Since no current at 63.86 MHz can pass through the resonant circuit, this sets any resonant wave's node at 46.5 cm along the wire. This effectively shortened the length of the wire and decreased the wire's self-inductance and decreased the distributive capacitance. These changes then "tuned" the wire to be closer to a resonance wave length of the magnetic resonance imaging scanner's transmitted radio frequency excitation wave resulting in an increase in the current at the distal end of the wire.

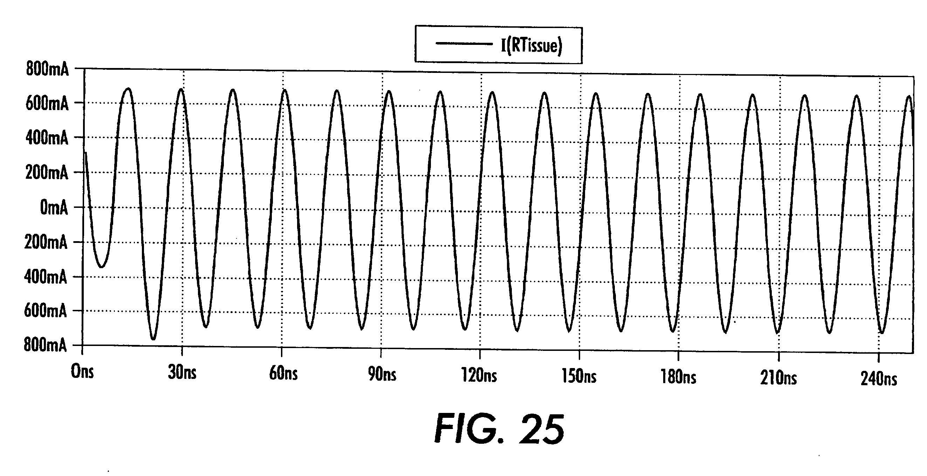

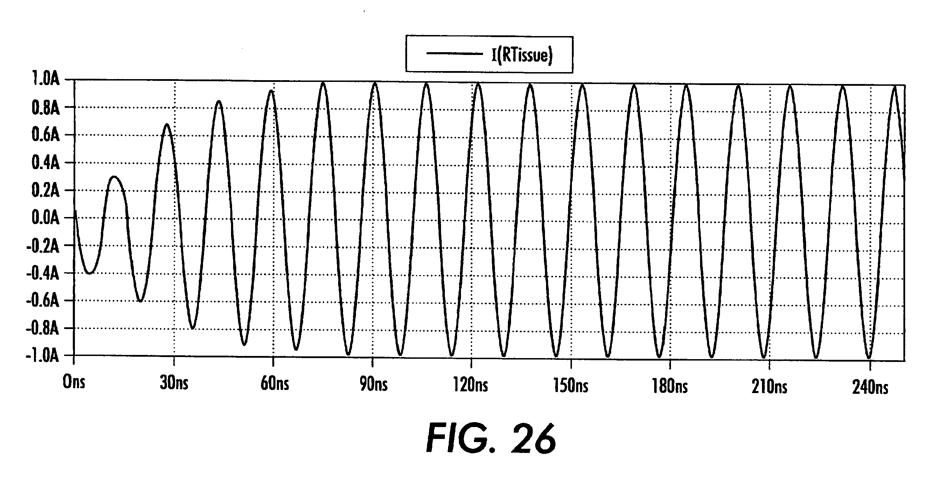

The effective length of the wire with the resonant circuit is now 46.5 cm rather than the physical length of 52 cm. That is, the inductance and capacitance of the wire is now such that its inherent resonance frequency is much closer to that of the applied radio-frequency. Hence, the modeled current through the distal end into the surrounding tissue increases from about 0.65 Amps when there is no resonant circuit at the proximal end (Figure 25) to about 1.0 Amps when the resonant circuit is inserted at the proximal end of the wire (Figure 26).

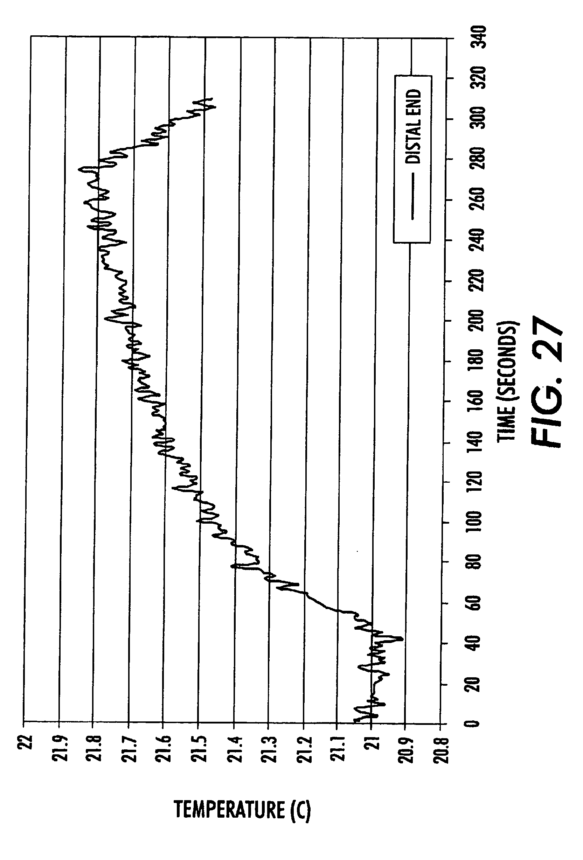

As illustrated in Figure 27 (which is a close up of trace "C" in Figure 24), when the wire includes a resonant circuit at the distal end, the temperature rise is significantly less (about 0.9° C after 3.75 minutes). On the other hand, when the wire includes a resonant circuit at the proximal end, the temperature rise at the distal end is greater (See trace **"A"** in Figure 24).

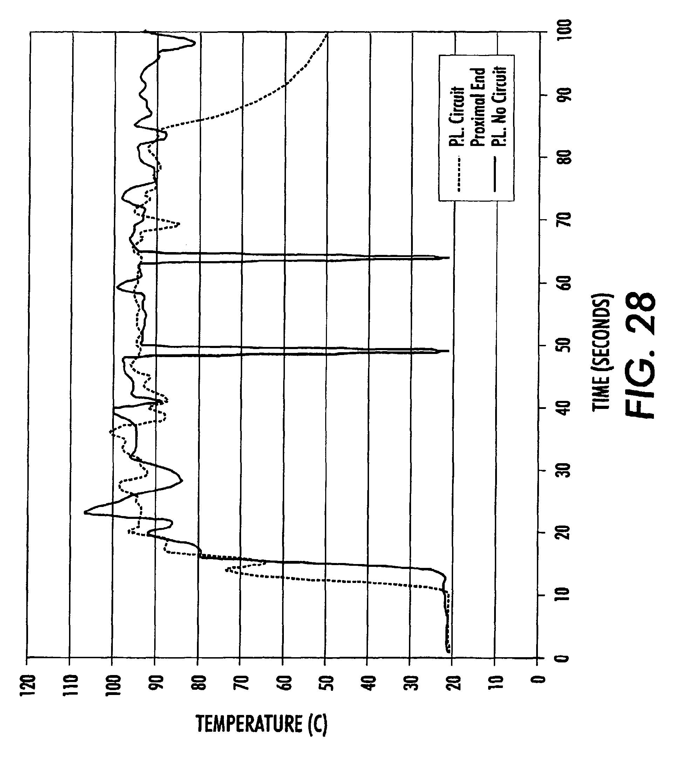

Experimental results with the resonant circuit at the proximal end of a 52 cm long bipolar pacing lead did not demonstrate a significant altering of the heating of the tissue at the distal end, as illustrated in Figure 28. The attachment of the resonant circuit to the proximal end of a pacing lead places a wave node at the end of the pacing lead (no real current flow beyond the end of wire, but there is a displacement current due to the capacitance coupling to the semi-conductive fluid). That is, adding the resonant circuit to the proximal end of the pacing lead, which does not change the effective length of the pacing lead, does not change the electrical behavior of the pacing lead.

Now referring back to Figure 15, it is noted that the current (**Lfilter1**) through the resonant circuit inductor **5110** of Figure 14 is also illustrated. As can be seen, the current (**Lfilter1**) through the resonant circuit inductor **5110** of Figure 14 is larger than the original current passing through a prior art lead, as illustrated in Figure 3. Although the heating of the tissue is significantly decreased with the addition of a resonant circuit, there still may be a problem in that inductors is rated for a certain amount of current before the inductor is damaged.

In anticipation of a possible problem with using inductors not having a high enough current rating, the present invention may provide multiple resonant circuits, each resonant circuit being connected in series therewith and having the same inductor and capacitor (and resistor) value as the original resonant circuit.

As noted above, Figure 7 illustrates an example of multiple serially connected resonant circuits. Although previously described as having values to create different resonance values, the resonant circuits **2000** and **3000** of Figure 7 may also have substantially the same resonance values so as to reduce the current flowing through any single inductor in the resonant circuits **2000** and **3000** of Figure 7.

Moreover, in anticipation of a possible problem with using inductors not having a high enough current rating, the present invention may provide resonant circuits with inductors having larger inductive values. It is noted that it may be difficult to implement an inductor having a larger inductive value in a small diameter lead, such as a pacing lead or DBS lead. In such a situation, the inductor may be constructed to be longer, rather than wider, to increase its inductive value.

It is further noted that the resonance values of the resonant circuits **2000** and **3000** of Figure 7 may be further modified so as to significantly reduce the current through the tissue as well as the current through the resonant circuit's inductor. More specifically, the multiple resonant circuits may be purposely tuned to be off from the operating frequency of the magnetic resonance imaging scanner. For example, the resonance frequency of the resonant circuit may be 70.753 MHz or 74.05 MHz.

In this example, when one resonant circuit of the multiple resonant circuits is purposely not tuned to the operating frequency of the magnetic resonance imaging scanner, the current through the tissue is reduced, while the current through the resonant circuit's inductor is also reduced. Moreover, when two resonant circuits of the multiple resonant circuits are purposely not tuned to the operating frequency of the magnetic resonance imaging scanner, the current through the tissue is further reduced, while the current through the resonant circuit's inductor is also further reduced.

It is further noted that when the two (or more) resonant circuits are not tuned exactly to the same frequency and not all the resonant circuits are tuned to the operating frequency of the magnetic resonance imaging scanner, there is significant reduction in the current through the tissue as well as the current through the resonant circuits' inductors.

In summary, putting the resonant circuit at the proximal end of a pacing lead may not reduce the heating at the distal end of the pacing to a safe level. However, placing the resonant circuit at the proximal end of the pacing lead can protect the electronics in the implanted pulse generator, which is connected at the proximal end. To protect the circuit in the implanted pulse generator, a resonant circuit is placed at the proximal end of the pacing lead so as to block any induced currents from passing from the pacing lead into the implanted pulse generator.

Since the current in the resonant circuit, when in the magnetic resonance imaging scanner (or other radio-frequency field with a frequency of the resonant frequency of the circuit) may be larger than the induced current in the lead (or wire) without the resonant circuit, there may be some heating in the resistive elements of the resonant circuit (in the wires, connection methods, inductor, etc.). Thus, it would be advantageous to connect high thermal conductive material to the resonant circuit to distribute any heating of the circuit over a larger area because heating is tolerable when it is not concentrated in one small place. By distributing the same amount of heating over a larger area, the heating problem is substantially eliminated.

To distribute the heat, the inside of the pacing lead polymer jacket can be coated with a non-electrical conductive material, which is also a very good thermal conductor, and this connected to the circuit. Moreover, filaments of non-electrically conductive but thermally conductive material can be attached to the circuit and run axially along the inside of the pacing lead assembly.

As discussed above, a lead may include a conductor having a distal end and a proximal end and a resonant circuit connected to the conductor. The resonant circuit has a resonance frequency approximately equal to an excitation signal's frequency of a magnetic-resonance imaging scanner. The resonant circuit may be located at the distal end of the conductor or the proximal end of the conductor. The resonant circuit may be: an inductor connected in parallel with a capacitor; an inductor connected in parallel with a capacitor wherein a resistor and capacitor are connected in series; an inductor connected in parallel with a capacitor wherein a resistor and the inductor are connected in series; an inductor connected in parallel with a capacitor and connected in parallel with a resistor; or an inductor connected in parallel with a capacitor wherein a resistor is connected in series with both the capacitor and inductor.

It is noted that a plurality of resonant circuits may be connected in series, each having a unique resonance frequency to match various types of magnetic-resonance imaging scanners or other sources of radiation, such as security systems used to scan individuals for weapons, etc. It is further noted that the lead may include a heat receiving mass located adjacent the resonant circuit to dissipate the heat generated by the resonant circuit in a manner that is substantially non-damaging to surrounding tissue. Furthermore, it is noted that the lead may include a heat dissipating structure located adjacent the resonant circuit to dissipate the heat generated by the resonant circuit in a manner that is substantially non-damaging to surrounding tissue.

It is also noted that the above described lead may be a lead of a bipolar lead circuit.

Moreover, as discussed above, an adapter for a lead may include a housing having a first connector and a second connector, the first connector providing a mechanical and electrical connection to a lead, the second connector providing a mechanical and electrical connection to a medical device, and a resonant circuit connected to the first and second connectors. The resonant circuit may have a resonance frequency approximately equal to an excitation signal's frequency of a magnetic-resonance imaging scanner. The resonant circuit may be an inductor connected in parallel with a capacitor or an inductor connected in parallel with a capacitor and a resistor, the resistor and capacitor being connected in series.

It is noted that a plurality of resonant circuits may be connected in series, each having a unique resonance frequency to match various types of magnetic-resonance imaging scanners or other sources of radiation, such as security systems used to scan individuals for weapons, etc. It is further noted that the adapter may include a heat receiving mass located adjacent the resonant circuit to dissipate the heat generated by the resonant circuit in a manner that is substantially non-damaging to surrounding tissue. Furthermore, it is noted that the adapter may include a heat dissipating structure located adjacent the resonant circuit to dissipate the heat generated by the resonant circuit in a manner that is substantially non-damaging to surrounding tissue.

Furthermore, as discussed above, medical device may include a housing having electronic components therein; a lead mechanically connected to the housing and electrically connected through the housing; and a resonant circuit, located within the housing, operatively connected to the lead and the electronic components. The resonant circuit may have a resonance frequency approximately equal to an excitation signal's frequency of a magnetic-resonance imaging scanner. The resonant circuit may be an inductor connected in parallel with a capacitor or an inductor connected in parallel with a capacitor and a resistor, the resistor and capacitor being connected in series.

It is noted that a plurality of resonant circuits may be connected in series, each having a unique resonance frequency to match various types of magnetic-resonance imaging scanners or other sources of radiation, such as security systems used to scan individuals for weapons, etc. It is further noted that the adapter may include a heat receiving mass located adjacent the resonant circuit to dissipate the heat generated by the resonant circuit in a manner that is substantially non-damaging to surrounding tissue. Furthermore, it is noted that the adapter may include a heat dissipating structure located adjacent the resonant circuit to dissipate the heat generated by the resonant circuit in a manner that is substantially non-damaging to surrounding tissue.

It is noted that although the various embodiments have been described with respect to a magnetic-resonance imaging scanner, the concepts of the present invention can be utilized so as to be tuned to other sources of radiation, such as security systems used to scan individuals for weapons, etc. In these instances, the frequency of an electromagnetic radiation source is the "normal" frequency of an electromagnetic wave. Even if the electromagnetic wave is "circularly polarized," it is not the circular frequency, but the "normal" frequency.

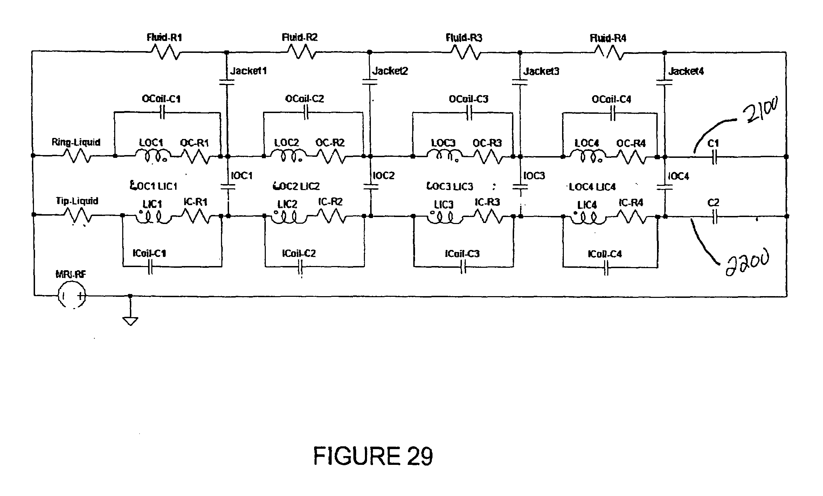

In another example for reducing the heat generated by the induced current in the tissue, Figure 29 provides a circuit representation of another bipolar pacing lead according to the concepts of the present invention. As illustrated in Figure 29, the bipolar pacing lead includes two lead conductors (**2100** and **2200**). A first pacing lead conductor **2100** is segmented into at least four sections. It is noted that the pacing lead could be segmented into more segments, but four segments are being used for this representation. Each section includes coil self-capacitance (**OCoil-C1, OCoil-C2, OCoil-C3,** and **OCoil-C4**). In parallel with the coil self-capacitance, each section has a coil inductance (**LOC1, LOC2,**

**LOC3,** and **LOC4**) and a coil resistance (**OC-R1, OC-R2, OC-R3,** and **OC-R4**). The first pacing lead **2100** is covered with a polymer jacket to prevent direct contact with the body, thus radial capacitors (**Jacket1, Jacket2**, **Jacket3,** and **Jacket4**) are formed along the length of the lead.

The second pacing lead conductor **2200** is segmented into at least four sections. It is noted that the pacing lead could be segmented into more segments, but four segments are being used for this representation. Each section includes self-capacitance (**ICoil-C1, ICoil-C2, ICoil-C3,** and **ICoil-C4**). In parallel with the coil self-capacitance, each section has a coil inductance (**LIC1, LIC2, LIC3,** and **LIC4**) and a coil resistance (**IC-R1, IC-R2, IC-R3,** and **IC-R4**). The second pacing lead **2200** is covered with a polymer tube or coating, thus forming capacitors (**IOC1, IOC2, IOC3,** and **IOC4**) along its length with the inside surface of the outer coiled wire being one of the capacitor's conductive surfaces.

Figure 29 further illustrates a sine wave voltage source **magnetic resonance imaging-RF** to model the induced potentials in the body in which the lead is placed. Furthermore, Figure 29 has not modeled the pulse generator, which is normally connected to the leads in place of capacitors **C1** and **C2.** Two resistors, **Ring-Liquid** and **Tip-Liquid,** represent the electrodes in contact with the body. It is noted that **Tip-Liquid** represents the inner coiled wire in contact with the body, through which minimal magnetic resonance imaging induced current flow is desired.

Figure 29 also illustrates mutual inductive coupling (**LOC1LIC1, LOC2LIC2, LOC3LIC3,** and **LOC4LIC4**) between the outer coiled wire and the inner coiled wire. It is noted that the polarity of the coupling needs to be considered. This accounts for the left-right hand winding of the two coiled wires. The outer coiled wire may be wound left-handed and the inner coiled wire might be wound right-handed. Alternatively, both might be wound left-handed. In one embodiment, the mutual inductive coupling can be adjusted to control the magnetic resonance imaging induced currents through the coiled wires.

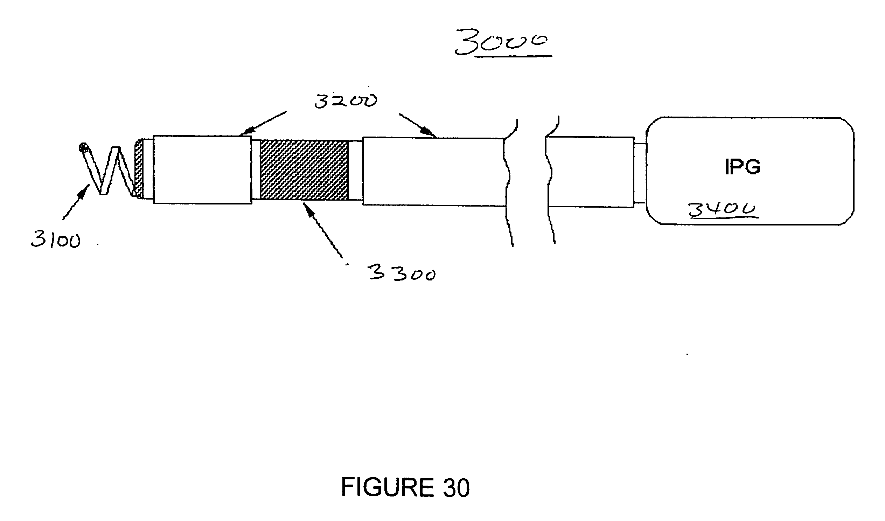

Figure 30 illustrates an embodiment of a bipolar pacing device **3000.** The bipolar pacing device **3000** includes an implantable pulse generator **3400** connected to the bipolar pacing leads. The pacing leads are connected to a tip electrode **3100** and a ring electrode **3300.** The tip electrode **3100** may be corkscrew shaped.

The pacing leads are covered with a shrink wrap **3200.** It is noted that the properties of the shrink wrap can be adjusted to reduce the magnetic resonance imaging induced current through the lead. Adding the shrink wrap material over the existing polymer jacket of the pacing lead, or by changing the type and/or dielectric and/or resistive properties and/or the thickness of the polymer jacket changes the capacitance of capacitors (**Jacket1, Jacket2, Jacket3**, and **Jacket4**) of Figure 29.

In one embodiment, the parameters of the various elements of Figure 29 can be adjusted to change the lead's electrical properties, thereby changing the effect of magnetic resonance imaging induced heating (current). For example, the capacitance of the capacitors (**Jacket1** through **Jacket4**) can be adjusted by changing the lead's outer polymer jacket. More specifically, the outer polymer jacket can be changed, thereby changing the capacitance of the capacitors (**Jacket1** through **Jacket4**), by changing the material, the thickness, and/or dielectric properties.

In another embodiment, the inductance of inductors (**LOC1** through **LOC4**) can be adjusted. More specifically, the inductance (per unit length) of the outer coiled lead can be changed by changing the number of windings, changing the number of filar in the wire used, changing the cross sectional profile of the wire used, and/or coating the wire with material whose magnetic saturation is higher than the DC static field strength used in the magnetic resonance imaging scanner.

In another embodiment, the capacitance of capacitors (**OCoil-C1** through **OCoil-C4**) can be adjusted. More specifically, the inter-loop capacitance of the outer coiled wire can be changed by changing the pitch of the coiled wire, changing the cross sectional shape of the wire used, changing the number of filars used for the coiled wire, coating the wire, and/or coating filars comprising the wire with non-conductive materials; e.g., dielectric materials.

In a further embodiment, the resistance of resistors (**OC-R1** through **OC-R4**) can be adjusted. More specifically, the resistance (per unit length) of the outer coiled lead can be changed by changing the wire material, changing the number of filar in the wire used, and/or changing the cross sectional profile of the wire used.

In a further embodiment, the capacitance of capacitors (**IOC1** through **IOC4**) can be adjusted. More specifically, the capacitance formed between the inner coiled wire and the outer coiled wire can be changed by changing the radial distance between the inner coiled wire's outer radius and the outer coiled wire's inner radius, changing the material interposed between the inner and the outer coiled wires, changing the pitch (number of loops) of the outer coiled wire, and/or changing the number of loops of the inner coiled wire.

In another embodiment, the mutual inductive coupling of inductors (**LOC1L1C1** through **LOCC4LIC4**) can be adjusted. More specifically, the mutual inductive coupling between the inner and outer coiled wires can be changed by changing the coiling handedness of the inner and or the outer coiled wires, changing the number of coiling loops of the outer coiled wire, changing the number of coiling loops of the inner coiled wire, changing the material between the inner and outer coiled wires, and/or changing the material within or on the coiled inner wire.

In another embodiment, the inductance of inductors (**LIC1** through **LIC4**) can be adjusted. More specifically, the inductance (per unit length) of the inner coiled lead can be changed by changing the number of windings, changing the number of filar in the wire used, changing the cross sectional profile of the wire used, and/or coating the wire with material whose magnetic saturation is higher than the DC static field strength used in the magnetic resonance imaging scanner.

In a further embodiment, the inter-loop capacitance of capacitors (**ICoil-C1** through **ICoil-C4**) can be adjusted. More specifically, the inter-loop capacitance of the inner coiled wire can be changed by changing the pitch of the coiled wire, changing the cross sectional shape of the wire used, and/or changing the number of filars used for the coiled wire, coating the wire and/or filars comprising the wire with non-conductive materials, e.g. dielectric materials.

In a further embodiment, the resistance of resistors (**IC-R1** through **IC-R4**) can be adjusted. More specifically, the resistance (per unit length) of the inner coiled lead can be changed by changing the wire material, changing the number of filar in the wire used, and/or changing the cross sectional profile of the wire used.

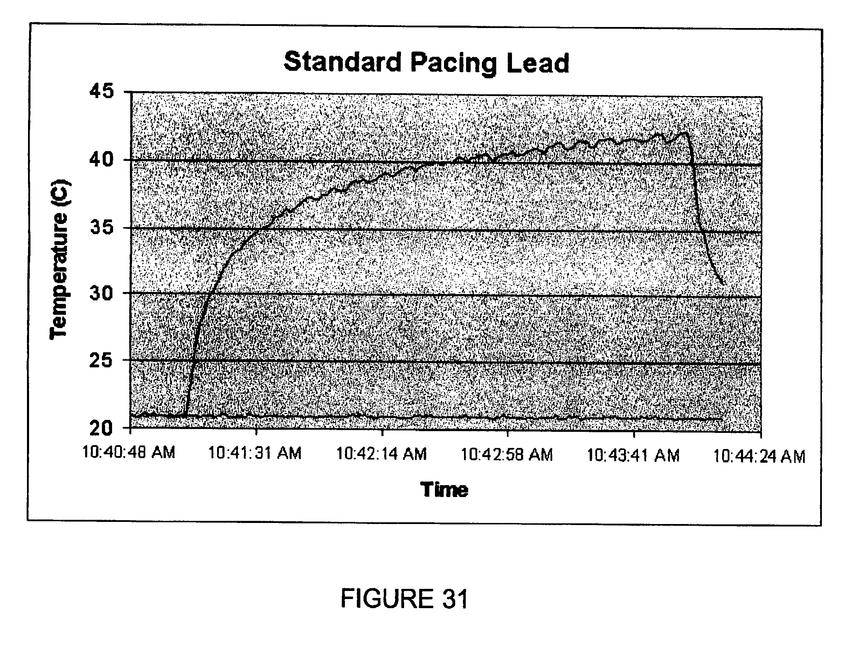

With respect to the description of Figures 31-35, Table 1 gives the measured bipolar pacing lead sample's DC resistance for the inner and outer conductors (coiled wires).

Table 1

| Lead Label | Inner Coil DC Resistance (Ohms) | Outer Coil DC Resistance (Ohms) |
|---|---|---|
| Closed Pitch #1 | 37.8 | 74.2 |
| Closed Pitch #2 | 37.0 | 70.2 |
| Closed Pitch #3 | 37.2 | 68.1 |
| Medium Pitch #1 | 18.1 | 73.8 |
| Medium Pitch #2 | 18.7 | 68.6 |
| Medium Pitch #3 | 18.3 | 69.8 |
| Open Pitch #1 | 12.0 | 71.8 |
| Open Pitch #2 | 18.6 | 67.6 |
| Open Pitch #3 | 14.1 | 68.1 |

Moreover, each of the samples illustrated in Figures 31-35 were subjected to the conditions listed in Table 2.

**Table 2**

| Imaging Parameters | |
|---|---|
| Field Strength | 1.5 Tesla |
| Scanner Coil | Body |
| Sequence | FSE XL |
| Imaging Plane | Axial |
| TE | 16 ms |
| $TE^2$ | 30 ms |
| TR | 67 |
| EchoTrainLength | 4 |
| FOV | 48 |
| Slice Thickness | 10 mm |
| Spacing | 2 mm |
| Freq | 256 |
| Phase | 192 |
| Phase FOV | 1.0 |
| NEX | 1 |
| Bandwidth | 50 |
| Body mass | 79 kg |

(continued)

| Imaging Parameters | |
| --- | --- |
| Number of Slices | 20 thru the leads |

These parameters provided an SAR level of 1.8528 W/kg. The scan time was 2 minutes 52 seconds.

In Figure 31, a conventional pacing lead was tested using the parameters in Table 2. As illustrated in Figure 31, the temperature measured at the distal end of the pacing lead was about 42˚ Celsius.

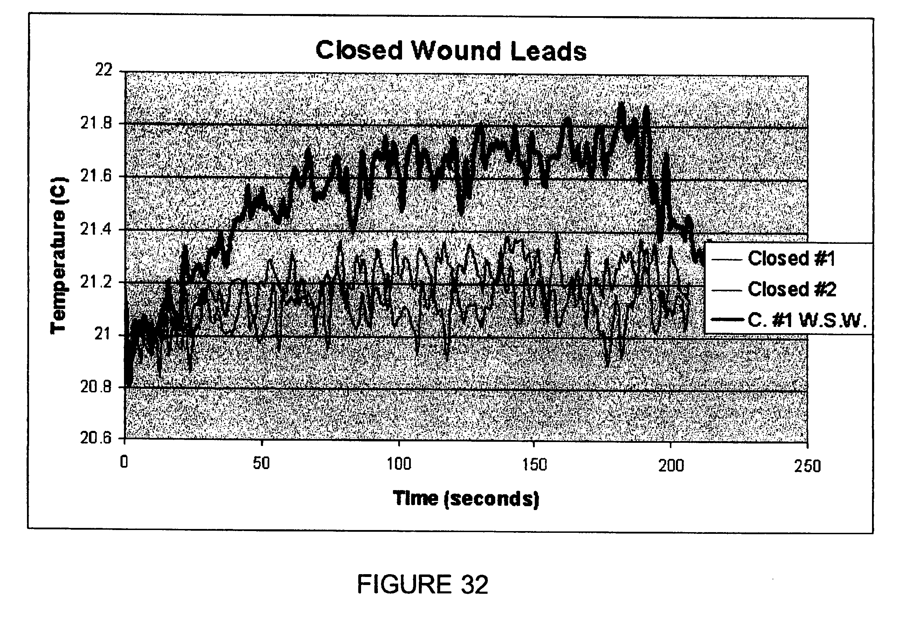

Figure 32 illustrates three pacing leads with closed windings wherein coil **C. #1 W.S.W.** has a single layer of polymer shrink wrap as illustrated in Figure 30. As illustrated in Figure 32, the temperature measured at the distal end of the pacing lead was between 21˚ Celsius and 21.8˚ Celsius.

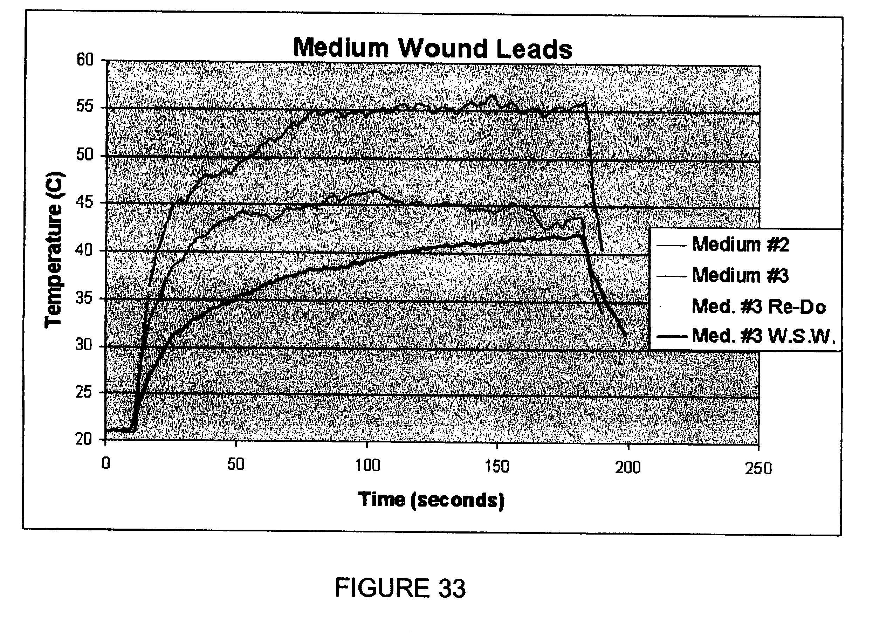

Figure 33 illustrates three pacing leads with medium-closed windings wherein coil **Med. #3 W.S.W.** has a single layer of polymer shrink wrap as illustrated in Figure 30. As illustrated in Figure 33, the temperature measured at the distal end of the pacing lead was between 40˚ Celsius and 55˚ Celsius.

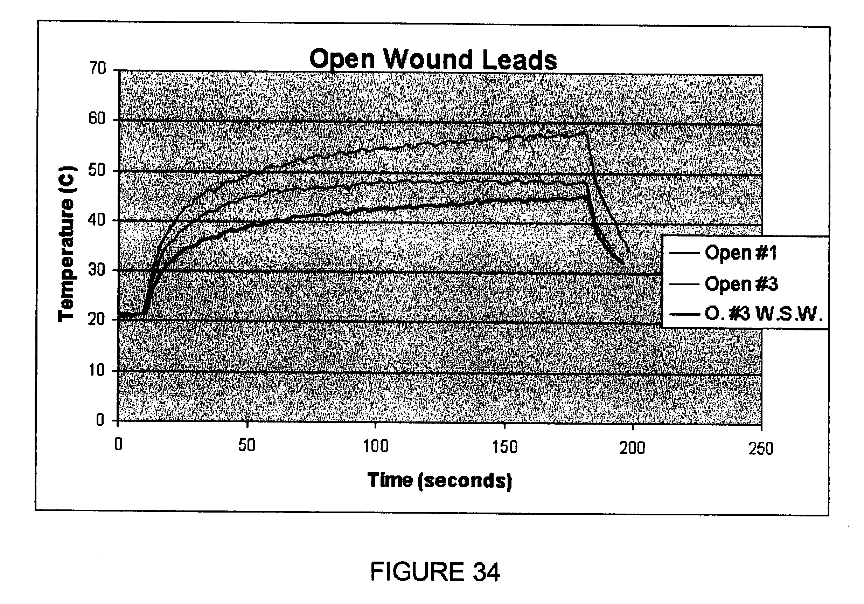

Figure 34 illustrates three pacing leads with open windings wherein coil **O. #3 W.S.W.** has a single layer of polymer shrink wrap as illustrated in Figure 30. As illustrated in Figure 34, the temperature measured at the distal end of the pacing lead was between 40˚ Celsius and 60˚ Celsius.

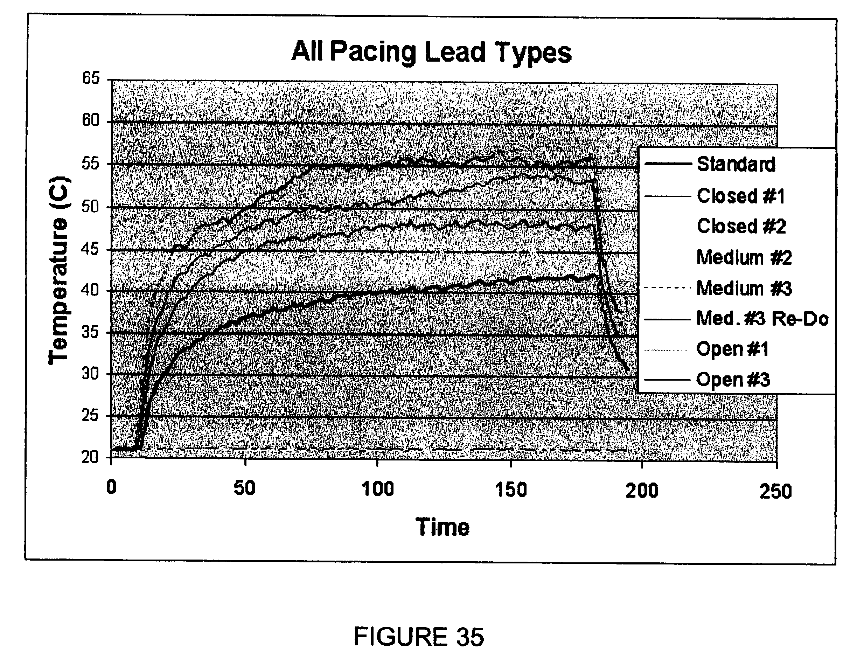

Figure 35 illustrates three pacing leads with open windings wherein coil **C. #1 W.S.W.** has a single layer of polymer shrink wrap as illustrated in Figure 29; three pacing leads with open windings wherein coil **Med. #3 W.S.W.** has a single layer of polymer shrink wrap as illustrated in Figure 29; and three pacing leads with closed windings wherein coil **O. #3 W.S.W.** has a single layer of polymer shrink wrap as illustrated in Figure 29. As illustrated in Figure 35, the temperature measured at the distal end of the pacing lead was between 20˚ Celsius and 60˚ Celsius.

In one embodiment, a possible solution to the pacing lead magnetic resonance imaging heating problem is to tune the coiled windings to have a self-resonance frequency close to or at the resonance frequency (RF frequency, operating frequency) of the magnetic resonance imaging scanner.

In one embodiment, another solution to the pacing lead magnetic resonance imaging heating problem is to have some portion of the long coiled wires include a self-resonance at the operating frequency of the magnetic resonance imaging scanner, preferably, close to the distal end of the coiled wire(s).

The self-resonance is formed by the coiled loops of the wire (providing an inductance) creating a distributive capacitance between adjacent loops, thus forming a RLC circuit with the capacitance in parallel with the resistor and inductor. The coiled wire can be tuned by changing the loop-to-loop spacing (the pitch of the coiled wire) or by changing the material between the loops (change the dielectric material forming the distributive capacitance.)

The capacitance (and inductance and resistance) of the coiled wire (or a portion of the coiled wire) can also be tuned to have a self-resonance close to or at the operating frequency of the magnetic resonance imaging scanner by changing the cross-sectional geometry of the wire used to form the coiled wire; i.e., a wire that has a square cross-section, rather than the typical round cross-section.

It is noted that pacemakers and other devices can create risks to their patients when exposed to magnetic resonance imaging by: excessive heating of the device (multiple causes) capable of producing uncontrolled tissue heating and thermogenic damage; induced voltages in the device that can interfere with organ function and device diagnostic and therapeutic capabilities; and/or magnetic resonance image disruption and distortion that prevents the visualization of tissues "close" to the device.

While it is relatively easy to demonstrate a heating or induced voltage problem, it is far more difficult to prove a solution to these problems, due to the complex and unpredictable nature, which includes factors such as: RF field strength; patient position in the coil; type of imaging sequence; patient characteristics; duration of imaging procedure; body structure being imaged; lead design; specific type of medical device; lead orientation within patient; the degree of perfusion near the device; temperature measurement procedure; and respiratory phase.

Magnetic resonance imaging energy is coupled into conductive leads in two ways, antennae effect and electrical potential induced within the body (implant acts as an electrical "short circuit"). High electrical current densities at the lead-tissue interface induce resistive heating in tissue. However, tissue heating can be substantially reduced by increasing the high frequency (i.e. 64MHz) electrical impedance of the lead.

In one embodiment, the magnetic resonance imaging scanner's frequency is fixed. Thus, the lead's self-resonance frequency should be shifted by changing coil inductance and capacitance properties.

More specifically, in one embodiment, changing the wire form design changes the capacitance-inductance characteristics of the lead and its impedance. Moreover, it is noted that adding a discrete component, high frequency resonator to the lead changes the capacitance-inductance characteristics of the lead and its impedance.

In one embodiment, lead design geometry has a strong influence on magnetic resonance imaging induced heating at 1.5 Tesla. Thus, the lead heating can be reduced to acceptable levels by properly choosing wire form design geometry or using discrete component resonator.

It is further noted that minimally disruptive lead design can reduce lead heating to acceptable levels. When implanted, these designs can provide a greater margin of patient safety and/or allow a greater number of patients access to magnetic resonance imaging. These designs can also be applied to other similar design conductive implants such as ICD and DBS leads, guidewires, catheters, etc.

In another embodiment of the present invention, a resonance tuning module is used in conjunction with implantable devices that incorporate one or more leads that may be subject to unwanted heating at the distal tip due to RF energy used in magnetic resonance imaging.

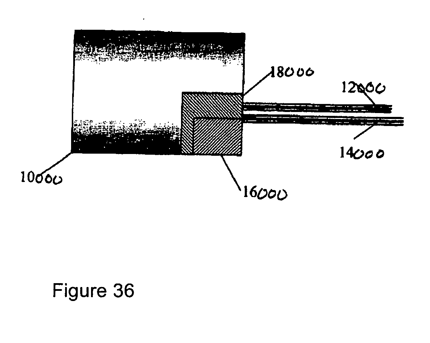

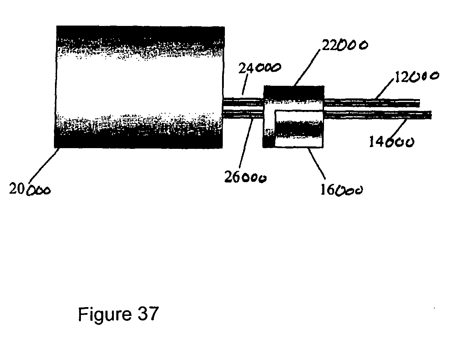

As illustrated Figures 36 and 37, Leads **12000** and/or **14000** may be single or multiple wires, and each may comprise one or more single filar or multifilar conductors, parallel or concentric, such as may be used in any of a variety of implantable devices used to sense conditions in the body and/or stimulate tissues in the body.

Device **10000** of Figure 36 (in this example a pacemaker) has an integrated resonance tuning module **18000** that in turn has a control and adjustment subsystem **16000;** as such it is a newly designed system requiring the kind of design/development cycle and regulatory approvals typical for a pacemaker. It will be obvious that device **10000** may be substituted for a pre-existing implanted pulse generator, while utilizing the existing implanted lead(s) thus not requiring explanation and replacement of the lead(s).

Device **20000** of Figure 37 is a standard "off-the-shelf" pacemaker that is connected via two short leads **24000** and **26000** to a standalone resonance tuning module implant **22000** that has a control and adjustment subsystem **16000** that is identical to that found in device **10000.** It will be obvious that device **22000** may be interposed between an existing implanted pulse generator and its existing implanted leads with a relatively minor surgical procedure.

It is known that if the overall pacemaker system is properly tuned so that the lead is "self-resonant" at the RF frequency of the magnetic resonance imaging system (e.g. 64MHz for a 1.5T system) heating at the distal end of the pacing lead will be significantly or completely eliminated.

It is also known that the resonant frequency of the pacemaker system is influenced by the design, materials, and construction of the lead(s), the path the lead(s) take(s) in the body, the electromagnetic characteristics of body tissues, the pulse generator lead(s) connect(s) to, and other factors. Thus it is impractical or impossible to create a single design for a pacing lead that will be properly self-resonant once implanted in the body as part of a pacing system.

The purpose of this invention is to provide for a module that is either integral to the pulse generator, or connected between it and the traditional multifilar lead, such that the overall system may be tuned to be self-resonant in spite of the variables described above.

Once the connections are made and the surgery is completed, part of the setup procedure for the system involves instructing the control and adjustment subsystem **16000** to iteratively test for the resonant frequency of the system and adjust inductive and/or capacitive elements within the resonance tuning module **18000** or **22000** to reach the desired resonant frequency and fix it permanently or until such time as it is desired to be readjusted (e.g. to 128 MHz for a 3.0 T system).

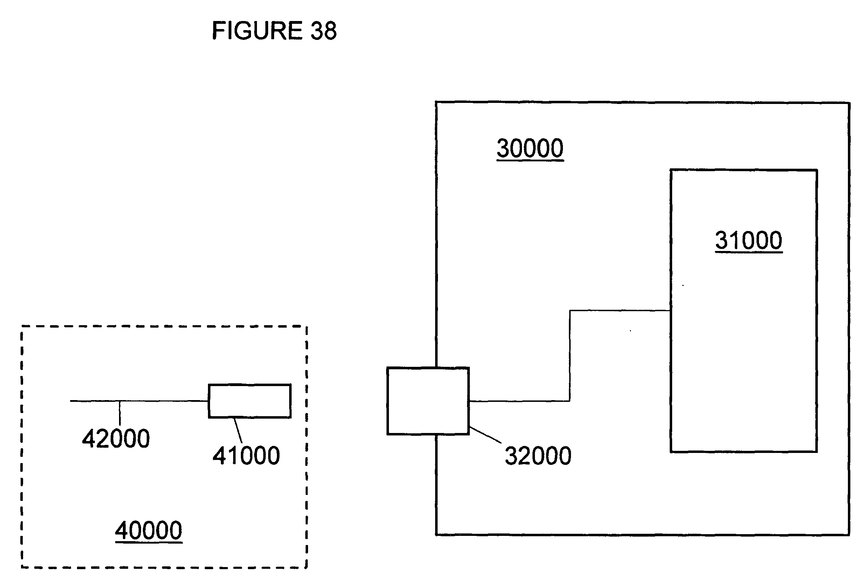

Figure 38 illustrates a conventional electronic device **30000** that includes a connection port **32000,** a circuit **31000,** and an electrical line that electrically connecting the connection port **32000** to the circuit **31000.** An assembly **40000** that includes a wire **42000** and a connector **41000** can be connected to the electronic device **30000** by connecting connector **41000** to connection port **32000.**

In one embodiment, assembly **40000** is a wire connection between an electrical device (not shown) and the electronic device **30000.** The wire **42000** may pickup ambient electrical signals from the surrounding environment. These signals may be intended to be picked up by assembly **40000** or these signals may be unintentionally picked up, i.e., noise, by assembly **40000.**

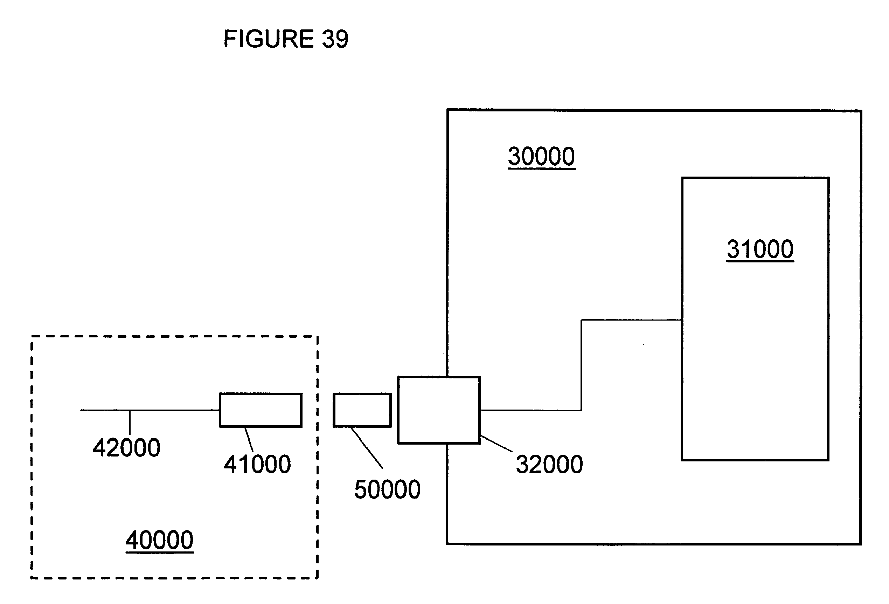

Figure 39 illustrates an insertion of resonant circuit adaptor **50000** suitable for connecting to connector **41000** and connecting to connection port **32000** which places a resonant circuit in series with electrical wire **42000** and circuit **31000.** In this embodiment, the insertion of the resonant circuit adaptor **50000** converts the assembly **40000** into an anti-antenna for the frequencies to which the resonant circuit adaptor **50000** is tuned. It is noted that the resonant circuit adaptor **50000** may be tuned to be equal to a frequency of the undesirable signals in the environment or the resonant circuit adaptor **50000** may be tuned to a frequency that takes into account the environment (in vitro) in which the assembly **40000** is located; i.e., the resonant frequency of resonant circuit adaptor **50000** may take into account the interaction of blood to the resonant frequency of the in vitro assembly **40000.**

The resonant circuit adaptor **50000** may also include multiple RLC resonant circuits in series, each tuned to a different frequency. Thus signals on the assembly **40000** of the frequencies, to which the adaptor **50000** is tuned, are significantly blocked from reaching the circuit **31000.**

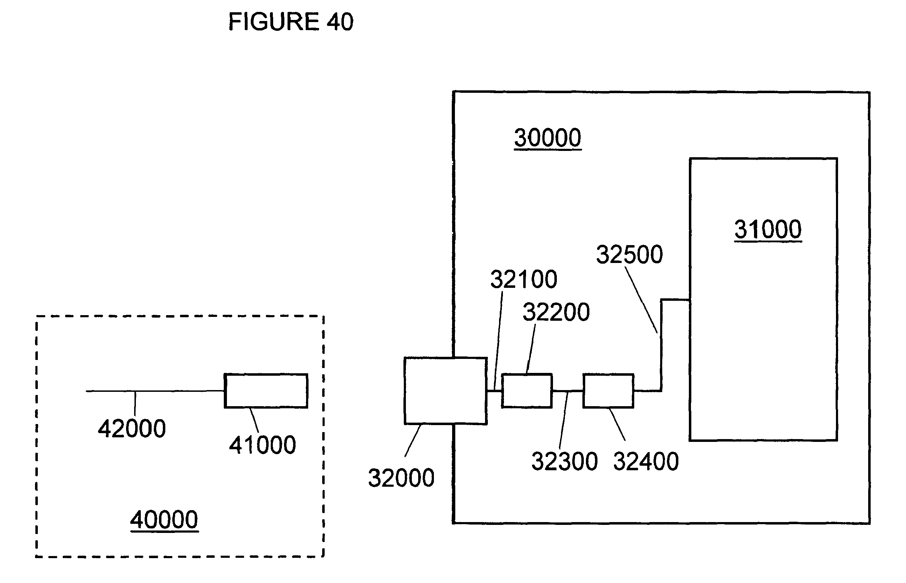

Figure 40 illustrates another electronic device **30000** that includes a connection port **32000** and a circuit **31000.** The electronic device **30000** further includes resonant circuits (**32200** and **32400**) and electrical lines (**32100, 32300,** and **32500**). The resonant circuits (**32200** and **32400**) are in series with connection port **32000** and circuit **31000.** It is noted that, in one embodiment, the resonant circuits (**32200** and **32400**) may be tuned to difference resonant frequencies.

When assembly **40000** is connected to connection port **32000,** the assembly **40000** plus resonant circuits (**32200** and **32400**) may act as an anti-antenna for the frequencies to which the resonant circuits (**32200** and **32400**) are tuned. Thus, signals, including intentional signals as well as noise, that match the frequency of the resonant circuits (**32200** and **32400**) which are picked up (or received) by the assembly **40000,** either intentionally or unintentionally, are significantly reduced from reaching circuit **31000.**

It is noted that the resonant circuits (**32200** and **32400**) may be tuned to be equal to frequencies of the undesirable signals in the environment or the resonant circuits (**32200** and **32400**) may be tuned to a frequency or frequencies that take into account the environment (in vitro) in which the assembly **40000** is located; i.e., the resonant frequencies of resonant circuits (**32200** and **32400**) may take into account the interaction of blood to the resonant frequency of the in vitro assembly **40000.**

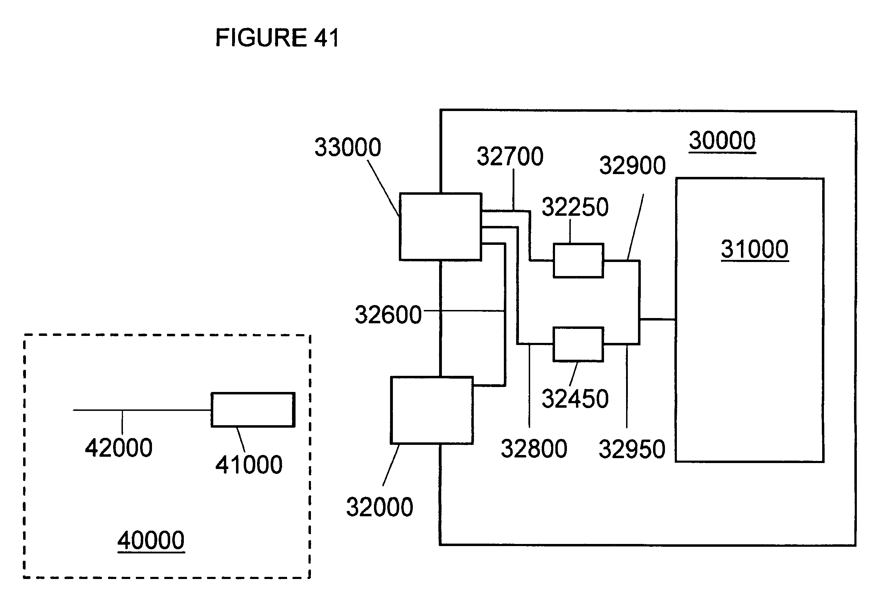

In another embodiment, as illustrated in Figure 41, an electronic device **30000** includes a circuit **31000,** a connection port **32000,** and a switch **33000.** The switch **33000,** which may be a manually operated switch or an automatic switch, selects which resonant circuit (**32250** or **32450**) is connected in series with the assembly **40000** and the circuit **31000.**

The resonant circuit **32450** is connected to the switch **33000** by electrical line **32800** and to the circuit **31000** by electrical line **32950.** The resonant circuit **32250** is connected to the switch **33000** by electrical line **32700** and to the circuit **31000** by electrical line **32900.** The connection port **32000** is connected to the switch **33000** via electrical line **32600.**

When the assembly **40000** is connected to the connection port **32000,** the assembly **40000** plus series resonant circuit **32450** or the assembly **40000** plus series resonant circuit **32250** forms an anti-antenna connected to the circuit **31000.** The anti-antenna significantly reduces signals at the resonant frequency of the resonant circuits (**32250** or **32450**) that are picked up by the assembly **40000.**

The switch **33000** can be operated to select different anti-antenna frequencies by connecting the different resonant circuits (**32250** or **32450** in series with assembly **40000** and circuit **31000.**

It is noted that the resonant circuits (**32250** and **32450**) may be tuned to be equal to frequencies of the undesirable signals in the environment or the resonant circuits (**32250** and **32450**) may be tuned to a frequency or frequencies that take into account the environment (in vitro) in which the assembly **40000** is located; i.e., the resonant frequencies of resonant circuits (**32250** and **32450**) may take into account the interaction of blood to the resonant frequency of the in vitro assembly **40000.**

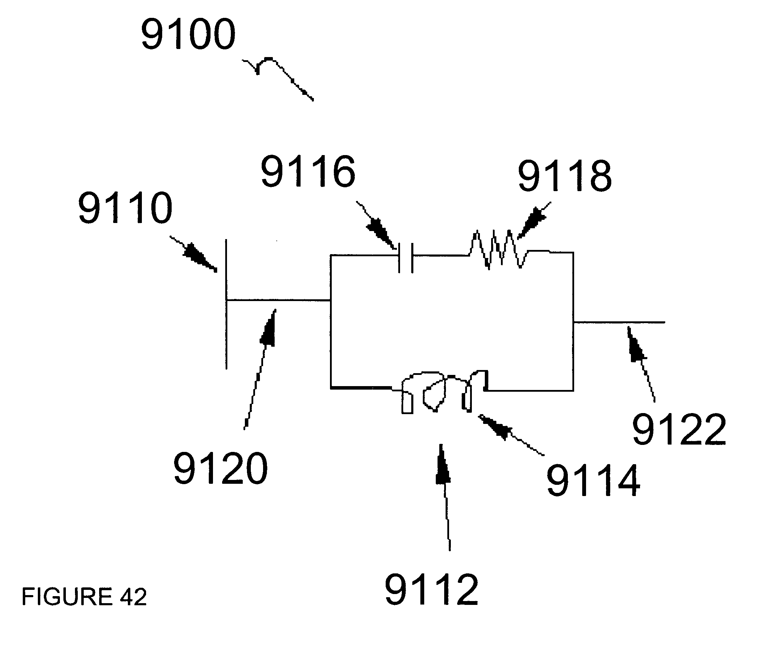

It is noted that the resistor-inductor-capacitor (RLC) resonance circuit of the present invention may be constructed as a substantially cylinder shaped component. Examples of a substantially cylinder shaped component are illustrated in Figures 42-48. The substantially cylinder shaped component may be cylinder shaped electrode, or an insert to be placed inline in a lead (pacing, DBS, pain relief, etc.).

The substantially cylinder shaped component may include an RLC circuit having a resonance frequency wherein the resonance frequency may be the resonance frequency of a magnetic resonance imaging scanner, a harmonic of a magnetic resonance imaging scanner frequency, or other appropriate tuned or detuned frequency. The substantially cylinder shaped component may also include a connection points to attach an external lead wire to the RLC resonance circuit wherein one connection point may be located on the "proximal" side. In the case where the component is not itself the electrode, an additional connection point may be located on the "distal" side of the component.

The substantially cylinder shaped component may further include through hole(s) or other channels or conduits for making electrical contact with other lead wires on both sides of the component. The substantially cylinder shaped component may include a device, system, or medium for carrying any heat away from the component, thereby providing a heat sink function.

Figure 42 provides a diagram of the electrical features of the substantially cylinder shaped component **9100.** In Figure 42, an RLC resonance circuit **9112** connected to an electrode **9110.** As illustrated in Figure 42, the RLC resonance circuit **9112** includes an inductor **9114** connected in parallel with a series circuit of a resistor **9118** and capacitor **9116.** It is noted that the actual configuration of the RLC resonance circuit **9112** is dependent upon the resonance or non-resonance specifications for the circuit. The RLC resonance circuit **9112** is connected to a lead **9122** and to the electrode **9110,** via lead **9120.**

In one embodiment, the substantially cylinder shaped component **9100** may include a low pass filter built rather than the RLC resonance circuit. In this embodiment, a "ground" connection point is to be provided in addition to the lead wire connections.

It is further noted that the electrode surface, associated with substantially cylinder shaped component **9100,** may be one "plate" of the capacitor **9116.**

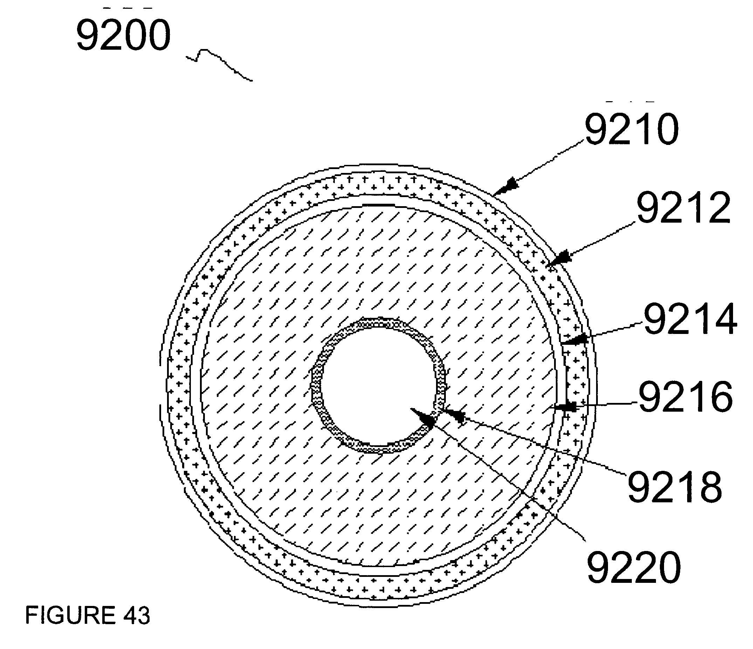

Figure 43 is one embodiment of a substantially cylinder shaped component implemented with a resonance circuit. As illustrated in Figure 43, a substantially cylinder shaped component **9200** includes an outer conductive cylinder electrode **9210,** a ring of dielectric material **9212,** an inner conductive ring **9214** forming the second capacitor plate, insulative wire **9216** wrapped to form an inductor, and a non-conductive ring spacer **9218** which forms a feed through channel allowing other coiled wire **9220** to pass through.

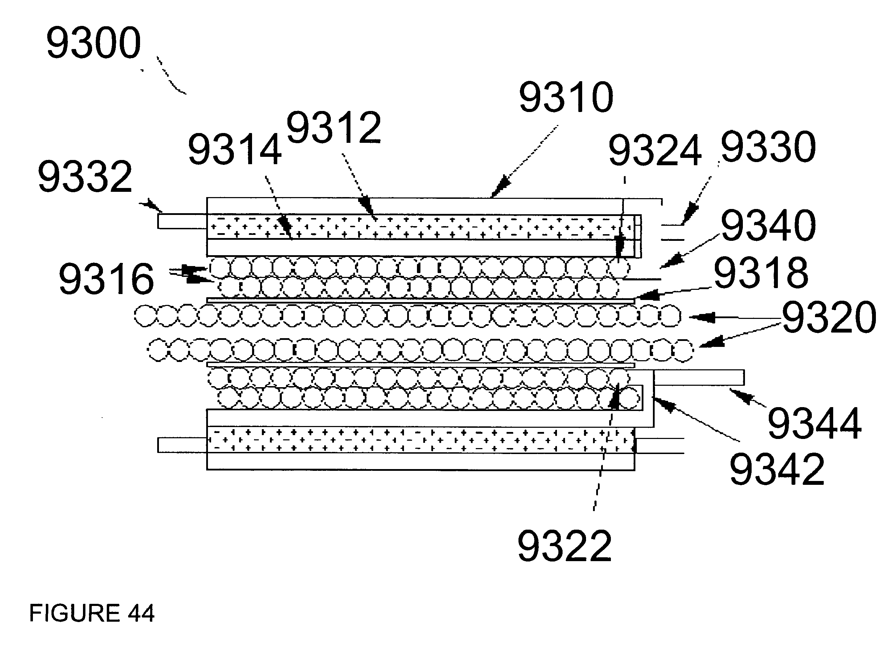

Figure 44 is a side view of an embodiment of a substantially cylinder shaped component implemented with a resonance circuit. As illustrated in Figure 44, a substantially cylinder shaped component **9300** includes an outer conductive cylinder electrode **9310,** a ring of dielectric material **9312,** an inner conductive ring **9314** forming the second capacitor plate, insulative wire wrapped to form an inductor **9316,** and a non-conductive ring spacer **9318** which forms a feed through channel allowing other coiled wire **9320** to pass through. A first end **9322** of the inductor coil **9316** is connected to the inner conductive ring **9314** by conductive connection element **9342.** This conductive connection element **9342** is also electrically connected to connection tab **9344,** which allows a wire to be electrically attached to the component **9300.** A second end **9324** of the inductor coil **9316** is connected to the outer cylinder conductor **9310** by conductive connection element **9340.** Tabs **9330** and **9332** are used, for example, to attach a polymer jacket tube (not shown) to each end of the component **9300.** The polymer jacket tube may be, e.g., the outer polymer jacket of a pacing lead, or the outer polymer jacket of a deep brain stimulation lead, etc.

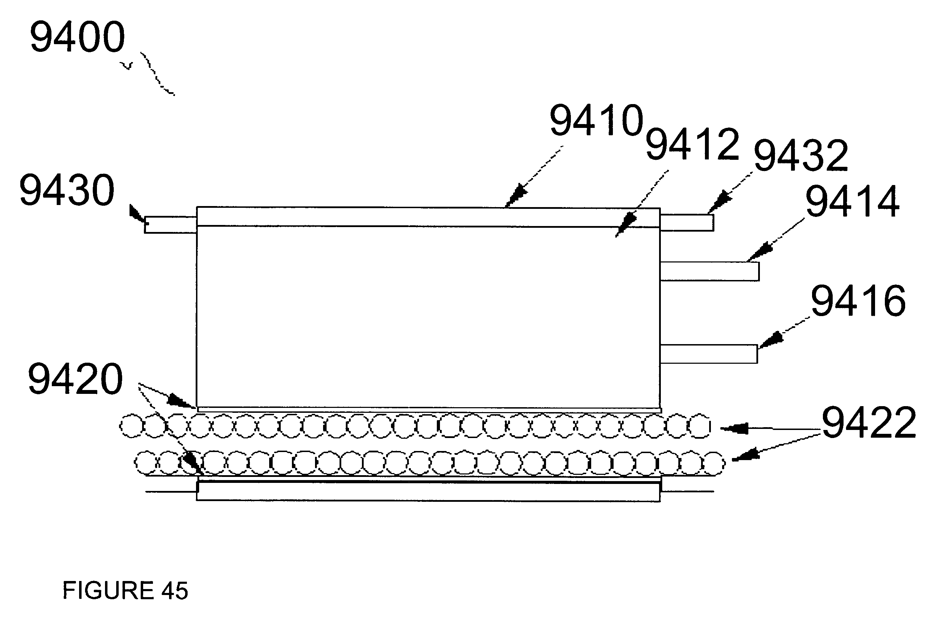

Figure 45 is a side view of another embodiment of a substantially cylinder shaped component. As illustrated in Figure 45, a substantially cylinder shaped electrode **9400** comprising an outer cylinder conductive electrode **9410,** a volume **9412** in which a low pass filter (not shown) is implemented. The volume **9412** is electrically connected to the outer electrode **9410,** connection tab **9414** for connecting to one of the lead's conductive wires, connection tab **9416** for connecting to the ground wire in the lead, and a feed through **9420,** through which other lead wires **9422** may pass through the component **9400.** Tabs **9430** and **9432** are used, for example, to attach a polymer jacket tube (not shown) to each end of the component **9400.** The polymer jacket tube may be, e.g., the outer polymer jacket of a pacing lead, or the outer polymer jacket of a deep brain stimulation lead, etc.

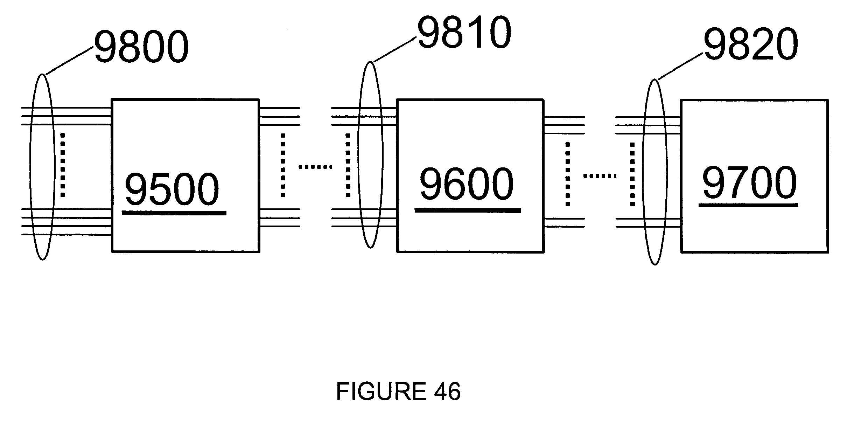

Figure 46 illustrates a plurality of substantially cylinder shaped component connected along a medical device, such as a guidewire, pacing lead, or deep brain stimulation lead. As illustrated in Figure 46, a plurality of electrical wires **9800** engage a proximal side of a substantially cylinder shaped component **9500.** The substantially cylinder shaped component **9500** may be an actual electrode or tissue interface, associated with an electrode or tissue interface, or electronic component with no electrode or tissue interface.

It is further noted that the substantially cylinder shaped component may be located anywhere along a lead's length. In other words, it is also noted that the RLC or LC circuit, contained within the substantially cylinder shaped component, may be contained in another type housing and may be located anywhere along a lead's length.

Moreover, it is noted that any number of substantially cylinder shaped components may be associated with a single lead. Furthermore, it is noted that any number of RLC or LC circuits, contained within the substantially cylinder shaped component, may be contained in another type housing and may be associated with a single lead. In this example, the different RLC or LC circuits may be tuned to the same frequency, different frequencies, or any combination thereof.

A plurality of electrical wires **9810** engage a proximal side of a substantially cylinder shaped component **9600** and a distal side of the substantially cylinder shaped component **9500.** The substantially cylinder shaped component **9600** may be an actual electrode or tissue interface, associated with an electrode or tissue interface, or electronic component with no electrode or tissue interface.

A plurality of electrical wires **9820** engage a proximal side of a substantially cylinder shaped component **9700** and a distal side of the substantially cylinder shaped component **9600.** The substantially cylinder shaped component **9700** may be an actual electrode or tissue interface, associated with an electrode or tissue interface, or electronic component with no electrode or tissue interface.

Depending upon the functionality of the substantially cylinder shaped components **9500, 9600,** and **9700,** all the wires or a portion of the wires may pass therethrough.

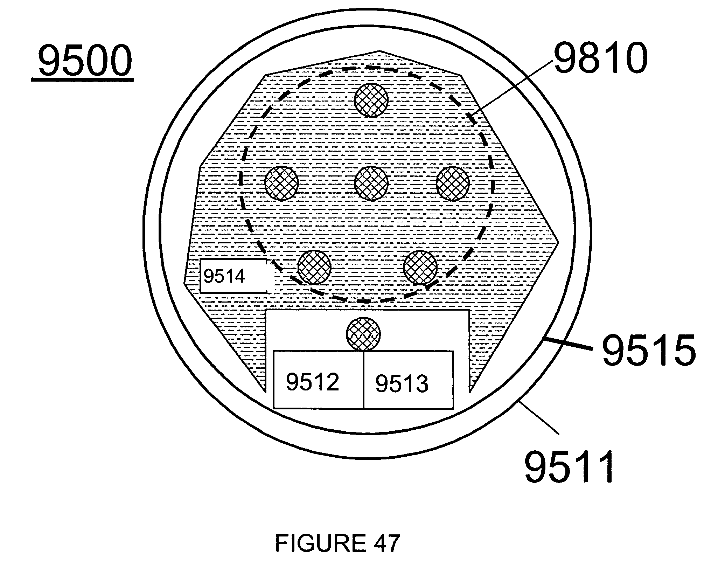

Figure 47 is a cut (or end) view of one embodiment of a substantially cylinder shaped component **9500** implemented with a resonance circuit. As illustrated in Figure 47, a substantially cylinder shaped component **9500** includes an outer conductive cylinder electrode **9511,** a ring of dielectric material between an inner conductive ring **9515** and the outer conductive cylinder electrode **9511** to form a capacitor. The substantially cylinder shaped component **9500** further includes an inductor **9512** and a resistor **9513** connected to one of the wires from the plurality of electrical wires **9800.** The remaining wires, a plurality of electrical wires **9810,** pass through a volume **9514.**

The volume **9514** may be a polymer or other substance with predefined channels for the plurality of electrical wires **9810.** The channels may form a predefined pattern so as to reduce or eliminate electrical interference or cross-talk.

Moreover, volume **9514** may be filled with a polymer or other substance after the plurality of electrical wires **9810** is located therein. In this embodiment, the volume may have a skeletal structure to provide a predefined pattern for the plurality of electrical wires **9810.**

Furthermore, the volume **9514** may be filled with a polymer or other substance that provides heat sink functionality for the inductor **9512** and resistor **9513** circuit.

It is noted that the substantially cylinder shaped component **9500** of Figure 47 may include a low pass filter instead of the RLC resonance circuit. IN this embodiment, the inductor **9512** and resistor **9513** are replaced with a low pass filter and the conductive rings are replaced with non-conductive components.

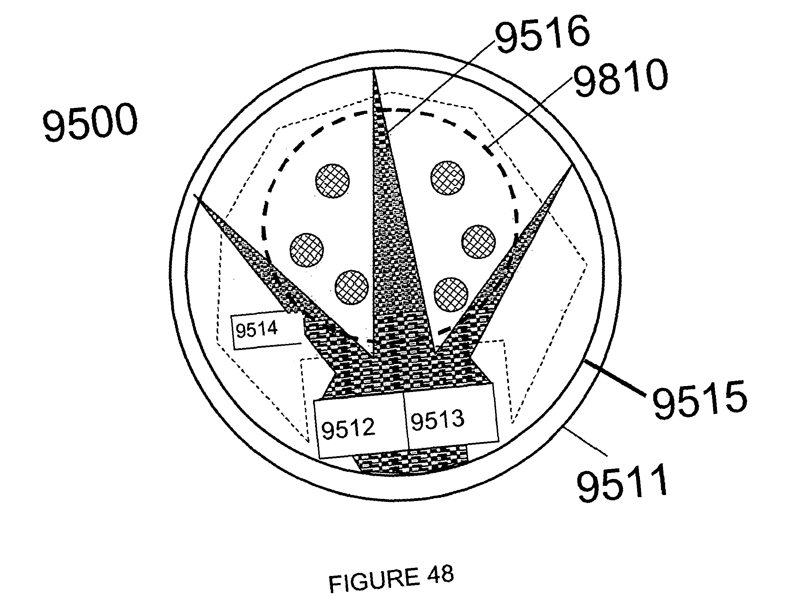

Figure 48 is a cut (or end)view of another embodiment of a substantially cylinder shaped component **9500** implemented with a resonance circuit. As illustrated in Figure 48, a substantially cylinder shaped component **9500** includes an outer conductive cylinder electrode **9511,** a ring of dielectric material between an inner conductive ring **9515** and the outer conductive cylinder electrode **9511** to form a capacitor. The substantially cylinder shaped component **9500** further includes an inductor **9512** and a resistor **9513** connected to one of the wires from the plurality of electrical wires **9800.** The remaining wires, a plurality of electrical wires **9810,** pass through a volume **9514.**

The volume **9514** may be a polymer or other substance with predefined channels for the plurality of electrical wires **9810.** The channels may form a predefined pattern to reduce or eliminate electrical interference or cross-talk.

Moreover, volume **9514** may be filled with a polymer or other substance after the plurality of electrical wires **9810** is located therein. In this embodiment, the volume may have a skeletal structure to provide a predefined pattern for the plurality of electrical wires **9810.**

Furthermore, the volume **9514** may include a sub-volume **9516,** which can be filled with a polymer or other substance that provides heat sink functionality for the inductor **9512** and resistor **9513** circuit.

It is noted that the substantially cylinder shaped component **9500** of Figure 48 may include a low pass filter instead of the RLC resonance circuit. IN this embodiment, the inductor **9512** and resistor **9513** are replaced with a low pass filter and the conductive rings are replaced with non-conductive components.

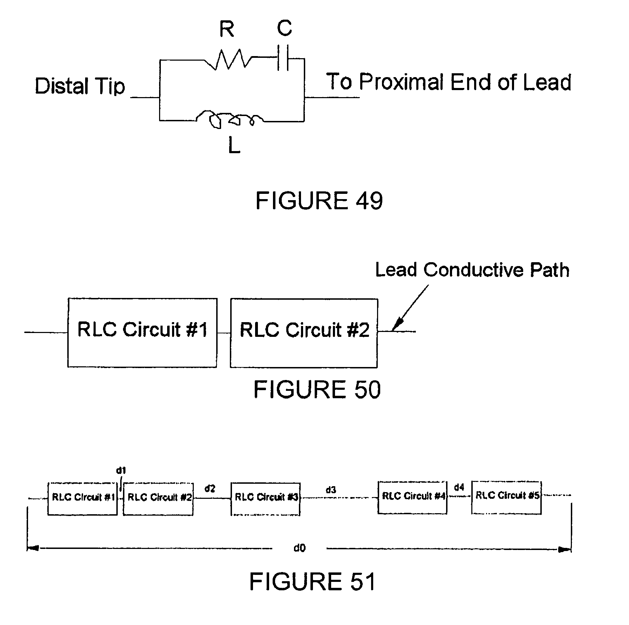

Figure 49 illustrates a lead with an RLC circuit at distal tip tuned to desired resonant frequency. In this embodiment, a single conductive path through lead, which can be a multi-filar conductor, is illustrated, but it is understood that a RLC circuit can be applied to each conductive path in the lead.

Moreover, it is noted that the tuning need not be perfect and that the tuning is with completed lead in blood (or blood substitute) rather than in air. Furthermore, it is noted that the resistor **R** reduces the current maximum in the resonant circuit that helps protect the inductor L from being damaged by too high a current. In addition, it is noted that the resistor **R** and the inductor L may be thermally connected to heat sinks to remove or distribute or limit the amount of heat that may build up in these components.

Figure 50 illustrates a lead with multiple RLC circuits at distal end of lead, in series with one another each RLC circuit tuned to block a different RF frequency, e.g. 63.8 MHz and 127.6 MHz. In this embodiment, a single conductive path through lead, which can be a multi-filar conductor, is illustrated, but it is understood that a RLC circuit can be applied to each conductive path in the lead.

Moreover, it is noted that the tuning need not be perfect and that the tuning is with completed lead in blood (or blood substitute) rather than in air. Furthermore, it is noted that the resistor **R** reduces the current maximum in the resonant circuit, which helps protect the inductor **L** from being damaged by too high a current. In addition, it is noted that the resistor **R** and the inductor L may be thermally connected to heat sinks to remove or distribute or limit the amount of heat that may build up in these components.

As illustrated in Figure 51, the lead with multiple RLC circuits positioned along the leads length. At least one at the distal end of the lead tuned to approximately the same resonant frequency. In this way, **circuit #1** of Figure 51 might reduce the induced current at the distal tip/tissue interface by 50% of the induced current that occurs without circuits, while **circuit #2** of Figure 51 may reduce the remaining induced current by another 50% resulting in a total of 75% reduction.

Additionally, multiple circuits distributed along the length of the lead reduce the amplitude of the induced current that each circuit alone would have to handle, thereby reducing the possibility that the induced current will exceed the component's rating.

As noted above, an example of multiple circuits distributed along the length of the lead to reduce the amplitude of the induced current that each circuit alone would have to handle is illustrated by Figure 51, which shows five RLC circuits separated by different intervals **d1, d2, d3, d4** along the length **d0** of the pacing lead. In other words, the lead is polyfurcated in that the lead is broken into multiple sections such the lead may not be necessarily continuous.

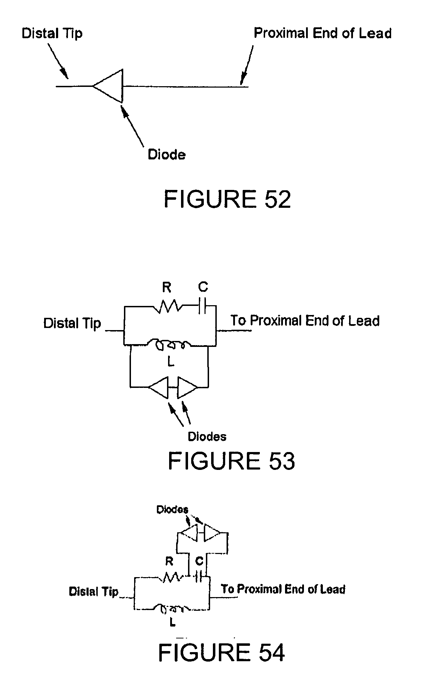

As illustrated in Figure 52, a diode is placed at the distal end of the lead. The diode blocks any induced current from running from the distal tip toward the proximal end of the lead; however, the diode does not prevent a pacing pulse (or induced currents) from traveling from the proximal end of the lead to the distal end of the lead. Therefore, the diode blocks 1/2 of the magnetic resonance imaging RF induced current from passing through the tip/tissue interface reducing the heating that occurs.

It is noted that one or more diodes can be utilized to prevent the current in the resonant circuit from exceeding the rating of the inductor or the voltage from exceeding the rating of the capacitor.

More specifically, as illustrated in Figure 53, if the voltage across the inductor is such that the inductor's rating would be exceeded, the diode's breakdown threshold would also be exceeded, thereby providing an alternative path around the inductor such that the inductor is protected. Figure 53 illustrates an RLC circuit as illustrated in Figure 49, wherein two diodes are connected back to back across the inductor **L.**

In a similar situation, Figure 54 illustrates an RLC circuit as illustrated in Figure 49, wherein two diodes are connected back to back across the capacitor **C.** If the voltage across the capacitor **C** is such that the capacitor's rating would be exceeded, the diode's breakdown threshold would also be exceeded, thereby providing an alternative path around the capacitor C such that the capacitor C is protected.

It is noted that current limiting diodes or constant current diodes may be used to limit the current in the resonant circuit or in the lead conductors itself.

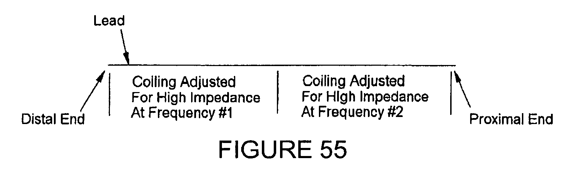

It is further noted that when the lead wire is a coiled wire, the coiling (pitch) of the wire and any insulating coating between loops of the wire are altered to adjust the self-resonance of the wire to be the desired magnetic resonance imaging operating frequency. In other words, the impedance of the wire is adjusted to be large at the operating frequency of the magnetic resonance imaging scanner. In this embodiment, the coiled wire of the lead is considered to be divided into two lengths, as illustrated in Figure 55.

As illustrated in Figure 55, the first half of the length of the coiled wire may be adjusted (coiling pitch, insulating-dielectric material spacers, other) to have a high impedance (self resonance) at the operating frequency of a first magnetic resonance imaging scanner, while the second half of the length of the lead is adjusted to have a high impedance (self resonance) at the operating frequency of a second magnetic resonance imaging scanner. Thus, the lead has high impedance at two different frequencies, for example 64 and 128 MHz.

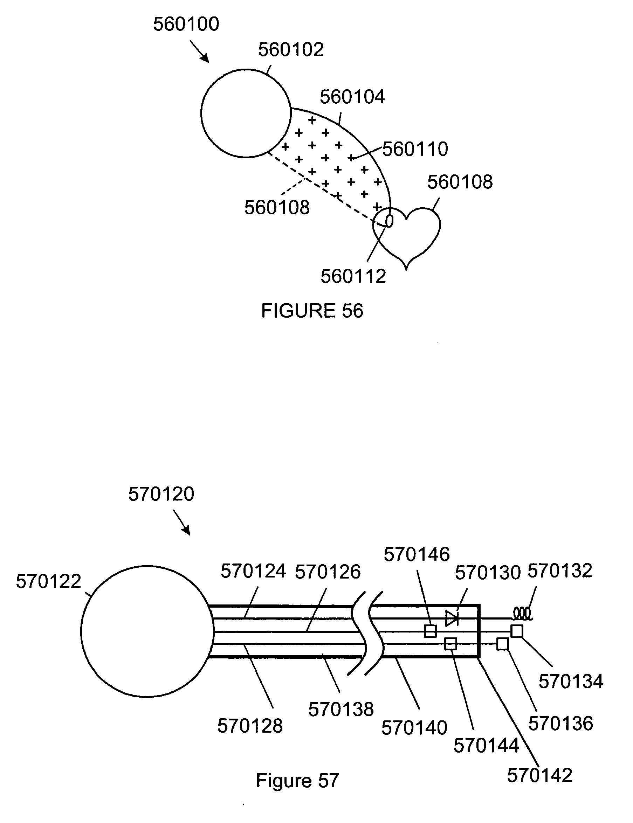

Figure 56 is a schematic showing a typical pacemaker arrangement **560100**. The pacemaker comprises a pulse generator canister **560102** housing a power supply (not shown) and electronic components (not shown) for sensing and producing electrical pacing pulses. The pulse generator canister **560102** has connected to it insulated conductive leads **560104** that pass through the body (not shown) and into the heart **560106.** Conventional bipolar pacemaker leads have two conductive strands, one for pacing and sensing, and the other for ground. The path of the leads **560104** is generally not straight. The leads **560104** have one or more electrodes **560112** in contact with the heart **560106.** The path **560108** from the heart **560106,** where the electrodes **560112** are placed, to the generator canister **560102** represents a conductive path comprising body tissue (not shown) and fluids (not shown). The completed loop from the pacemaker canister **560102,** through the leads **560104,** and back to the pacemaker canister **560102** along the path **560108** is subject to Lenz's law. That is, a changing magnetic field **560110** through the area enclosed by the completed loop (from the pacemaker canister **560102,** through the leads **560104,** and back to the pacemaker canister **560102** along the path **560108)** can induce unwanted voltages in the leads **560104** and across the heart **560106.**

In one embodiment of the present invention, and referring to Figure 56, the pacemaker canister **560102** is made out of a non-conductive material. In another embodiment, the canister **560102** is coated or covered with various non-conductive insulating materials. This increases the overall resistance of the conductive path loop and thus reduces the voltage across the tissue between electrodes **560112** and the canister **560102.**

Using a three-strand lead design allows for the separation of the pacing signals from the sensing signals and allows for different filtering techniques to be utilized on each separate conductive strand: one strand for the pacing signal for stimulating the heart, one conductive strand for the sensing of the heart's electrical state, pre-pulse, ecg, etc., and one strand for the ground path. Current bi-polar designs use only two conductive strands. This means that the pacing and the sensing signals are carried on the same strand.

For example, in conventional bipolar pacemaker leads, the pacing signal goes "down" (from generator canister to heart) the pacing lead (conductive strand) while the sensing signal travels "up" (from heart to generator canister) the pacing lead. This is the "standard" bipolar pacing setup. If a filter is added to the pacing/sensing strand to block the switch gradient induced signal caused by a magnetic resonance imaging system, the pacing pulse/signal must travel through the filter, thereby distorting the pacing pulse.

According to the concepts of the present invention, by adding a third conductive strand, a diode, for example, can be put on the pacing strand and one or more filters can be put on the sensing strand. The filters on the sensing lead may be at the distal end of the pacemaker lead or in the generator canister. Thus, by using separate strands, the present invention is able to utilize different kinds of filters (radio-frequency filters, high/low pass filters, notch filters, etc.) or other electronics in conjunction with each strand depending on the different signal characteristics and/or signal direction along the conductive strand.

Figure 57 shows a schematic of a pacemaker arrangement **570120** including a generator canister **570122** containing a pacing pulse generator (not shown), sensing electronics (not shown) and other electronic components (not shown). Attached to the generator canister **570122** is a lead assembly **570140** having three conductive strands **570124, 570126,** and **570128** through lumen **570138.** Each of the conductive strands **570124, 570126,** and **570128** pass through the distal tip **570142** of the lead assembly **570140** to exposed electrodes **570132, 570134,** and **570136,** respectively. The exposed electrodes **570132, 570134,** and **570136** are placed in contact with or next to the heart.

Conductive strand **570124** and electrode **570132** are used to deliver pulses to the heart from a pacing generator within the canister **570122.** Conductive strand **570126** and electrode **570134** are used as a ground. Conductive strand **570128** and electrode **570136** are utilized for sensing the electrical signals generated by the heart. In this way, the sensing functionality of pacemakers can be separated from the delivery of pacing pulses.

To block any induced voltage signals from the magnetic resonance imaging system's changing magnetic fields (the radio-frequency or the gradient fields) from propagating along the conductive pulse delivery strand **570124,** a diode 570130 is inserted into the conductive strand **570124** near the distal tip of the lead assembly **570142.** In another embodiment (not shown), the diode **570130** is placed in the generating canister **570122.**

With respect to Figure 57, other electronic components (i.e. radio-frequency chocks, notch filters, etc.) may be placed into the other conductive strands **570126** and **570128** shown as by components **570146** and **570144,** respectively. In another embodiment (not shown), these optional electronic components **570146** and **570144** can be placed in the generator canister **570122.**

Optional electronic components **570146** and **570144** are used to block or significantly reduce any unwanted induced signals caused by the magnetic resonance imaging system from passing along conductive strands **570126** and **570128** respectively while allowing the desired sensing signals from the heart to pass along conductive strand **570126** to electronics in the generator canister **570122.**

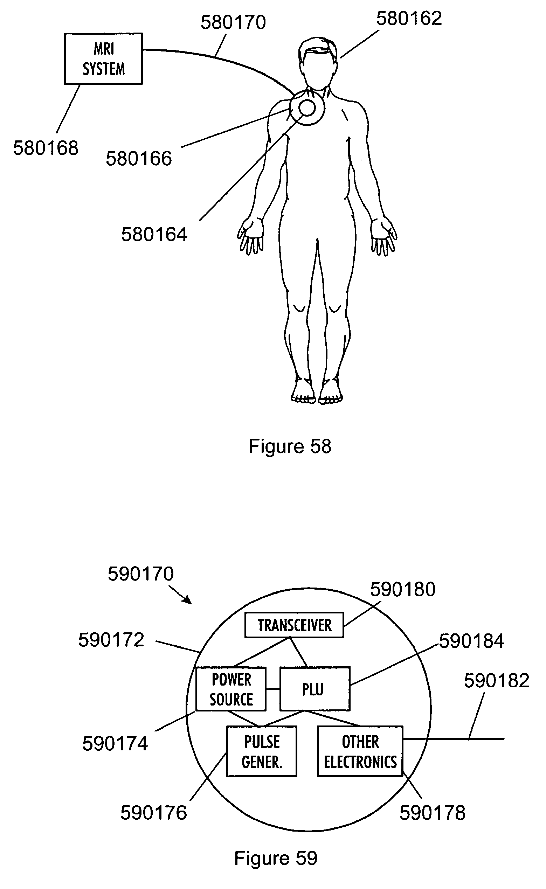

Figure 58 is a schematic of an embodiment of the present invention. As illustrated in Figure 58, a patient **580162** is located within a magnetic resonance imaging system **580168,** wherein the patient **580162** has an implanted heart pacemaker pulse generator canister **580164.** A surface sensor/transceiver **580166** is placed on the exterior of the patient's body **580162** over or near the location of the implanted pacemaker generator **580164.** The sensor/transceiver **580166** is in communication with the magnetic resonance imaging system **580168** via communication line **580170,** which may be a magnetic resonance imaging safe cable such as a fiber optical cable. Additionally, the sensor/transceiver **580166** is in communication with the implanted pacemaker pulse generator canister **580164.** The means of communication between the sensor/transceiver **580166** and the implanted pacemaker generator **580164** may be acoustic, optical, or other means that do not interfere with the imaging capabilities or image quality of the magnetic resonance imaging system. The signals may be digital or analog.

Moreover, with respect to this embodiment of the present invention, a transmitter/receiver is placed in the pacemaker canister **580164** so that the magnetic resonance imaging system **580168** can be in operative communication with the pacemaker system and vice versa. Thus, the pacing system can transmit signals to the magnetic resonance imaging system **580168** indicating when the pacemaker is about to deliver a pacing pulse to the heart. The transmitted signals may be digital or analog. In response to this transmitted signal, the magnetic resonance imaging system **580168** stops or pauses the magnetic resonance imaging switched gradient field (imaging scanning sequence) to allow the pacing pulse to occur. After the pacing pulse has been delivered to the heart, the magnetic resonance imaging system **580168** resumes or begins a new imaging scanning sequence.

In another mode of operation, the magnetic resonance imaging system **580168** sends signals to the implanted heart pacemaker pulse generator canister **580164** through the sensor/transceiver **580166** indicating the application of switched gradient fields. The pacemaker may use this information to switch filters or other electronics in and out of the circuit to reduce or eliminate voltages induced in the pacemaker leads by the gradient fields. For example, the pacemaker may switch in additional resistance or inductance or impedance into the pacing/sensing and/or ground strands based on the signal from the magnetic resonance imaging system **580168** signifying the application of the gradient fields.

In another configuration, there is no surface sensor/transceiver or communication line to the magnetic resonance imaging system **580168.** Instead, there is a special sensor in the implanted heart pacemaker pulse generator canister **580164** that can sense the application of the gradient fields. In response thereof, the pacemaker switches into the electrical circuit of the pacing/sense and/or ground leads a charging source which is used to charge the implanted heart pacemaker pulse generator canister **580164,** leads, and/or electrodes to an electrical potential opposite to that which would be induced by the gradient fields. In this way, the induced voltages caused by the gradient fields are cancelled out or reduced to a safe level, by the application of this voltage source.

In a preferred embodiment of the present invention, the charging/voltage source receives its power from inductively coupling to the magnetic resonance imaging system's radio-frequency field. The oscillating radio-frequency field supplies power to charge special capacitors in the implanted heart pacemaker pulse generator canister **580164.** It is noted that other external power sources can be used to power the charging/voltage source in the implanted heart pacemaker pulse generator canister **580164.**

Figure 59 is a diagram of an assembly **590170** for the pacemaker generator components comprising the canister housing **590172,** a programmable logic unit (PLU) **590184,** a power source **590174,** and a pulse generator **590176.** Additionally, means for communicating with an external sensor/transceiver is provided by transceiver **590180.** Other electronic components **590178;** e.g., signal filters, signal processors, lead connectors, etc. are also located in the canister **590172.** The pacing leads **590182** pass through the canister **590172** and connect to the internal electronics **590178.** During a magnetic resonance imaging examination, the signals transmitted and received by the transceiver **590180** may be used to synchronize the magnetic resonance imaging system's scanning sequences with the delivery of the pacing signals.

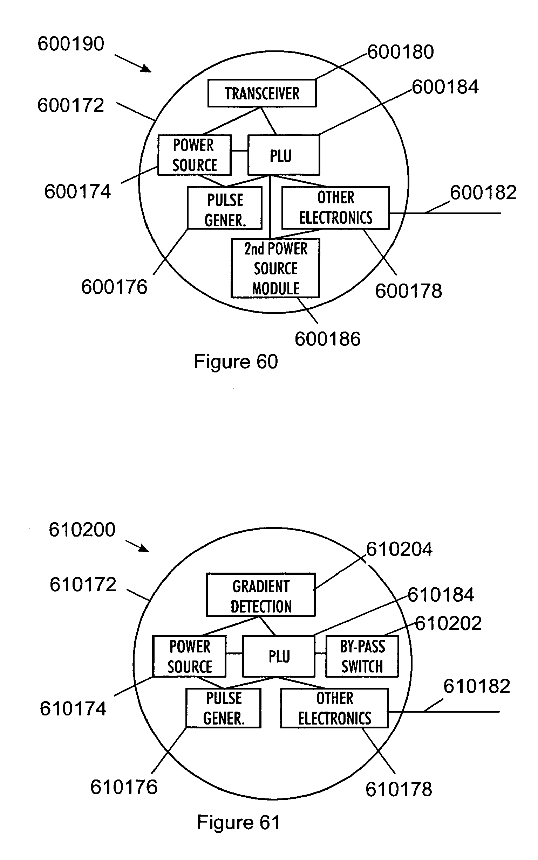

In another embodiment, as depicted in Figure 60, the pacing generator assembly **600190** further includes a second power module **600186** which may be an inductive coil and/or capacitor bank, suitable for capturing and storing power from the magnetic resonance imaging system's transmitted radio-frequency signal.

In one embodiment, the power stored in the power module **600186** is used to develop an electrical potential in the leads **600182** that is opposed to that which is induced by the application of the magnetic resonance imaging system's gradient fields.

In another embodiment, the power stored in the power module **600186** is used to operate various switches in the electronics module **600178** which may switch in or out various power serge protection circuits in-line and/or signal filters to the leads **600182.**

In a further embodiment, and referring to Figure 60, the module **600186** may be used to electrically charge the pacemaker canister **600172,** which is made of a conductive material, in synchronization with the application of the magnetic resonance imaging system's gradient fields so that the electrical potential difference between the pacing electrodes and the pacemaker canister **600172** is reduced. That is, the sum of the induced voltage difference due to the application of the gradient fields plus the voltage difference due to the application of the electrical charge stored in the power module **600186** results is a net voltage significantly below any threshold level, above which a problem may develop.

Figure 61 depicts another assembly **610200,** which includes the basic components of Figure 60 less the transceiver **610180,** a gradient field detector **610204,** and a by-pass switch component **610202.** By detecting the gradient signal in the pacemaker canister **610172** with gradient field detector **610204,** the pacemaker can switch filters and/or other electronics **610178** in or out of the circuit.

In one embodiment, when no gradient fields are detected, the switch **610202** is closed to by-pass the electronics component **610178,** which may be a combination of low-pass, high-pass, notch filters, diodes, and/or other electronics. In this mode (switched closed), the pacing pulse (and sensing signals) by-pass the filters components **610178.** When gradient field detector **610204** detects the gradient signals, the switch **610202** is opened and any gradient fields induced signals in the leads **610182** are blocked or significantly reduced by the filters components **610178.** In the open mode, the pacing and sensing signals pass through the filters component **610178** as well.

The gradient detector **610204** may communicate the sensing of the gradient field to other components in the pacemaker via its connection to the PLU **610184** so that the pacing signal can be modified, if necessary, to compensate for any distortion it may suffer by now going through the filters component **610178.** Additionally, the sensing signal, now also passing through the filter components **610178** may be distorted. This may be compensated for by including signal recovery/reconstruction logic into the PLU or into a separate signal-processing component.

Referring back to Figure 56, by increasing the impedance of the leads **560104,** the voltage across the tissue gap or spacing from the electrodes **560112** and the pacemaker canister **560102** can be reduced. Inserting a resistor or using a higher resistive wire for the pacemaker leads **560104** will reduce the current induced in the current loop, which includes the virtual loop portion across the (heart **560112**) tissue to the pacemaker generator canister **560102.**

By using various inductors in-line with the various leads **560104,** it is possible to make the leads **560104** have high impedance for the low frequency magnetic resonance imaging gradient fields' frequency and low impedance for the magnetic resonance imaging system's radio-frequency frequency. Alternatively, different impedances (inductors/resistors/capacitors) may be switched in-line or out of the leads' circuitry depending on the timing and application of the gradient and/or radio-frequency fields.

In another embodiment, not shown, the pacemakers' electronics can be augmented to include one or more digital signal processors. By converting the sensing signal into a digital signal, the digital signal processor (DSP) can reconstruct the sensing signal after it has passed through filters and has been distorted by the filtering or other elements that may have been added to the lead circuit. The DSP may also be used to reject any signals that do not have a correct cardiac signature, thus rejecting any signals caused by the switched gradient fields, which is a non-cardiac signal.

In another embodiment of the present invention, a pacemaker lead or other medical device, having a long conductive lead and functioning in a magnetic resonance imaging environment, may be configured, according to the concepts of the present invention, to include additional loops to cancel the induced voltage effects in the leads of the original current loop formed by the leads.

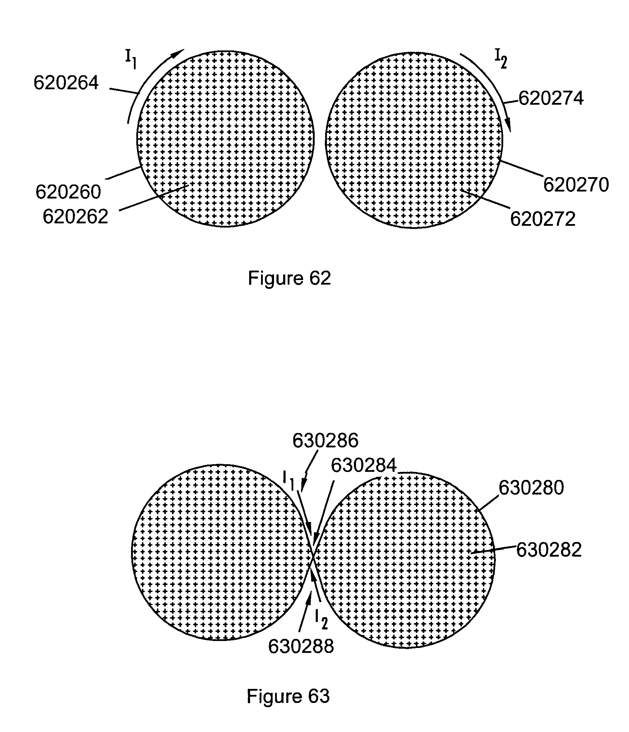

In Figure 62, two conductive loops **620260** and **620270** having the same amount of area and in the same plane, positioned in a changing magnetic field **620262** and **620272,** develop currents **620264** and **620274.** In Figure 62, both induced currents $I_1$ and $I_2$ travel in the same direction (clockwise direction shown) at all times as the magnetic field **620262** and **620272** oscillate.

Figure 63 shows that by connecting the two conductive loops **620260** and **620270** of Figure 62 to form a single conductor **630280,** the currents induced in each lobe can be made to cancel each other out. The two loops are connected so that a single conductor is formed which crosses over itself at **630284.** In this case, as shown in Figure 8, the two currents **630286** and **630288** cancel each other out resulting in net current of zero magnitude around the conductor **630280.** This type of configuration of conductors in a changing magnetic field may be used to cancel induced currents in the conductors.

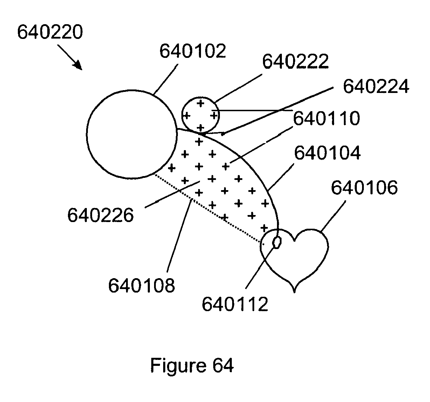

Figure 64 depicts an implanted pacemaker system **640220** comprising a pacing generator canister **640102,** conductive leads **640104,** and electrodes **640112** positioned in the heart **640106.** Additional loops **640222** are added to the overall configuration of the lead **640104** in the body with one or more crossings **640224.** In accordance with the concepts of the present invention, the plane of the loop **640222** is in the same plane as defined by the rest of the lead geometry.

The same oscillating magnetic field **640110** passes through loop **640222** and the loop defined by generator canister **640102,** conductive leads **640104,** electrodes **640112,** and conductive path **640108** through the body from the heart **640106** to the generator canister **640102.** It is noted that the total area enclosed by the loops can be adjusted by adding or removing loops **640222** or by changing the area enclosed by the loops (singly or collectively).

In one embodiment, the total area of the loop **640222** is the same as the loop area **640226.** In another embodiment, the total area of the loop **640222** is different from loop area **640226.** In another embodiment, the plane of loop **640222** is different from the plane of loop area **640226.** In yet another embodiment, loop **640222** and/or loop area **640226** do not define a single plane but are curved in three different spatial directions. In yet another embodiment, loop **640222** consists of at least three loops in three orthogonal planes.

In a further embodiment (not shown), new additional loops can be positioned in such a way as to encircle the pacemaker's generator canister. In another embodiment (not shown), the additional loops may be positioned inside the pacemaker's generator canister.

Referring back to Figure 64, a fastener (not shown) can be used at the loop cross over point **640224** to allow for adjustment of the loop's enclosed area and/or orientation and, once adjusted, to lock in the loop's adjustments. This same fastener can also be used to adjust a plurality of loops.

In a further embodiment of Figure 64, pacemaker's generator canister **640102** may include an orientation subsystem for automatically changing a spatial orientation of the coil to modify the strength of the magnetic resonance imaging switched gradient field induced current. In this embodiment, the orientation subsystem may sense the magnitude of the magnetic resonance imaging switched gradient field induced current (voltage) and spatially tune the orientation of the coils so as to produce more current to oppose the magnetic resonance imaging switched gradient field induced current or less current to oppose the magnetic resonance imaging switched gradient field induced current based upon the sensed magnitude of the magnetic resonance imaging switched gradient field induced current (voltage).

In other words, if greater current is needed to oppose the magnetic resonance imaging switched gradient field induced current, the orientation subsystem would spatially move or adjust one or more coils such that their surface planes become more perpendicular to the magnetic resonance imaging gradient field lines, thereby inducing a greater magnitude of current to oppose the magnetic resonance imaging switched gradient field induced current. On the other hand, if less current is needed to oppose the magnetic resonance imaging switched gradient field induced current, the orientation subsystem would spatially move or adjust one or more coils such that their surface planes become less perpendicular and more parallel to the magnetic resonance imaging gradient field lines, thereby inducing a lesser magnitude of current to oppose the magnetic resonance imaging switched gradient field induced current.

In another aspect of the present invention, a selection mechanism can be included in the pacemaker system. This selection mechanism is used to adjust the number of loops to include in the circuit.

For example, if the loops are located within the pacemaker canister, the selection mechanism can be used to manually select how many loops to include in the lead circuit depending on where the pacemaker can is placed in the body and the length of the lead. Alternatively, the selection mechanism may be used to automatically select how many loops to include in the lead circuit depending on where the pacemaker can is placed in the body and the length of the lead. In this alternative embodiment, the present invention monitors the voltages on the pacemaker's lead(s) and selects a different number of loops to connect to the lead(s) to cancel any induced voltages. Lastly, the selection mechanism may be externally programmed and transmitted to the pacemaker's PLU that then monitors and adjusts the number of loops in the lead circuit.

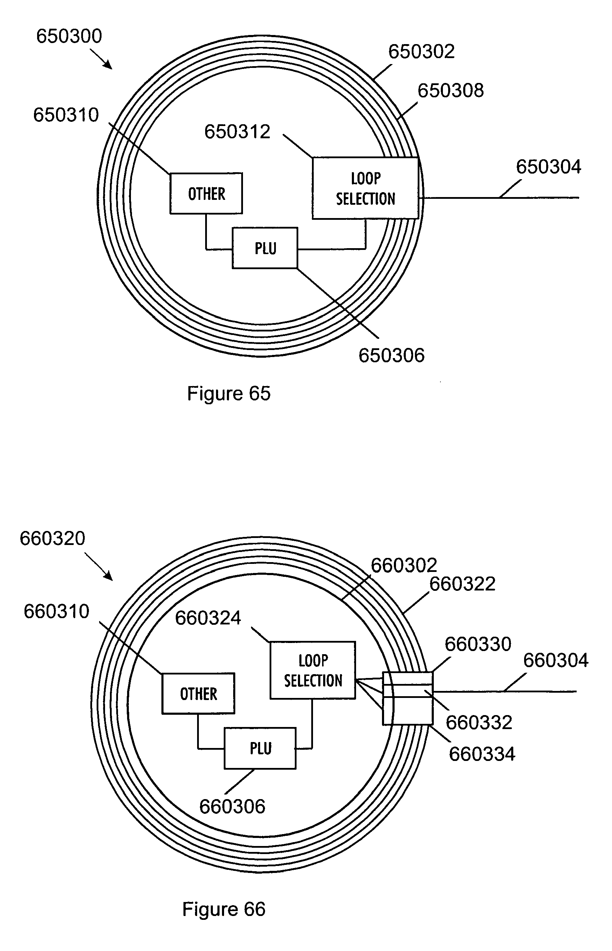

Figure 65 is a schematic of a pacemaker system **650300** that includes a pacemaker canister **650302** and the pacemaker's leads **650304.** The pacemaker's canister **650302** contains a programmable logic unit (PLU) **650306,** and other electronics **650310,** e.g. a pulse generator, power supply, etc. The system **650300** further includes conductive loops **650308** positioned within the pacemaker canister 650302.

The conductive loops are connected to a loop selection component **650312** that provides means for selectively adjusting the number of loops to be included in the leads' circuit **650304.** The leads **650304** are also connected to the loop selection component **650312** so that the leads **650304** can be electrically connected to the loops **650308.**

The loop selection component **650312** connects the loops **650308** to the leads' circuit **650304** in such a way that any induced voltages in the loops **650308** caused by changing magnetic fields in the environment, e.g. an magnetic resonance imaging environment, will cancel out or significantly reduce in magnitude any induced voltage along the leads **650304** that have also been caused by the environment's changing magnetic fields.

In one embodiment, the loop selection component **650312** is adjusted manually by screws, connection pins, and/or other means.

In another embodiment, the loop selection component **650312** is controlled by the PLU **650306.** The PLU **650306** may include means for receiving loop selection instructions from an external transmitter or may include sensors that measure environmental variables, e.g. changing magnetic fields in a magnetic resonance imaging environment. From this information, the PLU **650306** dynamically adjusts the loop selection component's **650312** adjustable parameters so as to change which loops are included in the leads' circuitry **650304**. It is noted that the loops **650308** need not be all in the same plane.

In a further embodiment of Figure 65, pacemaker's generator canister 650302 may include an orientation subsystem for automatically changing a spatial orientation of the coil to modify the strength of the magnetic resonance imaging switched gradient field induced current. In this embodiment, the orientation subsystem may sense the magnitude of the magnetic resonance imaging switched gradient field induced current (voltage) and spatially tune the orientation of the coils so as to produce more current to oppose the magnetic resonance imaging switched gradient field induced current or less current to oppose the magnetic resonance imaging switched gradient field induced current based upon the sensed magnitude of the magnetic resonance imaging switched gradient field induced current (voltage).

In other words, if greater current is needed to oppose the magnetic resonance imaging switched gradient field induced current, the orientation subsystem would spatially move or adjust one or more coils such that their surface planes become more perpendicular to the magnetic resonance imaging gradient field lines, thereby inducing a greater magnitude of current to oppose the magnetic resonance imaging switched gradient field induced current. On the other hand, if less current is needed to oppose the magnetic resonance imaging switched gradient field induced current, the orientation subsystem would spatially move or adjust one or more coils such that their surface planes become less perpendicular and more parallel to the magnetic resonance imaging gradient field lines, thereby inducing a lesser magnitude of current to oppose the magnetic resonance imaging switched gradient field induced current.

Figure 66 is a schematic of another pacemaker system **660320**. Pacemaker system **660320** includes conductive loops **660322** positioned externally to a pacemaker canister **660302**. In this embodiment, the loops **660332** are connected to an input port connection **660330** and to an output port connection **660334** which are electrically connected to the loop selection component **660324** located inside the pacemaker canister **660302**. Additionally, the pacemaker leads **660304** are connected to an electrical connector **660332** that is electrically connected to the loop selection component **660324**. It is noted that the conductive loops **660322** need not be all in the same plane.

In a further embodiment of Figure 66, pacemaker system **660320** may include an orientation subsystem for automatically changing a spatial orientation of the coil to modify the strength of the magnetic resonance imaging switched gradient field induced current. In this embodiment, the orientation subsystem may sense the magnitude of the magnetic resonance imaging switched gradient field induced current (voltage) and spatially tune the orientation of the coils so as to produce more current to oppose the magnetic resonance imaging switched gradient field induced current or less current to oppose the magnetic resonance imaging switched gradient field induced current based upon the sensed magnitude of the magnetic resonance imaging switched gradient field induced current (voltage).

In other words, if greater current is needed to oppose the magnetic resonance imaging switched gradient field induced current, the orientation subsystem would spatially move or adjust one or more coils such that their surface planes become more perpendicular to the magnetic resonance imaging gradient field lines, thereby inducing a greater magnitude of current to oppose the magnetic resonance imaging switched gradient field induced current. On the other hand, if less current is needed to oppose the magnetic resonance imaging switched gradient field induced current, the orientation subsystem would spatially move or adjust one or more coils such that their surface planes become less perpendicular and more parallel to the magnetic resonance imaging gradient field lines, thereby inducing a lesser magnitude of current to oppose the magnetic resonance imaging switched gradient field induced current.

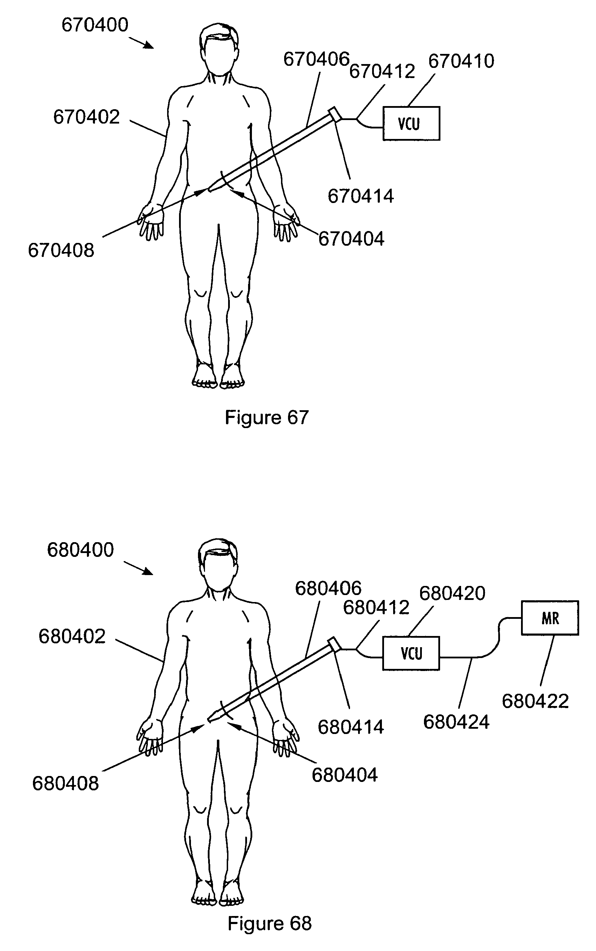

Figure 67 depicts a medical procedure in which a catheter **670406** or other medical device, e.g. a guidewire, which is comprised of conductive leads or other conductive components, may be partially inserted into a body **670402** and partially external to the body. In an magnetic resonance imaging environment, such conductive medical devices **670406** can develop problems like heating, induced voltages, etc. caused by the changing magnetic fields of the magnetic resonance imaging system. To compensate for induced currents and/or induced voltages in such devices **670406,** a voltage compensation unit (VCU) **670410** is electrically connected to the medical device **670406** via conductive leads **670412** and electrical connectors **670414,** externally to the patient's body **670402.**

The medical device 670406 is constructed with additional electrical connectors 670414 to allow for easy attachment of the VCU device 670410. The VCU device 670410 is connected to a power supply or may have a built in power supply, e.g. batteries. The VCU device 670410 has sensors built into it, which monitor the voltages of the conductive components in the medical device 670406, and delivers opposing voltages to the medical device 670406 to cancel out or significantly reduce any induced voltages caused by the changing magnetic fields in an magnetic resonance imaging (or other) environment.

Additionally or alternatively, the VCU device 670410 has sensors to detect the changing magnetic fields of the magnetic resonance imaging system and can synchronize the application of the canceling voltage with the magnetic resonance imaging system's changing fields.

In another embodiment depicted in Figure 68, the VCU device 680420 is connected to the magnetic resonance imaging system 680422 via communication means 680424 so that the start and end of the application of the magnetic resonance imaging system's 680422 fields may be communicated to the VCU device 680420. Other information that may be required (field strengths to be applied, magnetic resonance imaging scan sequence, etc.) may also be communicated to the VCU device 680420. The communication means 680424 may be electrical wires/coaxial/shielded/other, optical fiber, or a radio-frequency transmitter/receiver, or some sonic means of communication.

The conductive lead of a heart pacemaker is a filer winding. The filer winding may consist of two or more conductive stands coiled together in a spring-like configuration. The current (pulses, signals) then flows over the surface and through the contact points between one loop and the adjacent loop of the winding, rather than following the windings of the individual conductive strands. This occurs because there is no significant insulating material or surface coating between the contact points of the windings.

In accordance with the present invention, to reduce the alternating, induced current flowing, caused by a magnetic resonance system's changing magnetic fields, through the, for example, pacemaker's winding leads, the inductance value of the pacemaker's lead may be changed to increase the overall impedance of the pacemaker's lead.

Thus in one embodiment, a suitable radio-frequency choke is inserted inline with the pacemaker's lead, preferable near the distal tip. For example, referring back to Figure 57 and to the embodiment therein, electronic component **570146** and/or **570144** may comprise a radio-frequency choke. In a preferred embodiment, the radio-frequency choke has an inductance value of about 10 microHenries. In another embodiment, the inductance value is about 2 microHenries.

The specific value of inductance to introduce into the, for example, pacemaker's lead depends in part on the frequency of the induced signal from the magnetic resonance imaging system's imaging sequence that is to be blocked or significantly reduced.

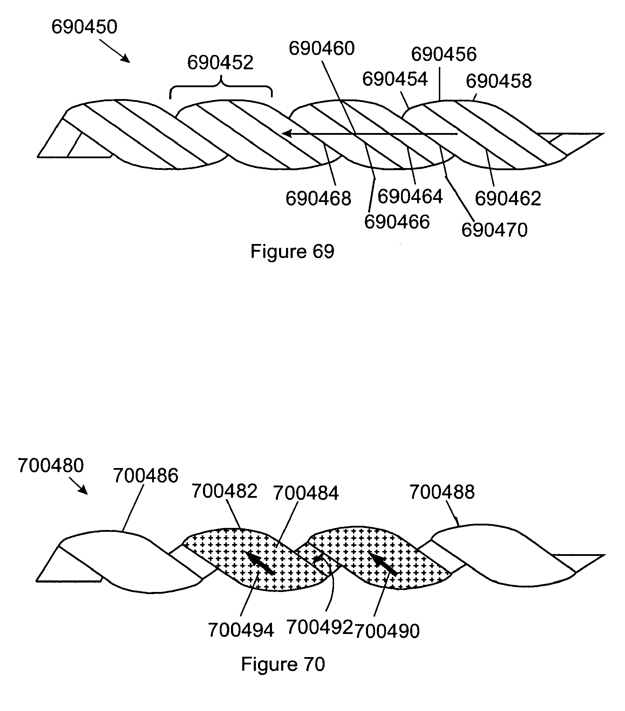

Figure 69 shows a portion of a coiled multi-filer lead **690450**. As illustrated in Figure 69, lead **690450** includes a plurality of coil loops **690452;** each coil loop **690452** consists of three conductive strands **690454, 690456,** and **690458.** A current **690460** through the lead **690450** can cross contact points **690464, 690466,** and **690462** between the strands as well as the coil contact points **690468** and **690470.** Thus, the current **690460** does not necessarily follow the coiling of the lead's conductive strands **690454, 690456,** and **690458.**

Figure 70 shows a portion of a coiled lead assembly **700480** including a region **700482** that has an insulating coating **700484** applied to its surface. The coiled lead assembly **700480** is depicted in an elongated position in which adjacent coil windings are not in contact with one another. It is to be understood that the normal, relaxed position of the lead assembly **700480** has all adjacent coiled windings in contact.

With the addition of an insulated coating **700484** over the winding region **700482,** the current **700490, 700492,** and **700494** is now forced to substantially follow the curvature of the coiled winding **700482,** thus forming an inductive coil inline with the conductive lead regions **700486** and **700488** which do not have an insulated coating. The inductive value of the created inductor can be adjusted by adjusting the length of the region to which the insulative coating **700484** is applied.

It is noted that the coating **700484** may be a partially resistive material. In such an example, the inductance is then adjusted by adjusting the resistive properties of the material **700484.**

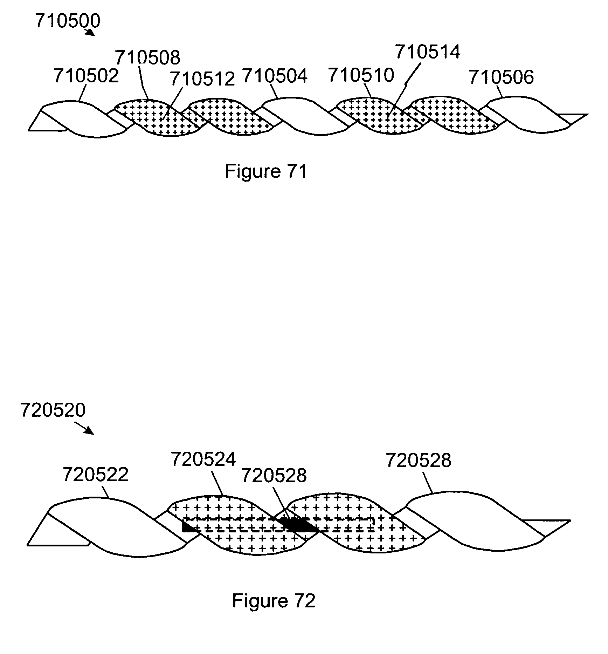

Figure 71 is a schematic of a coiled lead assembly **710500** comprised of uninsulated regions **710502, 710504,** and **710506,** and coated insulated regions **710508** and **710510** with coatings **710512,** and **710514,** respectively. Through the application of the coating, the current is forced to substantially follow the curvature of the coiled windings, thus forming an inductive coil inline with the conductive lead regions that do not have a coating applied thereto. The inductive value of the created inductor can be adjusted by adjusting the length of the region to which the insulative coatings **710512** and **710514** are applied. In one embodiment, coatings **710512** and **710514** are the same coatings. In another embodiment, the coatings **710512** and **710514** are different materials.

It is noted that coatings **710512** and **710514** may be the same coating material but having differing properties, e.g., the thickness of the coatings, or the length of the coated region **710508** and **710510.** It is further noted that the two-coated regions **710508** and **710510** may have different inductive values. It is also noted that more than two different regions along the length of the lead assembly can be coated.

Figure 72 is a schematic of a portion of a coiled lead assembly **720520** including at least one region **720524** with a coating applied thereto. Through the application of the coating, the current is forced to substantially follow the curvature of the coiled windings, thus forming an inductive coil inline with the conductive lead regions **720522** and **720526** that do not have a coating applied thereto. The inductive value of the created inductor can be adjusted by adjusting the length of the region to which the insulative coating **720524** is applied. Additionally, through the coated region **720524** is positioned a rod **720528** which also changes the inductive value of the coated region **720524.** It is noted that the rod **720528** may be of ferrite material. It is further noted that multiple rods can be inserted into multiple coated regions along the length of the coiled lead.

It is noted that multiple coatings can be applied to the same coated region of the coiled lead wherein the multiple coating layers may be comprised of different materials. It is further noted that one or more layers of the multiple layers of coatings may comprise ferrite material.

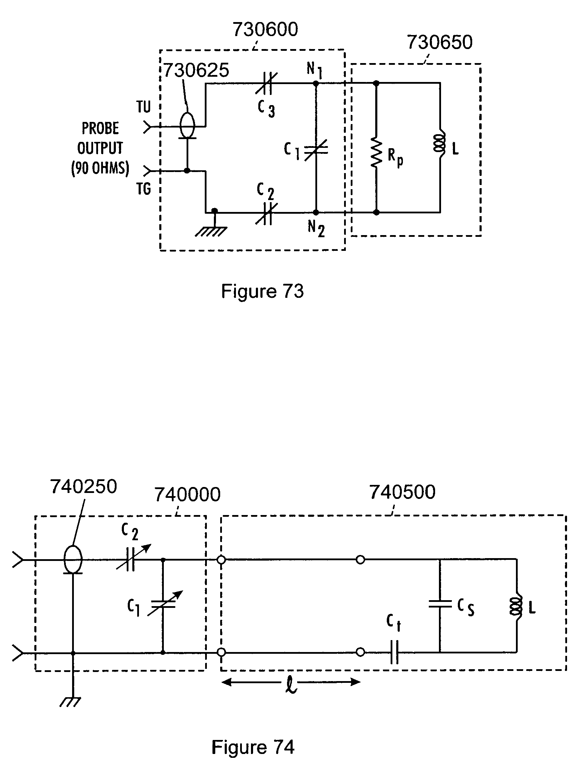

In another embodiment of the present invention, the heating and/or induced voltages on catheters or guide wires is controlled or substantially eliminated by introducing or creating detuned characteristic impedance at a proximal ends (ends that are not within the body) of the catheters or guide wires. This introduction or creation of detuned characteristic impedance will be discussed in more detail with respect to Figures 73-76.

As noted above, during magnetic resonance imaging procedures, catheters and guide wires (wire lines), with or without grounded shielding, are used to measure physiological signals. In such instances, two-wire catheters or guide wires having a grounded shield have one conductor that carries the actual measured signal and the other wire is grounded. In terms of characteristic impedance, the two-wire catheters or guide wires having a grounded shield are unbalanced. In contrast, a single wire catheter or guide wire has characteristic impedance that is balanced.

According to the concepts of the present invention, the characteristic impedance of the catheters and guide wires, used during magnetic resonance imaging procedures, should be unbalanced at the proximal end, under all conditions, to reduce or eliminate heating and induced voltages. To realize this reduction or elimination of heating and induced voltages at the proximal end of the catheters and guide wires, used during magnetic resonance imaging procedures, by creating an unbalanced characteristic impedance, the present invention proposes providing a Balun along the catheter and/or guide wire or at the proximal end of the catheter and/or guide wire.

Using a Balun to maintain unbalanced characteristic impedance, the reactance at the distal end of the catheter and/or guide wire approaches infinity. Thus, even when there is some potential on the wire, the unbalanced characteristic impedance has approximately four times the ground loop looses of a balanced line, thereby substantially avoiding any incident of thermal injury. An example of such an arrangement is illustrated in Figure 73.

As illustrated in Figure 73, a guide wire or catheter **730650** has characteristic impedance due to its intrinsic resistance from intrinsic resistor capacitors $R_P$ and its intrinsic inductance from intrinsic inductor L. To create the unbalanced characteristic impedance at the proximal end of the guide wire or catheter **730650,** a Balun **730600** is placed along the guide wire or catheter **730650.** In other words, the Balun **730600** is in vitro.

The Balun **730600** includes a variable capacitor $C_1$ connected in parallel with the guide wire or catheter **730650** and two variable capacitors $C_2$ and $C_3$ connected in series with the guide wire or catheter **730650.** It is noted that one end of the variable capacitor $C_2$ is connected to the shield **730625** and ground or a known voltage. The capacitance of the variable capacitors $C_1$, $C_2$, and $C_3$ are adjusted to create the unbalanced characteristic impedance.

More specifically, the variable capacitors $C_1$, $C_2$, and $C_3$ may be used for both matching and providing a certain amount of balancing for the guide wire or catheter **730650** characteristic impedance. In this example, the variable capacitors $C_1$, $C_2$, and $C_3$ lift the voltage on the guide wire or catheter **730650** from ground. The larger the reactance of the variable capacitors $C_1$, $C_2$, and $C_3$, the more symmetric and balanced the circuit of the guide wire or catheter **730650** becomes. Conversely, according to the concepts of the present invention, if the reactive capacitance of the Balun **730600** is detuned (made less resonant), the circuit of the guide wire or catheter **730650** becomes asymmetric and unbalanced, breaking down, to reduce the chances of thermal injury at the distal end of the guide wire or catheter **730650** due to heating from induced voltages.

Figure 74 illustrates another embodiment of the present invention wherein a guide wire or catheter **740500** has characteristic impedance due to its intrinsic capacitance from intrinsic capacitors $C_t$ and $C_s$ and its intrinsic inductance from intrinsic inductor **L.** To create the unbalanced characteristic impedance at the proximal end of the guide wire or catheter **740500,** a Balun **740000** is connected across the proximal end of the guide wire or catheter **740500.** In other words, the Balun **740000** is outside the body at the proximal end of the guide wire or catheter **74050.** By having the Balun **740000** outside the body, the varying of the reactance of the guide wire or catheter **6500** can be readily and manually controlled.

The Balun **740000** includes a variable capacitor $C_1$ connected in parallel with the guide wire or catheter **740500** and a variable capacitor $C_2$ connected in series with the guide wire or catheter **740500.** It is noted that one end of the variable capacitor $C_1$ is connected to the shield **740250** and ground or a known voltage. The capacitance of the variable capacitors $C_1$ and $C_2$ are adjusted to create the unbalanced characteristic impedance.

More specifically, the variable capacitors $C_1$, and $C_2$ may be used for both matching and providing a certain amount of balancing for the guide wire or catheter **740500** characteristic impedance. In this example, the variable capacitors $C_1$, $C_2$, and $C_3$ lift the voltage on the guide wire or catheter **740500** from ground. The larger the reactance of the variable capacitors $C_1$ and $C_2$, the more symmetric and balanced the circuit of the guide wire or catheter **740500** becomes. Conversely, according to the concepts of the present invention, if the reactive capacitance of the Balun **740000** is detuned (made less resonant), the circuit of the guide wire or catheter **740500** becomes asymmetric and unbalanced, breaking down, to reduce the chances of thermal injury at the distal end of the guide wire or catheter **740500** due to heating from induced voltages.

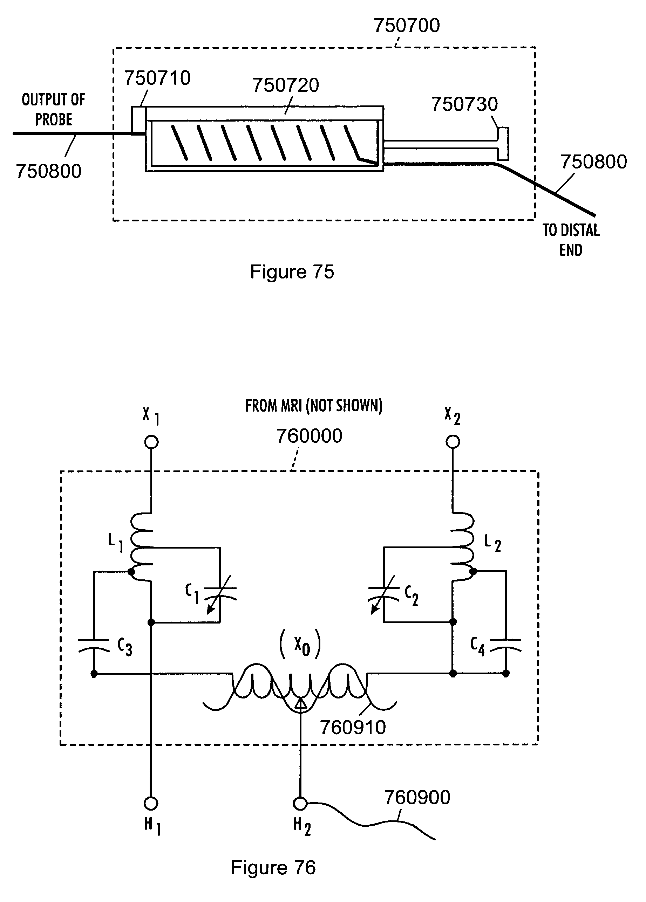

Figure 75 illustrates a further embodiment of the present invention wherein a guide wire or catheter **750800** is connected to a Balun **750700**. The Balun **750700** includes a variable capacitor **750710,** a copper foil **750720,** and a non-conductive tuning bolt **750730.** The Balun **750700** is further connected to the output of the probe **750800**

The Balun **750700** adjusts its characteristic impedance by increasing or decreasing the number wire coils are found within the copper foil **750720.** The combination of the coils and the copper foil **750720** forms a variable capacitor, having it impedance determined by the change in the surface area of the coils positioned opposite of the copper foil **750720.** As more coils are introduced into the volume created by the copper foil **750720,** the capacitance of this combination increases. Moreover, as fewer coils are introduced into the volume created by the copper foil **750720,** the capacitance of this combination decreases. Thus, the capacitance of the Balun **750700** is adjusted to create the unbalanced characteristic impedance.

Figure 76 illustrates another embodiment of the present invention wherein a guide wire or catheter **760900** is electronically isolated by a voltage control unit to always appear as an unbalanced line to any possible magnetic field that may be applied from a magnetic resonance imager unit (not shown). As current begins to flow due to the changing magnetic fields from the magnetic resonance imaging, a tapped voltage from a voltage-controlled oscillator in the magnetic resonance imaging unit is applied across terminals **X1** and **X2** of the voltage control unit.

According to the concepts of the present invention, to automatically maintain an unbalanced characteristic impedance at the distal end of the guide wire or catheter **760900,** a capacitance unbalanced balun unit **760000,** located within the voltage control unit, is connected through a variable inductor **760910** to the proximal end of the guide wire or catheter **760900.** In other words, the voltage control unit containing the capacitance unbalanced balun unit **760000** is outside the body at the proximal end of the guide wire or catheter **760900.** By having the capacitance unbalanced balun unit **760000** and variable inductor **760910** outside the body, the varying of the reactance (**X0**) of the guide wire or catheter **760900** can be readily adjusted and automatically controlled by the voltage control unit circuit's reactance to the tapped voltage from the voltage-controlled oscillator in the magnetic resonance imaging unit as it is applied across **X1** and **X2** for any instance of time from time zero (TO) or instantiation of the magnetic resonance imaging radio-frequency pulses.

The capacitance unbalanced balun unit **760000** includes two non-magnetic trimmer capacitors **C1** and **C2** connected in parallel with LC circuits (**L1,C3**) and (**L2,C4**), respectively, setting up a simplified dual T network that is effectively in series with the guide wire or catheter **760900.** It is noted that one end of the simplified dual T network is connected to neutral **H1** and the other end is connected to a continuously variable voltage **H2,** based on inputs to the circuit from the voltage-controlled oscillator in the magnetic resonance imaging unit at **X1** and **X2.** The reactance (**X0**) of the LC circuits in the T network is automatically adjusted to create the desired unbalanced characteristic impedance.

More specifically, the T network **L1, C1, C3** and **L2, C2, C4** respectively, may be used for both matching and unmatching characteristic impedance of the guide wire or catheter **760900** and to provide a certain amount of balancing or unbalancing for the guide wire or catheter **760900** by varying the circuit's capacitive or inductive reactance (**X0**).

In this example, as the voltage from the voltage-controlled oscillator in the magnetic resonance imaging unit is provided to the voltage control unit (**X1** and **X2**), the two non-magnetic trimmer capacitors **C1** and **C2,** connected in parallel with LC circuits, (**L1, C3**) and (**L2, C4**), lift the voltage on the guide wire or catheter **760900** from ground to an unbalanced state with respect to the radio-frequency pulse applied by the magnetic resonance imaging unit. The reactance of the T network and its LC circuits, (**L1, C3**) and (**L2, C4**), respectively, cause the guide wire or catheter **760900** to become asymmetric and unbalanced, automatically breaking down the reactance to ensure that resonance for the guide wire or catheter **760900** is never present, thus reducing the chances of thermal injury at the distal end of the guide wire or catheter 900 due to heating from induced voltages.

In the following descriptions, the term, "stretching," is used to describe a state of wires used in leads. The term, "stretching," is being used to convey a "permanent" state of deformation wherein the wire has been deformed to fabricate gaps between adjacent coiled wires or coiling loops. In other words, after stretching, the gaps remain when the wire is in a relaxed state. Furthermore, when a lead is placed in the body, there is no stretching. The gaps remain because the lead was originally wound with the gaps or the lead was permanently deformed during fabrication by stretching or other means. The gaps, thus, remain when the wire is in a relaxed state.

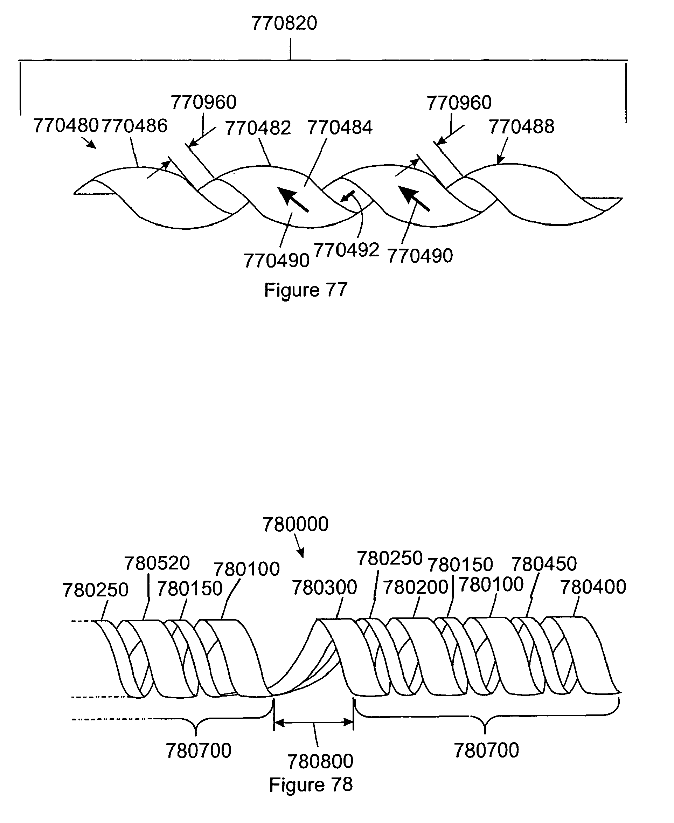

Figure 77 shows a portion of a coiled medical lead assembly **770480** including a region **770820** wherein the coils are stretched to form gaps or spacings **770960** between the coils in an axial direction. The coiled lead assembly **770800** is depicted in an elongated "permanent" deformed state in which adjacent coil windings are not in contact with one another due to the gaps or spacings **770960.** It is to be understood that the normal non-deformed state of the lead assembly **770800** has all adjacent coiled windings in contact, as illustrated in Figure 69.

In one embodiment of the present invention, the coiled wire **770480** may include a Teflon™ coating. The stretching, before final assembly, of the coiled wire(s) used in the final assembly of the coiled medical lead **770480** to produce the gaps or spacings **770960** forces the current **770490** and **770492** to substantially follow the curvature of the coiled winding **770820,** thus forming an inductive coil. The formation of the gaps or spacings **770960** also affects the capacitance of the coiled wire medical lead **770800,** thereby impacting the overall impedance of the coiled wire medical lead **770480.** The gaps or spacings **770960** reduce the inter-loop capacitance without significantly reducing the loop's inductance. The inter-loop capacitance can also be reduced by interpositioning non-conductive material between the loops, the interposition non-conductive material forming the gaps or spacings **770960.**

This impact upon the impedance of the coiled wire medical lead **770480,** from the formation of the gaps or spacings **770960,** reduces medical lead heating; such heating can be destructive to the surrounding tissue. The inductive impedance and the capacitance impedance of the coiled wire medical lead **770480** can be adjusted to minimize medical lead heating, by maximizing the overall impedance of the medical lead, by adjusting the width of the gaps or spacings **770960.**

This concept can also be applied to four-conductor deep brain stimulation lead. A deep brain stimulation lead is typically four thin, insulated, coiled wires bundled within polyurethane insulation. Each wire ends in an electrode, resulting in four electrodes at the tip of the lead. Deep brain stimulation leads can be implanted bilaterally in the subthalamic nucleus or the globus pallidus interna for Parkinson's disease, or unilaterally in the left or right ventral intermediate nucleus of the thalamus. The deep brain stimulation lead delivers stimulation using either one electrode or a combination of electrodes.

In this environment, a conventional deep brain stimulation lead may consist of four, multi-fillar conductors electrically insulated from one another by a thin Teflon™ coating. The thin Teflon™ coating by itself does not sufficiently enhance the electrical impedance of the medical lead, and therefore does not provide any significant reduction in lead heating. Such a thin coating may actually increase lead capacitance, which would actually increase lead heating.

To address this problem in a deep brain stimulation lead, the present invention deforms all four wires simultaneously by stretching the lead beyond the lead's safe shear stress limit, thereby inducing permanent deformation. This stretching, before final assembly, of all four wires creates gaps or spacings between coiled wires as shown in Figure 77 to produce gaps or spacings.

Stretching, before final assembly of the coiled wire(s) used in the final assembly of the deep brain stimulation lead to produce the gaps or spacings, forces the current to substantially follow the curvature of the coiled winding of the deep brain stimulation lead, thus forming an inductive coil. The formation of the gaps or spacings also affects the capacitance of the deep brain stimulation lead, thereby impacting the overall impedance of the deep brain stimulation lead. It is noted that the gaps or spacings can also be realized by utilizing controlled winding methods that control of the pitch of the winding of the deep brain stimulation lead to adjust and control the amount of gap or spacing between the wires.

This impact upon the impedance of the deep brain stimulation lead, from the formation of the gaps or spacings, reduces medical lead heating; such heating can be destructive to the surrounding tissue. The inductive impedance and the capacitance impedance of the deep brain stimulation lead can be adjusted to minimize medical lead heating, by maximizing the overall impedance of the medical lead, by adjusting the width of the gaps or spacings.

An example of deep brain stimulation lead using the concepts of the present invention is illustrated in Figure 78. As illustrated in Figure 78, the deep brain stimulation lead **780000** includes a four wire bundle represented by loops (**780100, 780200, 780300**, and **780400**) with a ground wire represented by loops (**780150, 780250, 780350**, and **780450**) between each of the loops (**780100, 780200, 780300,** and **780400**). The deep brain stimulation lead **780000** is stretched to create gaps or spacings **780800** between the four wire bundle with ground wire groups **780700.**

The stretching, before final assembly, of the coiled wire(s) used in the final assembly of the deep brain stimulation lead **780000** to produce the gaps or spacings **780800** forces the currents to substantially follow the curvature of the deep brain stimulation lead **780000,** thus forming an inductive coil. The formation of the gaps or spacings **780800** also affects the capacitance of the deep brain stimulation lead **780000,** thereby impacting the overall impedance of the deep brain stimulation lead **780000.** It is noted that the gaps or spacings can also be realized by utilizing controlled winding methods that control of the pitch of the winding of the deep brain stimulation lead to adjust and control the amount of gap or spacing between the wires.

This impact upon the impedance of the deep brain stimulation lead **780000,** from the formation of the gaps or spacings **780800,** reduces medical lead heating; such heating can be destructive to the surrounding tissue. The inductive impedance and the capacitance impedance of the deep brain stimulation lead **780000** can be adjusted too minimize medical lead heating, by maximizing the overall impedance of the medical lead, by adjusting the width of the gaps or spacings **780800.**

It is noted that the stretching can also cause gaps or spacings between the individual wires within the group to further impact the impedance of the deep brain stimulation lead **780000.** The gaps or spacings **780800** reduce the inter-loop capacitance without significantly reducing the loop's inductance. The inter-loop capacitance can also be reduced by interpositioning non-conductive material between the loops, the interposition non-conductive material forming the gaps or spacings **780800.**

It is noted that the four multi-filar wires in the deep brain stimulation lead may also be of different coil diameters (i.e. being concentric) and may contain wires with different thicknesses. With respect to the concepts of the present invention, the four, concentric, "stretched conductors" will not remain in contact with one another. Hence, the stretching will increase lead impedance due to increased gap or spacing between adjacent windings within any one conductor by affecting both the capacitance and inductance of the overall lead, since conductor to conductor contact is eliminated. The increase lead impedance and decrease lead capacitance significantly reduce heating effects of the deep brain stimulation lead.

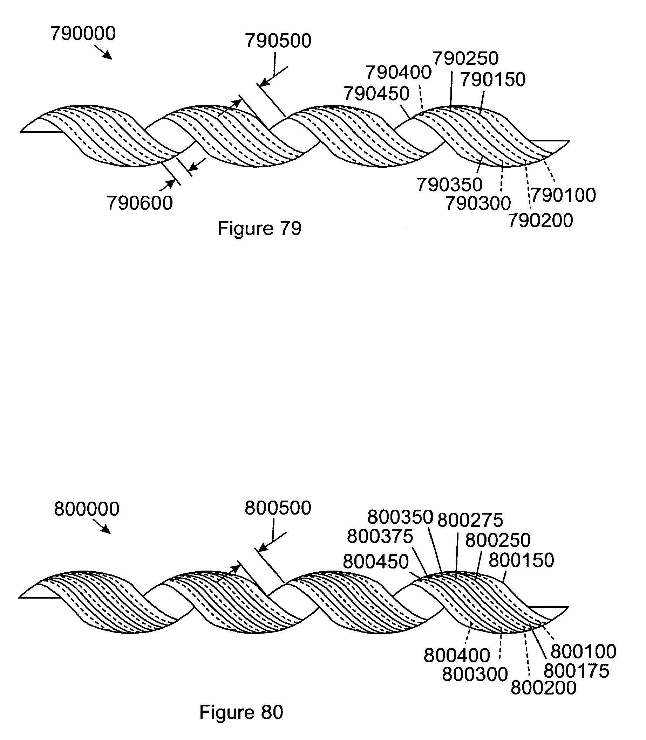

Another example of a multi-wire medical lead, such as a deep brain stimulation lead, using the concepts of the present invention is illustrated in Figure 79. As illustrated in Figure 79, the multi-wire medical lead **790000** includes four wires (**790100, 790200, 790300**, and **790400)** (dotted lines). Each wire (**790100, 790200, 790300**, and **790400**) is coated with a non-conductive sheath (**790150, 790250, 790350**, and **790450**). The non-conductive sheath may comprise PTFE, a polymer, or a dielectric material.

The thickness of each non-conductive sheath (**790150, 790250, 790350,** and **790450**) creates a non-conductive gap or spacing between adjacent wires (**790100, 790200, 790300**, and **790400**). For example, the thickness of non-conductive sheaths **790150** and **790250** creates a non-conductive gap or spacing between adjacent wires **790100** and **790200.**

The multi-wire medical lead **790000** can also be stretched to create gaps or spacings **790500** between the four wires (**790100, 790200, 790300**, and **790400**). The stretching, before final assembly, of the coiled wire(s) used in the final assembly of the multi-wire medical lead **790000** to produce the gaps or spacings **790500** and the gaps or spacings formed by non-conductive sheaths (**790150, 790250, 790350,** and **790450**) force the currents to substantially follow the curvature of the multi-wire medical lead **790000,** thus forming an inductive coil. The formation of the gaps or spacings **790500** and the gaps or spacings formed by non-conductive sheaths (**790150, 790250, 790350,** and **790450**) also affect the capacitance of the multi-wire medical lead **790000,** thereby impacting the overall impedance of the multi-wire medical lead **790000.** It is noted that the gaps or spacings can also be realized by utilizing controlled winding methods that control of the pitch of the winding of the multi-wire medical lead to adjust and control the amount of gap or spacing between the wires.

This impact upon the impedance of the multi-wire medical lead **790000,** from the formation of the gaps or spacings **790500** and the gaps or spacings formed by non-conductive sheaths (**790150, 790250, 790350,** and **790450**), reduces medical lead heating; such heating can be destructive to the surrounding tissue. The inductive impedance and the capacitance impedance of the multi-wire medical lead **790000** can be adjusted too minimize medical lead heating, by maximizing the overall impedance of the medical lead, by adjusting the width of the gaps or spacings **790500** and the gaps or spacings formed by non-conductive sheaths (**790150, 790250, 790350,** and **790450**).

A further example of a multi-wire medical lead, such as a deep brain stimulation lead, using the concepts of the present invention is illustrated in Figure 80. As illustrated in Figure 80, the multi-wire medical lead **800000** includes four wires (**800100, 800200, 800300,** and **800400**) (dotted lines). Each wire (**800100, 800200, 800300,** and **800400**) is coated with a non-conductive sheath (**800150, 800250, 800350,** and **800450**). The thickness of each non-conductive sheath (**800150, 800250, 800350,** and **800450**) adds to a gap or spacing between adjacent wires (**800100, 800200, 800300,** and **800400**). For example, the thickness of non-conductive sheaths **800150** and **800250** adds to a gap or spacing between adjacent wires **800100** and **800200.**

The multi-wire medical lead **800000** can also be stretched to create gaps or spacings **800500** between the four wires (**800100, 800200, 800300,** and **800400**). The stretching, before final assembly, of the coiled wire(s) used in the final assembly of the multi-wire medical lead **800000** can also create air gaps or spacings (**800175, 800275,** and **800375**) between adjacent non-conductive sheath (**800150, 800250, 800350,** and **800450**). The combination of the thickness of each non-conductive sheath (**800150, 800250, 800350,** and **800450**) adding to a gap or spacing between adjacent wires and the air gaps or spacings (**800175, 800275,** and **800375**) create an inter-wire gap. For example, an inter-wire gap or spacing is the combination of the thickness of non-conductive sheaths **800150** and **800250** and the air gap or spacing **800175.** It is noted that the gaps or spacings can also be realized by utilizing controlled winding methods that control of the pitch of the winding of the multi-wire medical lead to adjust and control the amount of gap or spacing between the wires.

The stretching, before final assembly, of the coiled wire(s) used in the final assembly of the multi-wire medical lead **800000** to produce the gaps or spacings **800500** and the inter-wire gaps or spacings, formed by non-conductive sheaths (**800150, 800250, 800350,** and **800450**) and the air gaps or spacings (**800175, 800275,** and **800375**), force the currents to substantially follow the curvature of the multi-wire medical lead **800000,** thus forming an inductive coil. The formation of the gaps or spacings **800500** and the inter-wire gaps or spacings, formed by non-conductive sheaths (**800150, 800250, 800350,** and **800450**) and the air gaps or spacings (**800175, 800275,** and **800375**), also affect the capacitance of the multi-wire medical lead **800000,** thereby impacting the overall impedance of the multi-wire medical lead **800000.**

This impact upon the impedance of the multi-wire medical lead **800000,** from the formation of the gaps or spacings **800500** and the inter-wire gaps or spacings, formed by non-conductive sheaths (**800150, 800250, 800350,** and **800450**) and the air gaps or spacings (**800175, 800275,** and **800375**), reduces medical lead heating; such heating can be destructive to the surrounding tissue. The inductive impedance and the capacitance impedance of the multi-wire medical lead **800000** can be adjusted too minimize medical lead heating, by maximizing the overall impedance of the medical lead, by adjusting the width of the gaps or spacings **800500** and the inter-wire gaps or spacings, formed by non-conductive sheaths (**800150, 800250, 800350,** and **800450**) and the air gaps or spacings (**800175, 800275,** and **800375**).

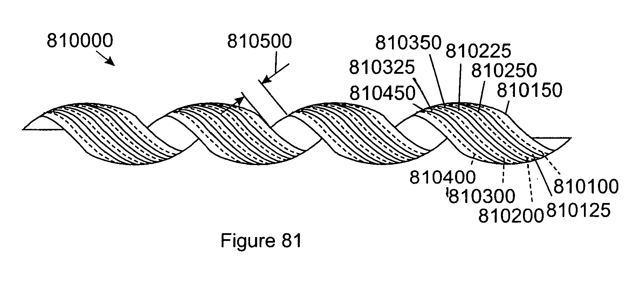

Another example of a multi-wire medical lead, such as a deep brain stimulation lead, using the concepts of the present invention is illustrated in Figure 81. As illustrated in Figure 81, the multi-wire medical lead **810000** includes four wires (**810100, 810200, 810300,** and **810400**) (dotted lines). Each wire (**810100, 810200, 810300,** and **810400**) is coated with a non-conductive sheath (**810150, 810250, 810350,** and **810450**). The thickness of each non-conductive sheath (**810150, 810250, 810350,** and **810450**) adds to a gap or spacing between adjacent wires (**810100, 810200,**

**810300,** and **810400**). For example, the thickness of non-conductive sheaths **810150** and **810250** adds to a gap or spacing between adjacent wires **810100** and **810200.**

The multi-wire medical lead **810000** is created with dummy (polymer) wires (**810125, 810225,** and **810325**) that are formed between adjacent wires (**810100, 810200, 810300,** and **810400**). For example, dummy (polymer) wire **810125** is formed between adjacent wires **810100** and **810200.** The combination of the thickness of each PTFE sheath (**810150, 810250, 810350,** and **810450**) adding to a gap or spacing between adjacent wires and the dummy (polymer) wires (**810125, 810225,** and **810325**) create an inter-wire gap. For example, an inter-wire gap or spacing is the combination of the thickness of non-conductive sheaths **810150** and **810250** and the dummy (polymer) wire **810125.**

The dummy (polymer) wires (**810125, 810225,** and **810325**) of the multi-wire medical lead **810000** can also be removed to form air gaps or spacings, similar to those illustrated in Figure 80, between adjacent non-conductive sheath (**810150, 810250, 810350,** and **810450**). The combination of the thickness of each non-conductive sheath (**810150, 810250, 810350,** and **810450**) adding to a gap or spacing between adjacent wires and the air gaps or spacings formed by removing the dummy (polymer) wires (**810125, 810225,** and **810325**) create an inter-wire gap. For example, an inter-wire gap or spacing is the combination of the thickness of non-conductive sheaths **810150** and **810250** and the air gap or spacing formed by removing the dummy (polymer) wire **810125.**

The multi-wire medical lead **8000** can also be stretched to create gaps or spacings **810500** between the four wires (**810100, 810200, 810300,** and **810400**). The stretching, before final assembly, of the coiled wire(s) used in the final assembly of the multi-wire medical lead **810000** can also create air gaps or spacings (**810185, 810285,** and **810385**) between adjacent PTFE sheath (**810150, 810250, 810350,** and **810450**). It is noted that the gaps or spacings can also be realized by utilizing controlled winding methods that control of the pitch of the winding of the multi-wire medical lead to adjust and control the amount of gap or spacing between the wires.

The stretching, before final assembly, of the coiled wire(s) used in the final assembly of the multi-wire medical lead **810000** to produce the gaps or spacings **8500** and the inter-wire gaps or spacings force the currents to substantially follow the curvature of the multi-wire medical lead **810000,** thus forming an inductive coil. The formation of the gaps or spacings **810500** and the inter-wire gaps or spacings also affect the capacitance of the multi-wire medical lead **810000,** thereby impacting the overall impedance of the multi-wire medical lead **810000.**

This impact upon the impedance of the multi-wire medical lead **810000,** from the formation of the gaps or spacings **810500** and the inter-wire gaps or spacings reduces medical lead heating; such heating can be destructive to the surrounding tissue. The inductive impedance and the capacitance impedance of the multi-wire medical lead **810000** can be adjusted to minimize medical lead heating, by maximizing the overall impedance of the medical lead, by adjusting the width of the gaps or spacings **810500** and the inter-wire gaps or spacings.

It is noted that the various examples illustrated in Figures 24-26 are equally applicable to two-wire medical leads, or other medical leads having multiple wires being wound to form the coiled medical lead.

Moreover, each wire could be replaced with a very small diameter coaxial cable, and then coiled. It is noted that this embodiment introduces an additional conductor between the existing conductors. The shielding of each coaxial cable can be connected to the electronics in a signal generator canister, to the electric ground of the circuit in the signal generator canister, or other electronics.

The present invention, as set forth above, reduces the parasitic capacitance between each adjacent wire loop in the coiled lead. The parasitic capacitance makes looped wire function as an electrical short when placed in the 64 MHz radio frequency fields of a magnetic resonance scanner. The parasitic capacitance allows the current to "hop" from one loop to the other. The parasitic capacitance does not prevent high frequency signals from traveling along. By breaking up this parasitic capacitance along (between) the loops of the coiled lead, the present invention reduces the ability of the magnetic resonance scanner's induced signals from passing along the coiled lead.

It is noted that the present invention adjusts both the (parasitic) capacitance and the inductance of the lead as the windings and spacing are changed. However, the present invention only changes the capacitance when the present invention changes the dielectric material, leaving the spacing the same. So, the present invention can change both the capacitive and inductive reactance. It is also possible to achieve specific beneficial values of these parameters from these values, such as achieving resonance or preventing resonance, as well as, significantly reducing the heating of the lead.

It is noted that for a bipolar pacing lead and other multi-conductor leads, i.e. deep brain stimulation leads, pain relief leads, one or more conductors through the lead is designated to act as the ground conductor with respect to the other "signal" or "sensing" conductors making up the lead. Moreover, it is noted that a lead in a magnetic resonance imaging environment can have electromagnetic forces or voltages induced therein at every point along the lead's length and in all the conductors comprising the lead due to the rotating magnetic field formed by the magnetic resonance imaging scanner. This rotating magnetic field is often called the RF field as well as the $B_1$ field of the magnetic resonance imaging scanner.

The equation giving the induced electric field and hence the induced voltages is given be Maxwell's equation:

$$\nabla \times \vec{E} = -\frac{\partial \vec{B}}{\partial t} \qquad (1)$$

For the rotating $\vec{B}_1$ field,

$$\vec{B} = B_1 \sin(\omega t)\,\hat{x} + B_1 \cos(\omega t)\,\hat{y} \qquad (2)$$

where $B_1$ is the magnetic amplitude function, $\omega$ is the angular frequency of the rotating magnetic field and $\hat{x}$ and $\hat{y}$ are unit vectors in the x and y axis directions, respectively.

The electric field equation is given by

$$\vec{E} = -B_1\,\omega\left(x\sin(\omega t) + y\cos(\omega t)\right)\hat{z} \qquad (3)$$

where $\hat{z}$ is the unit vector in the z-axis direction.

The induced electromagnetic force is then the line integral along the path of the lead conductors

$$EMF = \int \vec{E} \circ d\vec{s} \qquad (4)$$

It is noted that the amplitude function $B_1$ may be a time modulated function, e.g. a sinc(wt) function, in which case the above equations are appropriately modified.

For a bipolar pacing lead, there are two conductors in the lead. Most of the time, both conductors are at the same or nearly the same electrical potential. When a pacing pulse is generated, an electrical potential difference (voltage difference) is generated between the two conductors in the lead.

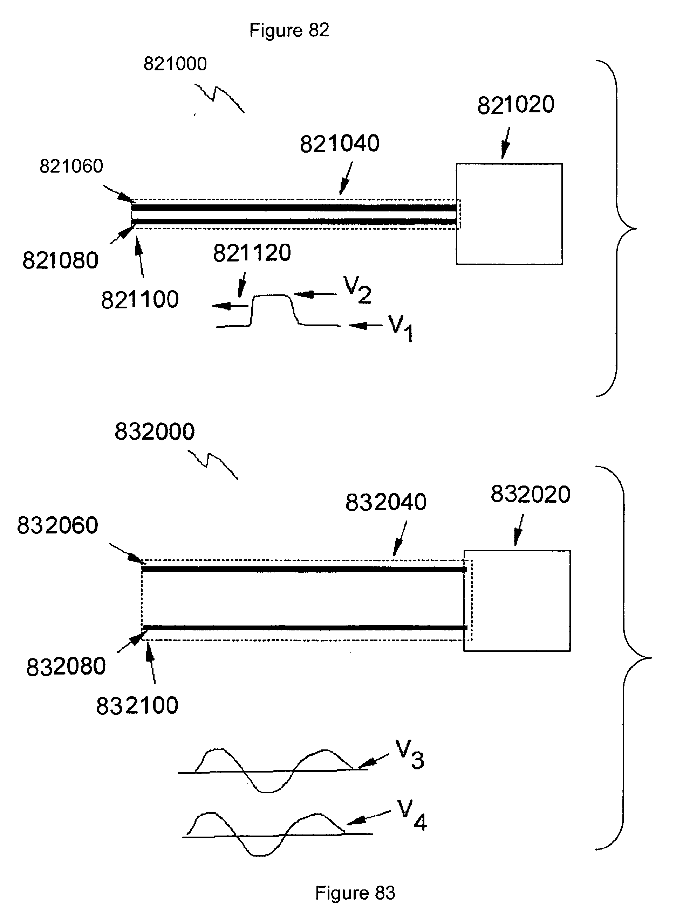

Figure 82 depicts a pacing system **821000** having a lead **821040** and a pulse generator **821020.** The lead **821040** includes two electrically isolated conductors **821060** and **821080.** At the distal tip **821100,** electrodes (not shown) are exposed to connect the conductors **821060** and **821080** with body tissue and/or fluids (not shown).

When a pulse is delivered **821120** from the generator **821020,** a voltage difference between the two conductors **821060** and **821080** is developed. More specifically, as illustrated in Figure 82, when a pulse **821120** is generated on conductor **821060,** the voltage on that conductor **821060** rises to $V_2$ for the duration of the pulse, pulsewidth, while the voltage on conductor **821080** is maintained at voltage $V_1$. This creates a temporary voltage difference, $|V_2 - V_1|$ between the two conductors **821060** and **821080.**

On the other hand, when the two conductors **821060** and **821080** are in a magnetic resonance imaging scanner environment, the B1 field produced by the magnetic resonance imaging scanner induces voltages in the two conductors **821060** and **821080** of the lead **821040.** The induced voltages are essentially equal in magnitude. Figure 83 provides an illustration of this situation.

Figure 83 illustrates a bipolar pacing system **832000** in magnetic resonance imaging scanner environment. As illustrated, a magnetic resonance imaging scanner induces voltages $V_3$ and $V_4$ in two conductors **832060** and **832080.** As noted above, these induced voltages are essentially equal in magnitude.

If it is assumed that conductor **832060** is the "ground" conductor and conductor **832080** is the "signal conductor," the "ground" conductor's potential is essentially the equal to the "signal" conductor's potential. Therefore, there is no effective "ground" at the distal tip of the pacing lead **832100** in which to implement a voltage filtering mechanism.

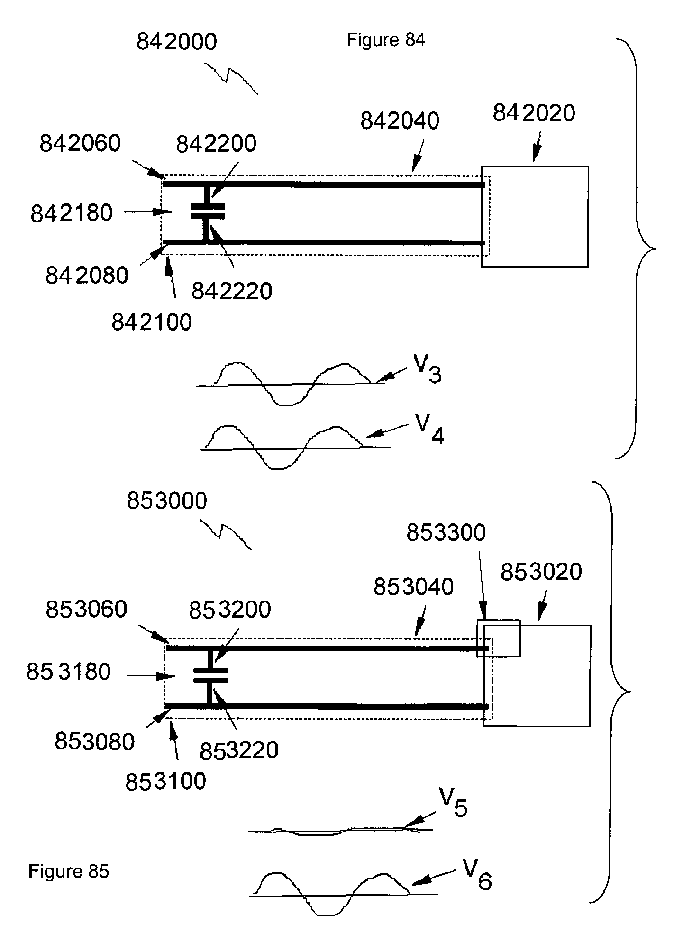

In order for various filters (e.g., a low pass filter) to perform correctly, a significant potential difference must exist. More specifically, when a filter is introduced into the distal tip of a lead to filter or block induced voltages due to the magnetic resonance imaging scanner's radio-frequency field, a significant potential difference must exist between the conductors **832060** and **832080** for the filter to operate properly. Figure 84 provides an illustration of this situation.

Figure 84 illustrates a pacing system **842000** with a lead **842040** including two conductors **842060** and **842080** with a low pass filter being implemented by the insertion of a capacitor **842180** between the two conductors **842060** and **842080.** Since the voltages in the two conductors **842060** and **842080** induced by the magnetic resonance imaging scanner's B1 field are essentially equal in magnitude, charge does not buildup on each side (**842200** and **842220**) of the capacitor **842180.** Therefore, the low pass filter (capacitor **842180**) fails to filter the voltages induced by the magnetic resonance imaging scanner's B1 field.

It is noted that some minor potential difference may occur between the two conductors due to the different construction of the lead. For example, one conductor may be a wound conductor having a different radius than the other conductor. However such a potential difference is not usually sufficient for the filter to function adequately. To ensure an effective potential difference a voltage compensation device is utilized with the lead. An example of a voltage compensation device utilized with a lead is illustrated in Figure 85.

Figure 85 illustrates a pacing system comprising a lead **853040,** a pulse generator **853020,** and a voltage compensation component **853300.** The voltage compensation component **853300** provides a compensation voltage on conductor **853060** so that there is a sufficient voltage difference at the distal tip **853100.** This compensation voltage enable the filter (capacitor **853180**) to effectively function in blocking the induced current in the conductor **853080** from passing through the distal tip electrode of the conductor **853080** and into the body tissue and/or fluid.

It is noted that the voltage compensation component **853300** may be constructed so as to be completely outside the pulse generator **853020,** may be positioned completely inside the pulse generator **853020,** or may be partially inside and partially outside the pulse generator **853020.**

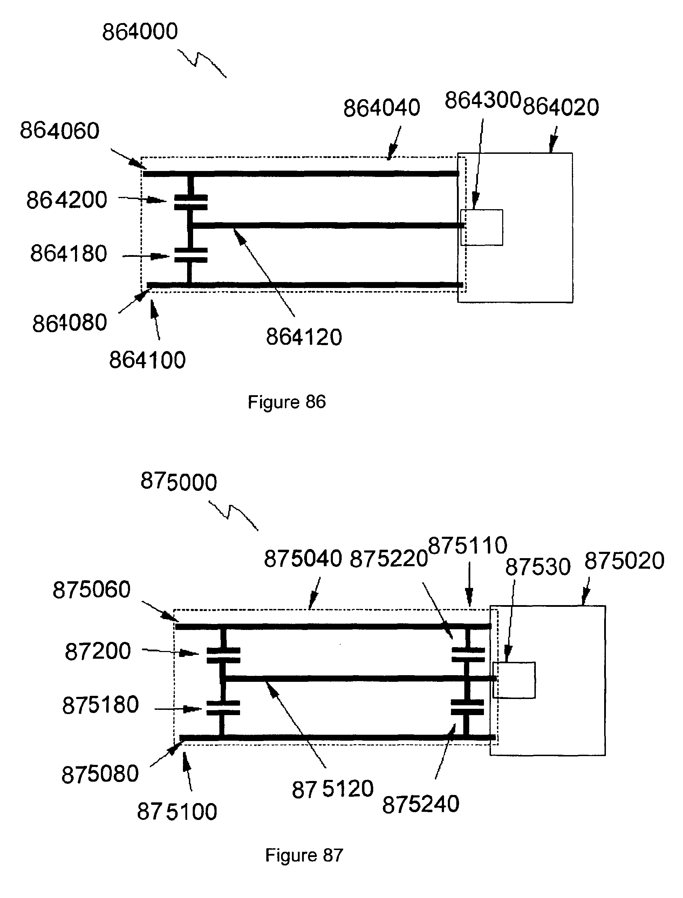

Figure 86 depicts another implementation of a voltage compensation device utilized with a lead in which an additional conductor **864120** is inserted into the lead **864040** together with the two conductors **864060** and **864080.** In this embodiment, the voltage compensation component **864300** is electrically connected to the conductor **864120.** The voltage compensation component **864300** generates a compensation voltage when there are voltages in the three conductors induced by the magnetic resonance imaging scanner's B1 field. This compensation voltage provides a sufficient voltage difference between the conductor **864120** and the conductors **864060** and **864080** so that filters **864200** and **864180** placed in the distal tip **864100** can effectively block the voltages and currents from passing from the conductors **864060** and **864080,** through electrodes (not shown), and into the body tissue and/or fluids.

In one embodiment, the voltage compensation unit **864300** may be a wound wire having N windings or turns. The windings or turns are oriented and positioned to form a coil such that when the magnetic resonance imaging scanner's B1 field is applied to the patient's body, the induced EMF along the lead's conductor, as explained in equations (1) through (4) above, is canceled by the EMF induced in the coil due to Lenz's Law, which states that for a coil with N turns

$$EMF = -N\frac{d\varphi}{dt} \qquad\qquad (5)$$

where $\varphi$ is the magnetic flux

$$\varphi = \vec{B} \circ \vec{A} \qquad\qquad (6)$$

where $\vec{B}$ is the magnetic induction field (B1 field) and $\vec{A}$ is the coil's perpendicular area vector.

In another embodiment, the voltage compensation unit **864300** is connected to a power source in the pulse generator. The voltage compensation unit **864300** generates a desired compensating voltage. Moreover, the voltage compensation unit **864300** may include a sensor or sensors to determine when a compensating voltage is required.

Such sensors may be a magnetic field sensor that senses the presence of the magnetic resonance imaging scanner's B1 field. In this embodiment, if a sensor detects the presence of the magnetic resonance imaging scanner's B1 field, the voltage compensation unit **864300** generates the appropriate compensating voltage.

The sensor may also sense the voltage on a conductor within the lead so as to determine when a voltage is being induced or a voltage level is out of range a predetermined operating range. The information from the sensor can be used to trigger the application of an appropriate compensating voltage, as well as, the magnitude and/or frequency of the appropriate compensating voltage.

Furthermore, the sensors may also sense the voltages on all of the conductors within the lead so as to determine when a voltage is being induced on a particular conductor or a voltage level is out of range a predetermined operating range. The information from the sensors can be used to trigger the application of an appropriate compensating voltage to the appropriate conductor, as well as, the magnitude and/or frequency of the appropriate compensating voltage for the appropriate conductor.

In a further embodiment, as illustrated in Figure 87, filters **875180, 875200, 875220,** and **875240** are placed at both the distal end **875100** and the proximal end **875110** of lead **875040.** The filters **875180** and **875200** at the distal end **875100** of the lead **875040** prevent harmful magnetic resonance imaging induced voltages and currents from passing through the lead's distal tip electrodes (not shown). On the other hand, filters **875220** and **875240** at the proximal end **875110** of the lead **875040** prevent harmful magnetic resonance imaging induced voltages and currents from passing into the pulse generator's circuitry (not shown) contained within pulse generator **875020.**

In this embodiment, the voltage compensation component **875300** is electrically connected to the conductor **875120.** The voltage compensation component **875300** generates a compensation voltage when there are voltages in the three conductors induced by the magnetic resonance imaging scanner's B1 field. This compensation voltage provides a sufficient voltage difference between the conductor **875120** and the conductors **875060** and **875080** so that filters **875200** and **875180** placed in the distal tip **875100** can effectively block the voltages and currents from passing from the conductors **5060** and **5080,** through electrodes (not shown), and into the body tissue and/or fluids. The compensation voltage provides a sufficient voltage difference between the conductor **875120** and the conductors **875060** and **875080** so that filters **875220** and **875240** placed in the proximal tip **875110** can effectively block the voltages and currents from passing from the conductors **875060** and **875080** into the pulse generator's circuitry (not shown) contained within pulse generator **875020.**

In one embodiment, the voltage compensation unit **875300** may be a wound wire having N windings or turns. The windings or turns are oriented and positioned to form a coil such that when the magnetic resonance imaging scanner's B1 field is applied to the patient's body, the induced EMF along the lead's conductor, as explained in equations (1) through (4) above, is canceled by the EMF induced in the coil due to Lenz's Law, which states that for a coil with N turns

$$EMF = -N\frac{d\varphi}{dt} \qquad\qquad (5)$$

where φ is the magnetic flux

$$\varphi = \vec{B} \circ \vec{A} \qquad\qquad (6)$$

where $\vec{B}$ is the magnetic induction field (B1 field) and $\vec{A}$ is the coil's perpendicular area vector.

In another embodiment, the voltage compensation unit **875300** is connected to a power source in the pulse generator. The voltage compensation unit 5300 generates a desired compensating voltage. Moreover, the voltage compensation unit **875300** may include a sensor or sensors to determine when a compensating voltage is required.

Such sensors may be a magnetic field sensor that senses the presence of the magnetic resonance imaging scanner's B1 field. In this embodiment, if a sensor detects the presence of the magnetic resonance imaging scanner's B1 field, the voltage compensation unit **875300** generates the appropriate compensating voltage.

The sensor may also sense the voltage on a conductor within the lead so as to determine when a voltage is being induced or a voltage level is out of range a predetermined operating range. The information from the sensor can be used to trigger the application of an appropriate compensating voltage, as well as, the magnitude and/or frequency of the appropriate compensating voltage.

Furthermore, the sensors may also sense the voltages on all of the conductors within the lead so as to determine when a voltage is being induced on a particular conductor or a voltage level is out of range a predetermined operating range. The information from the sensors can be used to trigger the application of an appropriate compensating voltage to the appropriate conductor, as well as, the magnitude and/or frequency of the appropriate compensating voltage for the appropriate conductor.

In summary, a system reduces the effects of magnetic resonance imaging induced signals to a safe level having a medical device that includes a housing having electronic circuitry therein, a first lead to provide an electrical path for a stimulation signal generated by the electronic circuitry to be applied to a desired tissue region, a second lead to provide an electrical path for a sensed physiological condition of the desired tissue region to be communicated to the electronic circuitry, and a third lead to provide an electrical ground. A diode is connected to the first lead to significantly reduce magnetic resonance imaging induced signals from traveling along the first lead to the electronic circuitry.

In the various embodiments described above, the filter may be an inductor, a resistor, and/or a capacitor. Moreover, in the various embodiments described above, the voltage compensation unit may be a source that generates an electrical potential opposite to that which would be induced by the magnetic resonance imaging switched gradient fields so as to reduce voltages induced by the magnetic resonance imaging switched gradient fields to a safe level or a coil that generates a magnetic resonance imaging switched gradient field induced current opposite to that which would be induced by the magnetic resonance imaging switched gradient fields in the leads so as to reduce voltages induced by the magnetic resonance imaging switched gradient fields to a safe level wherein the coil may be curved in three different spatial directions. The voltage compensation unit having the coil may also include an orientation subsystem for changing a spatial orientation of the coil to modify the strength of the magnetic resonance imaging switched gradient field induced current.

The voltage compensation unit may include a plurality of coils, each coil generating a magnetic resonance imaging switched gradient field induced current opposite to that which would be induced by the magnetic resonance imaging switched gradient fields in the leads so as to reduce voltages induced by the magnetic resonance imaging switched gradient fields to a safe level, and a switch connecting independently a number of the plurality of the coils, the number of connected coils corresponding to an amount of the voltage induced by the magnetic resonance imaging switched gradient fields and a current level produced in each coil when the sensor senses the application of switched magnetic resonance imaging gradient fields wherein the coils are curved in three different spatial directions. The voltage compensation unit may also include an orientation subsystem for changing a spatial orientation of the coils to modify the strength of the magnetic resonance imaging switched gradient field induced currents.

The voltage compensation unit may be three orthogonally planar coils, each coil generating a magnetic resonance imaging switched gradient field induced current opposite to that which would be induced by the magnetic resonance imaging switched gradient fields in the leads so as to reduce voltages induced by the magnetic resonance imaging switched gradient fields to a safe level, and a switch connecting independently a number of the coils, the number of connected coils corresponding to an amount of the voltage induced by the magnetic resonance imaging switched gradient fields and a current level produced in each coil when the sensor senses the application of switched magnetic resonance imaging gradient fields. The voltage compensation unit may also include an orientation subsystem for changing a spatial orientation of the coils to modify the strength of the magnetic resonance imaging switched gradient field induced currents wherein each coil defines a distinct plane that transverses the magnetic resonance imaging switched gradient fields.

The voltage compensation unit may also include a receiver to receive a signal from a magnetic resonance imaging system indicating an application of switched magnetic resonance imaging gradient fields. The voltage compensation unit may be a source that generates an electrical potential opposite to that which would be induced by the magnetic resonance imaging switched gradient fields so as to reduce voltages induced by the magnetic resonance imaging switched gradient fields to a safe level or a coil that generates a magnetic resonance imaging switched gradient field induced current opposite to that which would be induced by the magnetic resonance imaging switched gradient fields in the leads so as to reduce voltages induced by the magnetic resonance imaging switched gradient fields to a safe level wherein the coil may be curved in three different spatial directions. The voltage compensation unit having the coil may also include an orientation subsystem for changing a spatial orientation of the coil to modify the strength of the magnetic resonance imaging switched gradient field induced current.

The voltage compensation unit may include a plurality of coils, each coil generating a magnetic resonance imaging switched gradient field induced current opposite to that which would be induced by the magnetic resonance imaging switched gradient fields in the leads so as to reduce voltages induced by the magnetic resonance imaging switched gradient fields to a safe level, and a switch connecting independently a number of the plurality of the coils, the number of connected coils corresponding to an amount of the voltage induced by the magnetic resonance imaging switched gradient fields and a current level produced in each coil when the sensor senses the application of switched magnetic resonance imaging gradient fields wherein the coils are curved in three different spatial directions. The voltage compensation unit may also include an orientation subsystem for changing a spatial orientation of the coils to modify the strength of the magnetic resonance imaging switched gradient field induced currents.

The voltage compensation unit may be three orthogonally planar coils, each coil generating a magnetic resonance imaging switched gradient field induced current opposite to that which would be induced by the magnetic resonance imaging switched gradient fields in the leads so as to reduce voltages induced by the magnetic resonance imaging switched gradient fields to a safe level, and a switch connecting independently a number of the coils, the number of connected coils corresponding to an amount of the voltage induced by the magnetic resonance imaging switched gradient fields and a current level produced in each coil when the sensor senses the application of switched magnetic resonance imaging gradient fields. The voltage compensation unit may also include an orientation subsystem for changing a spatial orientation of the coils to modify the strength of the magnetic resonance imaging switched gradient field induced currents wherein each coil defines a distinct plane that transverses the magnetic resonance imaging switched gradient fields.

In a further embodiment, the present invention is directed to an electrical lead component for a medical device that reduces the effects of magnetic resonance imaging induced signals to a safe level. The electrical lead component includes a medical device electrical lead capable of providing an electrical path to a desired tissue region and a coil, a voltage compensation unit, that generates a magnetic resonance imaging switched gradient field induced current opposite to that which would be induced by the magnetic resonance imaging switched gradient fields in the medical device electrical lead so as to reduce voltages induced by the magnetic resonance imaging switched gradient fields to a safe level. The coil may be curved in three different spatial directions.

The embodiment may further include an orientation subsystem for changing a spatial orientation of the coil to modify the strength of the magnetic resonance imaging switched gradient field induced current.

In a further embodiment, the present invention is directed to an electrical lead component for a medical device that reduces the effects of magnetic resonance imaging induced signals to a safe level. The electrical lead component includes a medical device electrical lead capable of providing an electrical path to a desired tissue region and a plurality of coils, a voltage compensation unit, each coil generating a magnetic resonance imaging switched gradient field induced current such a combination of the magnetic resonance imaging switched gradient field induced currents provide a combined current that is opposite to that which would be induced by the magnetic resonance imaging switched gradient fields in the medical device electrical lead so as to reduce voltages induced by the magnetic resonance imaging switched gradient fields to a safe level. The coil may be curved in three different spatial directions.

The embodiment may further include an orientation subsystem for changing a spatial orientation of the coil to modify the strength of the magnetic resonance imaging switched gradient field induced current.

In a further embodiment, the present invention is directed to an electrical lead component for a medical device that reduces the effects of magnetic resonance imaging induced signals to a safe level. The electrical lead component includes a medical device electrical lead capable of providing an electrical path to a desired tissue region and three orthogonally planar coils, a voltage compensation unit, each coil generating a magnetic resonance imaging switched gradient field induced current such a combination of the magnetic resonance imaging switched gradient field induced currents provide a combined current that is opposite to that which would be induced by the magnetic resonance imaging switched gradient fields in the medical device electrical lead so as to reduce voltages induced by the magnetic resonance imaging switched gradient fields to a safe level.

In a further embodiment, the present invention is directed to an electrical lead component for a medical device that reduces the effects of magnetic resonance imaging induced signals to a safe level. The electrical lead component includes a medical device electrical lead capable of providing an electrical path to a desired tissue region and a voltage compensation unit. The voltage compensation unit includes a plurality of coils, each coil generating a magnetic resonance imaging switched gradient field induced current; a sensor to measure a strength of voltages induced by the magnetic resonance imaging switched gradient fields; and a switching device, operatively connected to the sensor and plurality of coils, to operatively connect a number of the plurality of coils in response to the measured strength of voltages induced by the magnetic resonance imaging switched gradient fields such that a combination of the magnetic resonance imaging switched gradient field induced currents produced by the number of operatively connected coils provide a combined current that is opposite to that which would be induced by the magnetic resonance imaging switched gradient fields in the medical device electrical lead so as to reduce voltages induced by the magnetic resonance imaging switched gradient fields to a safe level. The coils may be curved in three different spatial directions.

The embodiment may further include an orientation subsystem for changing a spatial orientation of the coil to modify the strength of the magnetic resonance imaging switched gradient field induced current.

In a further embodiment, the present invention is directed to an electrical lead component for a medical device that reduces the effects of magnetic resonance imaging induced signals to a safe level. The electrical lead component includes a medical device electrical lead capable of providing an electrical path to a desired tissue region and a voltage compensation unit. The voltage compensation unit includes three orthogonally planar coils, each coil generating a magnetic resonance imaging switched gradient field induced current; a sensor to measure a strength of voltages induced by the magnetic resonance imaging switched gradient fields; and a switching device, operatively connected to the sensor and the coils, to operatively connect a number of the coils in response to the measured strength of voltages induced by the magnetic resonance imaging switched gradient fields such that a combination of the magnetic resonance imaging switched gradient field induced currents produced by the number of operatively connected coils provide a combined current that is opposite to that which would be induced by the magnetic resonance imaging switched gradient fields in the medical device electrical lead so as to reduce voltages induced by the magnetic resonance imaging switched gradient fields to a safe level.

In a further embodiment, the present invention is directed to an electrical lead component for a medical device that reduces the effects of magnetic resonance imaging induced signals to a safe level. The electrical lead component includes a medical device electrical lead capable of providing an electrical path to a desired tissue region and a voltage compensation unit. The voltage compensation unit includes a plurality of coils, each coil generating a magnetic resonance imaging switched gradient field induced current; a transceiver to receive a signal indicating a number of coils to be connected; and a switching device, operatively connected to the transceiver and plurality of coils, to operatively connect a number of the plurality of coils in response to the received signal indicating the number of coils to be connected such that a combination of the magnetic resonance imaging switched gradient field induced currents produced by the number of operatively connected coils provide a combined current that is opposite to that which would be induced by the magnetic resonance imaging switched gradient fields in the medical device electrical lead so as to reduce voltages induced by the magnetic resonance imaging switched gradient fields to a safe level. The coils may be curved in three different spatial directions.

The embodiment may further include an orientation subsystem for changing a spatial orientation of the coil to modify the strength of the magnetic resonance imaging switched gradient field induced current.

In a further embodiment, the present invention is directed to an electrical lead component for a medical device that reduces the effects of magnetic resonance imaging induced signals to a safe level. The electrical lead component includes a medical device electrical lead capable of providing an electrical path to a desired tissue region and a voltage compensation unit. The voltage compensation unit includes three orthogonally planar coils, each coil generating a magnetic resonance imaging switched gradient field induced current; a transceiver to receive a signal indicating a number of coils to be connected; and a switching device, operatively connected to the transceiver and the coils, to operatively connect a number of the coils in response to the received signal indicating the number of coils to be connected such that a combination of the magnetic resonance imaging switched gradient field induced currents produced by the number of operatively connected coils provide a combined current that is opposite to that which would be induced by the magnetic resonance imaging switched gradient fields in the medical device electrical lead so as to reduce voltages induced by the magnetic resonance imaging switched gradient fields to a safe level.

In a further embodiment, the present invention is directed to a medical device that reduces the effects of magnetic resonance imaging induced signals to a safe level. The medical device includes a medical device capable of providing medical treatment to a desired tissue region and a coil, a voltage compensation unit, that generates a magnetic resonance imaging switched gradient field induced current opposite to that which would be induced by the magnetic resonance imaging switched gradient fields in the medical device so as to reduce voltages induced by the magnetic resonance imaging switched gradient fields to a safe level. The coil may be curved in three different spatial directions.

The embodiment may further include an orientation subsystem for changing a spatial orientation of the coil to modify the strength of the magnetic resonance imaging switched gradient field induced current.

In a further embodiment, the present invention is directed to a medical device that reduces the effects of magnetic resonance imaging induced signals to a safe level. The medical device includes a medical device capable of providing medical treatment to a desired tissue region and a plurality of coils, a voltage compensation unit, each coil generating a magnetic resonance imaging switched gradient field induced current such a combination of the magnetic resonance imaging switched gradient field induced currents provide a combined current that is opposite to that which would be induced by the magnetic resonance imaging switched gradient fields in the medical device so as to reduce voltages induced by the magnetic resonance imaging switched gradient fields to a safe level. The coils may be curved in three different spatial directions.

The embodiment may further include an orientation subsystem for changing a spatial orientation of the coil to modify the strength of the magnetic resonance imaging switched gradient field induced current.

In a further embodiment, the present invention is directed to a medical device that reduces the effects of magnetic resonance imaging induced signals to a safe level. The medical device includes a medical device capable of providing medical treatment to a desired tissue region and three orthogonally planar coils, a voltage compensation unit, each coil generating a magnetic resonance imaging switched gradient field induced current such a combination of the magnetic resonance imaging switched gradient field induced currents provide a combined current that is opposite to that which would be induced by the magnetic resonance imaging switched gradient fields in the medical device so as to reduce voltages induced by the magnetic resonance imaging switched gradient fields to a safe level.

In a further embodiment, the present invention is directed to a medical device that reduces the effects of magnetic resonance imaging induced signals to a safe level. The medical device includes a medical device capable of providing medical treatment to a desired tissue region and a voltage compensation unit. The voltage compensation unit includes a plurality of coils, each coil generating a magnetic resonance imaging switched gradient field induced current; a sensor to measure a strength of voltages induced by the magnetic resonance imaging switched gradient fields; and a switching device, operatively connected to the sensor and plurality of coils, to operatively connect a number of the plurality of coils in response to the measured strength of voltages induced by the magnetic resonance imaging switched gradient fields such that a combination of the magnetic resonance imaging switched gradient field induced currents produced by the number of operatively connected coils provide a combined current that is opposite to that which would be induced by the magnetic resonance imaging switched gradient fields in the medical device so as to reduce voltages induced by the magnetic resonance imaging switched gradient fields to a safe level. The coils may be curved in three different spatial directions.

The embodiment may further include an orientation subsystem for changing a spatial orientation of the coil to modify the strength of the magnetic resonance imaging switched gradient field induced current.

In a further embodiment, the present invention is directed to a medical device that reduces the effects of magnetic resonance imaging induced signals to a safe level. The medical device includes a medical device capable of providing medical treatment to a desired tissue region and a voltage compensation unit. The voltage compensation unit includes three orthogonally planar coil, each coil generating a magnetic resonance imaging switched gradient field induced current; a sensor to measure a strength of voltages induced by the magnetic resonance imaging switched gradient fields; and a switching device, operatively connected to the sensor and plurality of coils, to operatively connect a number of the plurality of coils in response to the measured strength of voltages induced by the magnetic resonance imaging switched gradient fields such that a combination of the magnetic resonance imaging switched gradient field induced currents produced by the number of operatively connected coils provide a combined current that is opposite to that which would be induced by the magnetic resonance imaging switched gradient fields in the medical device so as to reduce voltages induced by the magnetic resonance imaging switched gradient fields to a safe level.

In a further embodiment, the present invention is directed to a medical device that reduces the effects of magnetic resonance imaging induced signals to a safe level. The medical device includes a medical device capable of providing medical treatment to a desired tissue region and a voltage compensation unit. The voltage compensation unit includes a plurality of coils, each coil generating a magnetic resonance imaging switched gradient field induced current; a transceiver to receive a signal indicating a number of coils to be connected; and a switching device, operatively connected to the transceiver and the coils, to operatively connect a number of the coils in response to the received signal indicating the number of coils to be connected such that a combination of the magnetic resonance imaging switched gradient field induced currents produced by the number of operatively connected coils provide a combined current that is opposite to that which would be induced by the magnetic resonance imaging switched gradient fields in the medical device electrical lead so as to reduce voltages induced by the magnetic resonance imaging switched gradient fields to a safe level. The coils may be curved in three different spatial directions.

The embodiment may further include an orientation subsystem for changing a spatial orientation of the coil to modify the strength of the magnetic resonance imaging switched gradient field induced current.

In a further embodiment, the present invention is directed to a medical device that reduces the effects of magnetic resonance imaging induced signals to a safe level. The medical device includes a medical device capable of providing medical treatment to a desired tissue region and a voltage compensation unit. The voltage compensation unit includes three orthogonally planar coil, each coil generating a magnetic resonance imaging switched gradient field induced current; a transceiver to receive a signal indicating a number of coils to be connected; and a switching device, operatively connected to the transceiver and the coils, to operatively connect a number of the coils in response to the received signal indicating the number of coils to be connected such that a combination of the magnetic resonance imaging switched gradient field induced currents produced by the number of operatively connected coils provide a combined current that is opposite to that which would be induced by the magnetic resonance imaging switched gradient fields in the medical device electrical lead so as to reduce voltages induced by the magnetic resonance imaging switched gradient fields to a safe level.

In a further embodiment, the present invention is directed to a voltage control unit that reduces the effects of magnetic resonance imaging induced signals upon a medical device to a safe level. The voltage control unit includes a coil that generates a magnetic resonance imaging switched gradient field induced current opposite to that which would be induced by the magnetic resonance imaging switched gradient fields in the medical device so as to reduce voltages induced by the magnetic resonance imaging switched gradient fields to a safe level. The coil may be curved in three different spatial directions.

The embodiment may further include an orientation subsystem for changing a spatial orientation of the coil to modify the strength of the magnetic resonance imaging switched gradient field induced current.

In a further embodiment, the present invention is directed to a voltage control unit that reduces the effects of magnetic resonance imaging induced signals upon a medical device to a safe level. The voltage control unit includes a plurality of coils, each coil generating a magnetic resonance imaging switched gradient field induced current such a combination of the magnetic resonance imaging switched gradient field induced currents provide a combined current that is opposite to that which would be induced by the magnetic resonance imaging switched gradient fields in the medical device so as to reduce voltages induced by the magnetic resonance imaging switched gradient fields to a safe level. The coils may be curved in three different spatial directions.

The embodiment may further include an orientation subsystem for changing a spatial orientation of the coil to modify the strength of the magnetic resonance imaging switched gradient field induced current.

In a further embodiment, the present invention is directed to a voltage control unit that reduces the effects of magnetic resonance imaging induced signals upon a medical device to a safe level. The voltage control unit includes three orthogonally planar coils, each coil generating a magnetic resonance imaging switched gradient field induced current such a combination of the magnetic resonance imaging switched gradient field induced currents provide a combined current that is opposite to that which would be induced by the magnetic resonance imaging switched gradient fields in the medical device so as to reduce voltages induced by the magnetic resonance imaging switched gradient fields to a safe level.

In a further embodiment, the present invention is directed to a voltage control unit that reduces the effects of magnetic resonance imaging induced signals upon a medical device to a safe level. The voltage control unit includes a plurality of coils, each coil generating a magnetic resonance imaging switched gradient field induced current; a sensor to measure a strength of voltages induced by the magnetic resonance imaging switched gradient fields; and a switching device, operatively connected to the sensor and plurality of coils, to operatively connect a number of the plurality of coils in response to the measured strength of voltages induced by the magnetic resonance imaging switched gradient fields such that a combination of the magnetic resonance imaging switched gradient field induced currents produced by the number of operatively connected coils provide a combined current that is opposite to that which would be induced by the magnetic resonance imaging switched gradient fields in the medical device so as to reduce voltages induced by the magnetic resonance imaging switched gradient fields to a safe level. The coils may be curved in three different spatial directions.

The embodiment may further include an orientation subsystem for changing a spatial orientation of the coil to modify the strength of the magnetic resonance imaging switched gradient field induced current.

In a further embodiment, the present invention is directed to a voltage control unit that reduces the effects of magnetic resonance imaging induced signals upon a medical device to a safe level. The voltage control unit includes three orthogonally planar coil, each coil generating a magnetic resonance imaging switched gradient field induced current; a sensor to measure a strength of voltages induced by the magnetic resonance imaging switched gradient fields; and a switching device, operatively connected to the sensor and plurality of coils, to operatively connect a number of the plurality of coils in response to the measured strength of voltages induced by the magnetic resonance imaging switched gradient fields such that a combination of the magnetic resonance imaging switched gradient field induced currents produced by the number of operatively connected coils provide a combined current that is opposite to that which would be induced by the magnetic resonance imaging switched gradient fields in the medical device so as to reduce voltages induced by the magnetic resonance imaging switched gradient fields to a safe level.

In a further embodiment, the present invention is directed to a voltage control unit that reduces the effects of magnetic resonance imaging induced signals upon a medical device to a safe level. The voltage control unit includes a plurality of coils, each coil generating a magnetic resonance imaging switched gradient field induced current; a transceiver to receive a signal indicating a number of coils to be connected; and a switching device, operatively connected to the transceiver and the coils, to operatively connect a number of the coils in response to the received signal indicating the number of coils to be connected such that a combination of the magnetic resonance imaging switched gradient field induced currents produced by the number of operatively connected coils provide a combined current that is opposite to that which would be induced by the magnetic resonance imaging switched gradient fields in the medical device electrical lead so as to reduce voltages induced by the magnetic resonance imaging switched gradient fields to a safe level. The coils may be curved in three different spatial directions.

The embodiment may further include an orientation subsystem for changing a spatial orientation of the coil to modify the strength of the magnetic resonance imaging switched gradient field induced current.

In a further embodiment, the present invention is directed to a voltage control unit that reduces the effects of magnetic resonance imaging induced signals upon a medical device to a safe level. The voltage control unit includes three orthogonally planar coil, each coil generating a magnetic resonance imaging switched gradient field induced current; a transceiver to receive a signal indicating a number of coils to be connected; and a switching device, operatively connected to the transceiver and the coils, to operatively connect a number of the coils in response to the received signal indicating the number of coils to be connected such that a combination of the magnetic resonance imaging switched gradient field induced currents produced by the number of operatively connected coils provide a combined current that is opposite to that which would be induced by the magnetic resonance imaging switched gradient fields in the medical device electrical lead so as to reduce voltages induced by the magnetic resonance imaging switched gradient fields to a safe level.

In a further embodiment, the present invention is directed to a lead for medical applications that reduces the effects of magnetic resonance imaging induced signals to a safe level. The lead includes two coiled conductive strands forming a spring-like configuration such that current flows over a surface thereof, through contact points between adjacent loops of the coiled conductive strands, and an insulating coating formed over a portion of the two coiled conductive strands such that an inline inductive element is formed, the current flowing along a curvature of the two coiled conductive strands in the insulating coated portion of two coiled conductive strands.

This embodiment may further include a ferrite material positioned in the portion of the two-coiled conductive strands having the insulating coating formed thereon.

In a further embodiment, the present invention is directed to a lead for medical applications that reduces the effects of magnetic resonance imaging induced signals to a safe level. The lead includes two coiled conductive strands forming a spring-like configuration such that current flows over a surface thereof, through contact points between adjacent loops of the coiled conductive strands, and an adjustable resistive material formed over a portion of the two coiled conductive strands such that an inline inductive element is formed, the current flowing along a curvature of the two coiled conductive strands in the adjustable resistive material portion of two coiled conductive strands, an inductance of the inline inductive element being adjusted by adjusting the resistive properties of the adjustable resistive material.

This embodiment may further include a ferrite material positioned in the portion of the two-coiled conductive strands having the insulating coating formed thereon.

In a further embodiment, the present invention is directed to a voltage compensation unit for reducing the effects of induced voltages upon a medical device to a safe level. The voltage compensation unit includes a connection device to provide an electrical connection to the medical device; a sensing circuit to voltages of conductive components in the medical device; and a compensation circuit, operatively connected to the sensing circuit and responsive thereto, to provide opposing voltages to the medical device to reduce the effects of induced voltages caused by changing magnetic fields.

This embodiment may also include a power supply, such as a battery or a connection to an external power source. The embodiment may include a second sensing circuit to detect the changing magnetic fields and a compensation circuit, connected to the second sensing circuit and responsive thereto, to synchronize application of the opposing voltages to the medical device with the sensed changing magnetic fields.

In a further embodiment, the present invention is directed to a voltage compensation unit for reducing the effects of induced voltages upon a medical device to a safe level. The voltage compensation unit includes a connection device to provide an electrical connection to the medical device; a sensing circuit to detect changing magnetic fields; and a compensation circuit, operatively connected to the sensing circuit and responsive thereto, to synchronize application of opposing voltages to the medical device with the sensed changing magnetic fields, the opposing voltages reducing the effects of induced voltages caused by the changing magnetic fields.

This embodiment may also include a power supply, such as a battery or a connection to an external power source.

In a further embodiment, the present invention is directed to a voltage compensation unit for reducing the effects of induced voltages upon a medical device to a safe level. The voltage compensation unit includes a connection device to provide an electrical connection to the medical device; a communication circuit, communicatively linked to a magnetic resonance imaging system, to receive information associated with a start and end of an application of changing magnetic fields produced by the magnetic resonance imaging system; and a compensation circuit, operatively connected to the communication circuit and responsive thereto, to synchronize application of opposing voltages to the medical device with the sensed changing magnetic fields, the opposing voltages reducing the effects of induced voltages caused by the changing magnetic fields.

This embodiment may also include a power supply, such as a battery or a connection to an external power source. The communication circuit may receive information associated with field strengths to be applied by the magnetic resonance imaging system, and the compensation circuit would apply opposing voltages in accordance with communicated applied field strengths. The communication circuit may receive the information through electrical wires, coaxial wires, shielded wires, optical fibers, and/or a radio-frequency transmitter/receiver.

In a further embodiment, the present invention is directed to a voltage compensation unit for reducing the effects of induced voltages upon a medical device to a safe level. The voltage compensation unit includes a connection device to provide an electrical connection to the medical device; a communication circuit, communicatively linked to a magnetic resonance imaging system, to receive information associated with a start and end of an application of changing magnetic fields produced by the magnetic resonance imaging system; and a compensation circuit, operatively connected to the communication circuit and responsive thereto, to apply opposing voltages to the medical device, the opposing voltages reducing the effects of induced voltages caused by the changing magnetic fields.

This embodiment may also include a power supply, such as a battery or a connection to an external power source. The communication circuit may receive information associated with field strengths to be applied by the magnetic resonance imaging system, and the compensation circuit would apply opposing voltages in accordance with communicated applied field strengths. The communication circuit may receive the information through electrical wires, coaxial wires, shielded wires, optical fibers, and/or a radio-frequency transmitter/receiver.

In a further embodiment, the present invention is directed to a medical assembly. The medical assembly includes a multi-wire lead having a plurality of loops. The multi-wire lead has a gap region and a non-gap region. The gap region has adjacent loops of the multi-wire lead with gaps therebetween so as to form impedance in the multi-wire lead. The non-gap region has adjacent loops of the multi-wire lead with no gaps therebetween. The gaps may provide inter-loop capacitance. The multi-wire lead may have a plurality of gap regions, each gap region having a non-gap region therebetween.

In another embodiment, the present invention is directed to a medical assembly. The medical assembly includes a multi-wire lead having a plurality of wires, each wire having a plurality of loops. The multi-wire lead has a gap region and a non-gap region, the gap region having a first gap between a loop of a first wire and a loop of a second wire and the non-gap region having a second gap between a loop of a first wire and a loop of a second wire. The first gap is greater than said second gap. The gap region may form impedance in the multi-wire lead.

In another embodiment, the present invention is directed to a medical assembly. The medical assembly includes a multi-wire lead having a plurality of wires, each wire having a plurality of loops. The multi-wire lead has a gap region and a non-gap region, the gap region having a first lead intra-loop gap between a first loop of a first wire and a second loop of the first wire and the non-gap region having a second lead intra-loop gap between a third loop of the first wire and a fourth loop of the first wire. The first lead intra-loop gap is greater than said lead intra-loop second gap. The gap region may form impedance in the multi-wire lead. The multi-wire lead may have an intra-loop inter-wire gap between adjacent wires.

In another embodiment, the present invention is directed to a medical assembly. The medical assembly includes a multi-wire lead having a plurality of conductive wires and a plurality of non-conductive wires, each conductive wire having a plurality of loops, each non-conductive wire having a plurality of loops. The loops of a non-conductive wire are positioned between adjacent loops of two conductive wires to form a lead intra-loop non-conductive gap between the adjacent loops of two conductive wires. The multi-wire lead may have a gap region and a non-gap region, the gap region having a first lead intra-loop gap between a first loop of a first wire and a second loop of the first wire and the non-gap region having a second lead intra-loop gap between a third loop of the first wire and a fourth loop of the first wire. The first lead intra-loop gap is greater than said lead intra-loop second gap. The gap region may form different impedance in the gap region than an impedance in the non-gap region.

The equation giving the induced Electric Field dues to a rotation magnetic field, as in a magnetic resonance imaging scanner is given be Maxwell's equation:

$$\nabla \times \vec{E} = -\frac{\partial \vec{B}}{\partial t} \qquad (1)$$

For the rotating $\vec{B}_1$ field,

$$\vec{B} = B_1 \sin(\omega t)\, \hat{x} + B_1 \cos(\omega t)\, \hat{y} \qquad (2)$$

where $B_1$ is the magnetic amplitude function, $\omega$ is the angular frequency of the rotating magnetic field and $\hat{x}$ and $\hat{y}$ are unit vectors in the x and y axis directions, respectively, the electric field equation is given by

$$\vec{E} = -B_1\, \omega \left( x \sin(\omega t) + y \cos(\omega t) \right) \hat{z} \qquad (3)$$

where z is the unit vector in the z-axis direction.

The induced EMF is then the line integral along the path of the lead conductors

$$EMF = \int \vec{E} \circ d\vec{s} \qquad (4)$$

The induced EMF in a coil of wire is as given by Lenz's Law, which states that for a coil with N-turns

$$EMF = -N\frac{d\varphi}{dt} \qquad (5)$$

where $\varphi$ is the magnetic flux

$$\varphi = \vec{B} \circ \vec{A} \qquad (6)$$

where $\vec{B}$ is the magnetic induction field ($B_1$ field) and $\vec{A}$ is the coil's perpendicular area vector.

A lead comprising conductive wire may be so constructed that when placed in a rotating magnetic field, with appropriate orientation, that the induced EMF generated through the mechanism outline in equations (1) through (4) along a short section of the conductive wire may be essentially canceled by the induced EMF generated through the mechanism outlined in equations (5) and (6) applied to an adjacent short section of the conductive wire.

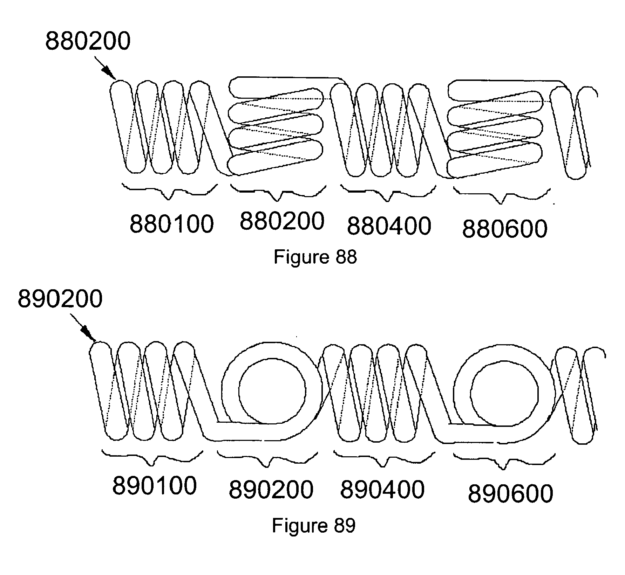

Such a coiled conductive wire is illustrated in Figure 88. The coiled wire **880200** comprises short sections **880100, 880400** coiled along the axis of the lead length and short sections **880200, 880600** coiled perpendicular to the leads length.

Figure 89 depicts the same coiled wire section as in Figure 88 from a different orientation.

Different coiling designs may be needed for leads placed in different paths through the body. Because each different path will have a different amount of the lead running parallel to the magnetic resonance imaging scanner's z-axis, a different number of perpendicular coils **880200** (**890200**), **880600** (**890600**) per unit length will be needed to eliminate the magnetic resonance imaging induced current through the lead.

In another embodiment, the number of perpendicular coils **880200** (**890200**), **880600** (**890600**) per unit length of the lead is varied along the leads total length such that when positioned in the patient's body, those sections of the lead which run essentially perpendicular to the scanner's z-axis (in an xy- plane) has little to no perpendicular coils **880200** (**890200**), **880600** (**890600**) while those sections of the lead which run essentially parallel to the scanner's z-axis have many perpendicular coils **880200** (**890200**), **880600** (**890600**).

It is important that the lead be oriented properly. Improperly orienting the coiled lead (for example, rotating the lead about its length axis by 180 degrees) will enhance the magnetic resonance imaging induced currents, not retard the magnetic resonance imaging induced currents.

As noted above, the present invention is directed to a device which enables imaging and visualization of the inner lumen of an implanted stent by means of a magnetic resonance imaging technique.

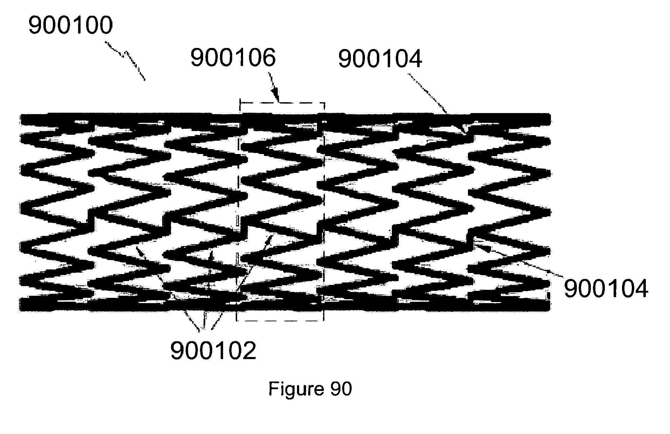

As illustrated in Figure 90, a stent structure **900100** is generally of a cylinder-like shape comprises a mesh of conductive struts **900102, 900104.** In the embodiment shown the stent structure **900100** comprises zig-zag rings **900106** composed of conductive struts **900102.** The zig-zag rings **900106** are connected to each other by connecting struts **900104** thus forming a conductive path from one zig-zag ring **900106** to next.

Such a stent structure **900100** of generally a cylinder shaped conductive mesh **900100** forms a Faraday cage that supports induced electrical currents through its conductive struts **900102** and **900104** when an oscillating magnetic field, e.g. the $B_1$ magnetic field of a magnetic resonance imaging scanner, is applied to a patient, in whom the stent structure **900100** has been embedded. The $B_1$ magnetic field is also known as the rotating magnetic field. Thus the conductive stent structure **100** shields its interior from the excitation $B_1$ field of the magnetic resonance imaging scanner resulting in poor quality images of the stent's lumen and material therein.

It is to be understood that the stent structure **900100** is but one example of one possible stent structure to which the concepts of the present invention is to be applied. Other stent structures in use will also benefit from the concepts of the present invention but are not shown herein.

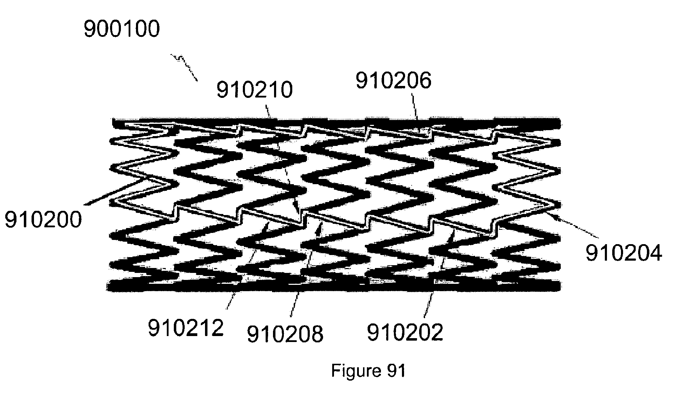

Figure 91 illustrates a stent structure that includes the stent structure **900100** of Figure 90 and an RLC circuit **910200** deposited over some of the stent's struts. As illustrated in Figure 91, the RLC circuit **910200** includes sections **910212, 910208, 910202** and **910206** that overlay the stent struts of stent structure **900100.** Moreover, as illustrated in Figure 91, the RLC circuit **910200** includes sections **910210** and **910204** that overlay zig-zag rings of stent structure **900100** between the stent struts of stent structure **900100.**

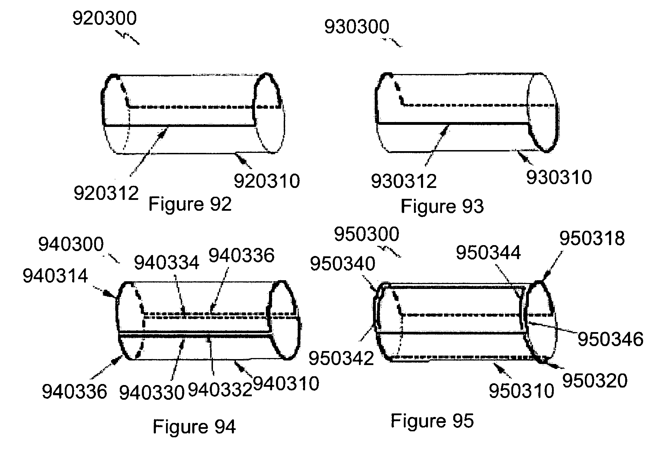

Figures 92-95 illustrate four different configurations of the RLC circuit, following the stent's struts. In Figure 92, the stent structure **920300** includes a stent **920310,** shown as a cylinder for ease of visualization, and a RLC circuit **920312,** shown as straight and half-circle lines. In this embodiment, the two half-circle lines of the circuit's conductive path are both go "up and around" the ends of the stents. In other words, the two half-circle lines of the circuit's conductive path are located in the same hemisphere of the stent structure **920300.**

In Figure 93, the stent structure **930300** includes a stent **930310,** shown as a cylinder for ease of visualization, and a RLC circuit **930312,** shown as straight and half-circle lines. In this embodiment, one of the two half-circle lines of the circuit's conductive path goes "up and around," while the other half-circle conductive path goes "down and around" along the stent's end. In other words, one of the half-circle lines of the circuit's conductive path is located in one hemisphere of the stent structure **930300,** and the other of the half-circle lines of the circuit's conductive path is located in the other hemisphere of the stent structure **930300.**

In Figure 94, the stent structure **940300** includes a stent **940310,** shown as a cylinder for ease of visualization, a first RLC circuit **940314,** shown as straight and half-circle lines, and a second RLC circuit **940316,** shown as straight and half-circle lines. In this embodiment, a conductive trace portion **940330** of second RLC circuit **940316** and a conductive trace portion **940332** of first RLC circuit **940314** may be deposited one on top of the other with an electrically insulative material interposed between the two conductive traces. Moreover, a conductive trace portion **940334** of second RLC circuit **940316** and a conductive trace portion **940336** of first RLC circuit **940314** may be deposited one on top of the other with an electrically insulative material interposed between the two conductive traces.

Figure 95, the stent structure **950300** includes a stent **950310,** shown as a cylinder for ease of visualization, a first RLC circuit **950318,** shown as straight and half-circle lines, and a second RLC circuit **950320,** shown as straight and half-circle lines. In this embodiment, the two RLC circuits **950318** and **950320** are positioned such that one circuit is oriented approximately 90˚ around the stent with respect to the position of the other circuit. The end half-circle **950340** of second RLC circuit **950320** may partially overlap the end half-circle **950342** of first RLC circuit **950318.** In addition, the end half-circle **950346** of first RLC circuit **950318** may partially overlap the end half-circle **950344** of second RLC circuit **950320.** An electrically insulative material is interposed between any portion of the two circuits that overlap each other. Other configurations involving one of more RLC circuits are also possible.

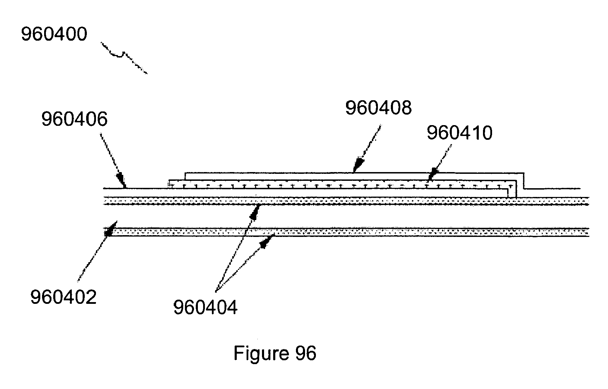

Figure 96 depicts the formation of a capacitive element of the circuit. In the embodiment depicted in Figure 96, one or more portions of one or more struts **960402** are coated with an electrically insulative material **960404.** In one embodiment, electrically insulative material **960404** is deposited over all the surfaces of the stent. In another embodiment, electrically insulative material **960404** is deposited over only those struts to which a portion of an RLC circuit is to be deposited.

Continuing to refer to Figure 96 and to the embodiment therein, a conductive trace **960406** is deposited over a portion of the electrically insulative material **960404.** Conductive trace **960406** maybe a portion of the conductive trace forming the inductor (not shown) of the RLC. The conductive trace **960406** may be a cured silver conductive ink, a cured gold conductive ink, or other conductive ink.

As depicted in Figure 96, over a portion of the conductive trace **960406** is deposited a dielectric material **960410,** which, in the embodiment depicted, is also deposited over a portion of the electrically insulative material **960404.** In one embodiment, the dielectric material **960410** is the same material as the electrically insulative material **960404.** In another embodiment, the dielectric material **960410** is not the same of material as the electrically insulative material **960404.**

Continuing to refer to Figure 96 and to the embodiment therein, a conductive trace **960408** is placed over a portion of the dielectric material **960410.** In one embodiment, the conductive trace is the second conductive end of the continuous conductive trace **960406.** The overlapping conductive trace ends 960406 and **960408** with a dielectric material **960410** interposed the conductive trace ends **960406** and **960408** form a capacitor for the RLC circuit.

In one embodiment, the conductive trace material is selected to have a particular resistivity. Thus, the resistor element of the RLC circuit is controlled by the resistivity of the material used to form the circuit. In another embodiment, the resistivity is controlled by the thickness of a portion of the conductive trace deposited on the stent structure. In another embodiment, the resistivity is controlled by the width of at least a portion of the conductive trace deposited on the stent structure.

In one embodiment, the RLC circuit forms a resonance circuit. In one embodiment, the RLC circuit is a resonance circuit tuned to the resonance frequency of a magnetic resonance imaging scanner's $B_1$ magnetic field's rotational frequency. In another embodiment, the RLC circuit is a resonance circuit tuned to approximately the resonance frequency of a magnetic resonance imaging scanner's $B_1$ magnetic field's rotational frequency. In another embodiment, the RLC circuit is a resonance circuit not tuned to the frequency of a magnetic resonance imaging scanner's $B_1$ magnetic field's rotational frequency but having a broad Q-factor such that there is sufficient beneficial response by the RLC circuit.

In another embodiment, the RLC circuit has induced into it, by the $B_1$ field of a magnetic resonance imaging scanner, a current such that the induced magnetic field of the induced current in the RLC circuit significantly reduces the magnetic field generated by the induced current in the stent's conductive stents, thereby allowing a significant portion of the B1 field to penetrate into the lumen of a stent. In one embodiment, the induced current in the RLC circuit is 180 degrees shifted from the induced current in a portion of the stent's conductive struts. In another embodiment, the induced current in the RLC circuit is approximately 180 degrees shifted from the induced current in a portion the stent's conductive struts. In another embodiment, the induced current in the RLC circuit results in a minimum Faraday cage shielding effect of the stent structure thus allowing a maximum penetration of the a portion of the B1's magnetic field to reach the material within the stent's lumen.

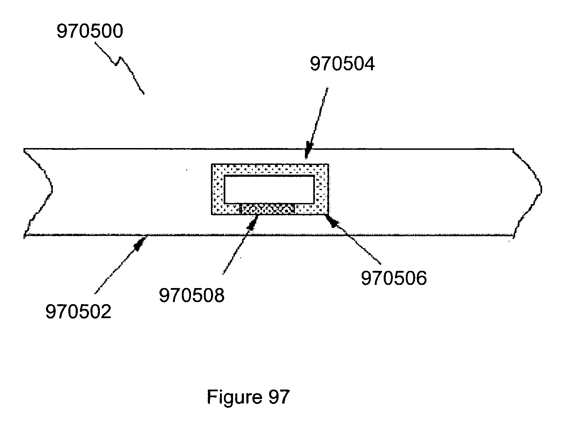

Figure 97 depicts a portion of a medical device **970500** including a medical device body **970502** and a RLC circuit **970504** composed onto a portion of the medical body **970502.** The RLC circuit **970504** comprises one or more conductive loops **970506** to form the inductor of the RLC circuit **970504** and overlapping ends (not shown but previously illustrated in Figure 96) of the conductive loops **970506** to form a capacitor **970508** of the RLC circuit **970504.**

It is noted that the medical device body **970502** may be a stent, a catheter, a stent introducer, an implanted access port, or other type of medical device.

It is noted that the conductive loops **970506** may form an essentially circular shaped inductive coil, an essentially rectangular shaped inductive coil, or other enclosed shaped inductive coil.

It is noted that the medical device **970500** may include multiple RLC circuits. In one embodiment, one or more RLC circuits **970504** on medical device 970500 forms a resonant circuit. In another embodiment, the resonant circuit is tuned to the resonance frequency of a magnetic resonance imaging scanner's $B_1$ magnetic field's rotational frequency. In another embodiment, the RLC circuit is a resonance circuit tuned to approximately the resonance frequency of a magnetic resonance imaging scanner's $B_1$ magnetic field's rotational frequency. In another embodiment, the RLC circuit is a resonance circuit not tuned to the frequency of a magnetic resonance imaging scanner's $B_1$ magnetic field's rotational frequency but having a broad Q-factor such that there is sufficient beneficial response by the RLC circuit.

In one embodiment, the resonance circuit provides an image marker in a magnetic resonance image. In another embodiment, the resonance circuit provides a tracking marker for real-time magnetic resonance imaging tracking of the medical device.

In one embodiment, the RLC circuits may be formed onto the stent or other medical device by means of depositing conductive, insulative, and dielectric inks by passing the inks through a fine tip pen or other dispensing nozzle. It is noted that the ink may contain silver or gold.

It is noted that the RLC circuit may be formed onto the stent or other medical device by vacuum deposition methods, a direct write method, lithographic means, or other method.

In another embodiment the RLC circuit of this invention is formed onto the stent or other medical device by a atomizing a slurry comprising a high quality of conductive particles, carrying the atomized particles to and through a nozzle by means of one or more gas flow techniques, directing the gas carrying conductive particles onto the stent or medical device, curing the deposited particles by means of a laser or other directed heating means.

In another embodiment, the RLC circuit is formed by depositing insulative, conductive, and dielectric material onto the medical device in combination with ablation techniques for removing unwanted material and shaping deposited materials to form the RLC circuit.

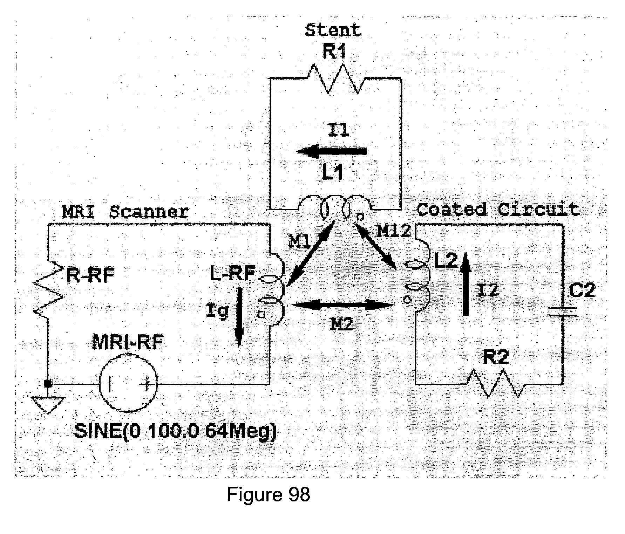

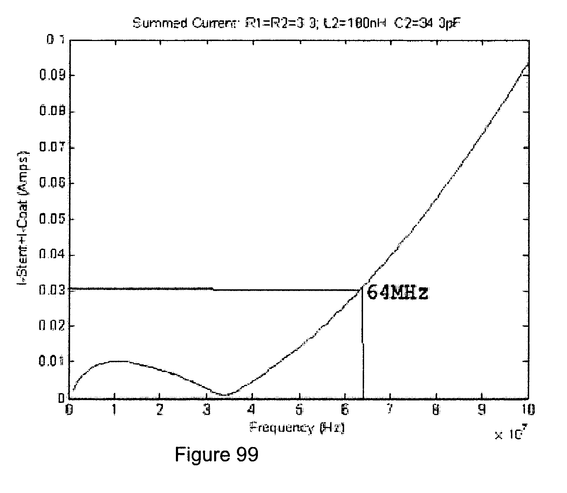

Figure 98 is a schematic of a circuit model of a RLC circuit formed on a stent having primarily an inductive and resistive characteristic, when placed into a magnetic resonance imaging scanner. The induced current **"I2"** through the circuit's inductor **"L2,"** the induced current **"I1"** through the stent's inductor **"L1,"** and the current **"Ig"** through the magnetic resonance scanner's coils **"L-RF"** are illustrated. An example of a plot of the summed currents **"I1 "+"I2"** versus the frequency of the current **"Ig"** for the circuit parameters indicated is presented in Figure 99.

Figure 99 is a plot of the summed currents **"I1"+"I2"** as the frequency of the current **"Ig"** varies. As can be seen, the summed current is a minimum at approximately 3.3 MHz, for the circuit parameters indicated. In particular, it is noted that the minimum summed current does not occur at the desired frequency of 63.8 MHz (approximately 64 MHz).

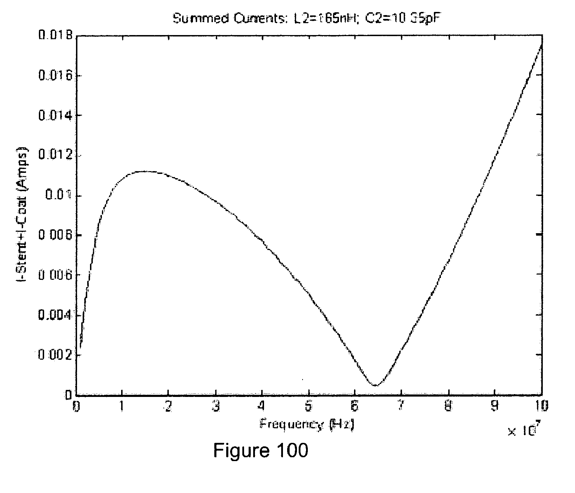

Figure 100 is also a plot of the summed currents **"I1"+"I2"** as the frequency of the current **"Ig"** varies. In this plot, the circuit's parameters has been altered without altering the stent's inductive or resistive values, such that the minimum summed current **"I1"+"I2"** is minimum at approximately 63.8 MHz.

It is noted that by minimizing the sum of the induced current in the stent plus the induced current in the RLC circuit formed on the stent that the Faraday cage shielding effect can be reduced. Such a reduction is desirable in magnetic resonance imaging to allow more of the excitation $B_1$ magnetic field to penetrate into the lumen of a stent thus providing brighter and sharper imaging of material in the stent lumen.

In summary, an implantable medical assist device includes a medical device including a housing and electronics contained therein; a lead to provide an electrical path to or from the electronics within the medical device; and a resonance tuning module located in the housing and operatively connected to the lead. The resonance tuning module includes a control circuit for determining a resonant frequency of the implantable medical assist device, and an adjustable impedance circuit.

The control circuit may operatively control: an impedance of the adjustable impedance circuit to change the resonant frequency of the implantable medical assist device; an impedance of the adjustable impedance circuit to change the resonant frequency of the implantable medical assist device to be substantially equal to a desired resonant frequency; an impedance of the adjustable impedance circuit to change the resonant frequency of the implantable medical assist device to be substantially equal to a radio-frequency of a magnetic resonance imaging scanner; an impedance of the adjustable impedance circuit to change the resonant frequency of the implantable medical assist device to be substantially equal to 64 MHz; an impedance of the adjustable impedance circuit to change the resonant frequency of the implantable medical assist device to be substantially equal to 128 MHz; an impedance of the adjustable impedance circuit to change the resonant frequency of the implantable medical assist device to be slightly detuned from a radio-frequency of a magnetic resonance imaging scanner; an impedance of the adjustable impedance circuit to change the resonant frequency of the implantable medical assist device to be slightly detuned from 64 MHz; or an impedance of the adjustable impedance circuit to change the resonant frequency of the implantable medical assist device to be slightly detuned from 128 MHz.

The adjustable impedance circuit includes a variable inductor and a variable capacitor; an adjustable inductive circuit and an adjustable capacitive circuit; a variable inductor, a variable capacitor, and a variable resistor; or an adjustable inductive circuit, an adjustable capacitive circuit, and an adjustable resistive circuit.

An implantable medical assist device includes a medical device including a housing and electronics contained therein; a first lead to provide an electrical path to or from the electronics within the medical device; a resonance tuning module; and a second lead to provide an electrical path to or from the resonance tuning module. The resonance tuning module includes a control circuit for determining a resonant frequency of the implantable medical assist device and an adjustable impedance circuit.

The control circuit may operatively control: an impedance of the adjustable impedance circuit to change the resonant frequency of the implantable medical assist device; an impedance of the adjustable impedance circuit to change the resonant frequency of the implantable medical assist device to be substantially equal to a desired resonant frequency; an impedance of the adjustable impedance circuit to change the resonant frequency of the implantable medical assist device to be substantially equal to a radio-frequency of a magnetic resonance imaging scanner; an impedance of the adjustable impedance circuit to change the resonant frequency of the implantable medical assist device to be substantially equal to 64 MHz; an impedance of the adjustable impedance circuit to change the resonant frequency of the implantable medical assist device to be substantially equal to 128 MHz; an impedance of the adjustable impedance circuit to change the resonant frequency of the implantable medical assist device to be slightly detuned from a radio-frequency of a magnetic resonance imaging scanner; an impedance of the adjustable impedance circuit to change the resonant frequency of the implantable medical assist device to be slightly detuned from 64 MHz; or an impedance of the adjustable impedance circuit to change the resonant frequency of the implantable medical assist device to be slightly detuned from 128 MHz.

The adjustable impedance circuit includes a variable inductor and a variable capacitor; an adjustable inductive circuit and an adjustable capacitive circuit; a variable inductor, a variable capacitor, and a variable resistor; or an adjustable inductive circuit, an adjustable capacitive circuit, and an adjustable resistive circuit.

A resonance tuning module includes a first interface to provide an electrical connection with a medical device having electronics therein; a second interface to provide an electrical connection with a lead; a control circuit for determining a combined resonant frequency of the medical device and lead; and an adjustable impedance circuit.

The control circuit may operatively control: an impedance of the adjustable impedance circuit to change the resonant frequency of the implantable medical assist device; an impedance of the adjustable impedance circuit to change the resonant frequency of the implantable medical assist device to be substantially equal to a desired resonant frequency; an impedance of the adjustable impedance circuit to change the resonant frequency of the implantable medical assist device to be substantially equal to a radio-frequency of a magnetic resonance imaging scanner; an impedance of the adjustable impedance circuit to change the resonant frequency of the implantable medical assist device to be substantially equal to 64 MHz; an impedance of the adjustable impedance circuit to change the resonant frequency of the implantable medical assist device to be substantially equal to 128 MHz; an impedance of the adjustable impedance circuit to change the resonant frequency of the implantable medical assist device to be slightly detuned from a radio-frequency of a magnetic resonance imaging scanner; an impedance of the adjustable impedance circuit to change the resonant frequency of the implantable medical assist device to be slightly detuned from 64 MHz; or an impedance of the adjustable impedance circuit to change the resonant frequency of the implantable medical assist device to be slightly detuned from 128 MHz.

The adjustable impedance circuit includes a variable inductor and a variable capacitor; an adjustable inductive circuit and an adjustable capacitive circuit; a variable inductor, a variable capacitor, and a variable resistor; or an adjustable inductive circuit, an adjustable capacitive circuit, and an adjustable resistive circuit.

An implantable medical assist device includes a first medical device. The first medical device includes a housing, electronics contained within the housing, and a first medical device interface to provide an electrical path to or from the electronics within the housing. The An implantable medical assist device further includes a second medical device including a second medical device interface to provide an electrical path to or from the second medical device and a removable resonance module, operatively connected between the first medical device interface and the second medical device. The removable resonance module provides an impedance such that a combined resonant frequency of the first medical device, the second medical device and the removable resonance module is substantially equal to a predetermined resonant frequency.

The predetermined resonant frequency may be: substantially equal to a radio-frequency of a magnetic resonance imaging scanner; substantially equal to 64 MHz; substantially equal to 128 MHz; slightly detuned from a radio-frequency of a magnetic resonance imaging scanner; slightly detuned from 64 MHz; or slightly detuned from 128 MHz.

The removable resonance module includes a variable inductor and a variable capacitor; an adjustable inductive circuit and an adjustable capacitive circuit; a variable inductor, a variable capacitor, and a variable resistor; or an adjustable inductive circuit, an adjustable capacitive circuit, and an adjustable resistive circuit. The second medical device may be a medical lead.

An implantable medical assist device includes a first medical device and a second medical device. The first medical device includes a housing, electronics contained within the housing, a first medical device interface to provide an electrical path to or from the electronics within the housing, a first resonance circuit operatively connected between the electronics and the first medical device interface, and a second resonance circuit operatively connected between the electronics and the first medical device interface. The second medical device includes a second medical device interface to provide an electrical path to or from the second medical device. The first resonance module provides an impedance such that a combined resonant frequency of the first medical device, the second medical device and the removable resonance module is substantially equal to a first predetermined resonant frequency. The second resonance module provides an impedance such that a combined resonant frequency of the first medical device, the second medical device and the removable resonance module is substantially equal to a second predetermined resonant frequency.

The first and second resonance modules include a variable inductor and a variable capacitor; an adjustable inductive circuit and an adjustable capacitive circuit; a variable inductor, a variable capacitor, and a variable resistor; or an adjustable inductive circuit, an adjustable capacitive circuit, and an adjustable resistive circuit. The first and second resonance modules may be connected in series or in parallel.

The implantable medical assist device further includes a switching circuit to operatively provide the first resonance circuit an independent connection between the electronics and the first medical device interface. The switching circuit operatively provides the first resonance circuit an independent disconnection between the electronics and the first medical device interface and operatively provides the second resonance circuit an independent connection between the electronics and the first medical device interface. The switching circuit operatively provides the second resonance circuit an independent disconnection between the electronics and the first medical device interface.

An electrode includes a conductive housing; an inner conductive layer; a dielectric material between the conductive housing and the inner conductive layer to form a capacitor; and an inductor coil connected to the inner conductive layer. The conductive housing may be substantially cylindrical, and the inner conductive layer may be substantially ring-shaped. The inductor coil and the capacitor may have a resonance frequency substantially equal to an operating frequency of a magnetic resonance imaging scanner; a resonance frequency substantially equal to a harmonic of an operating frequency of a magnetic resonance imaging scanner; or a resonance frequency slightly detuned from an operating frequency of a magnetic resonance imaging scanner.

The electrode may include a non-conductive spacer. The non-conductive spacer may include pass-through holes for enabling wires to pass therethrough. The non-conductive spacer may be substantially ring-shaped. The electrode may include a heat sink, a heating absorbing material, a heating wicking material, or a heating removing system. The non-conductive spacer may be located within a lumen of the inductor coil.

An electrode includes a conductive housing; a discrete component having a capacitor and an inductor; and a dielectric material between the conductive housing and the discrete component. The conductive housing may be substantially cylindrical, and the inner conductive layer may be substantially ring-shaped. The inductor coil and the capacitor may have a resonance frequency substantially equal to an operating frequency of a magnetic resonance imaging scanner; a resonance frequency substantially equal to a harmonic of an operating frequency of a magnetic resonance imaging scanner; or a resonance frequency slightly detuned from an operating frequency of a magnetic resonance imaging scanner.

The electrode may include a non-conductive spacer. The non-conductive spacer may include pass-through holes for enabling wires to pass therethrough. The non-conductive spacer may be substantially ring-shaped. The electrode may include a heat sink, a heating absorbing material, a heating wicking material, a heating removing system, or heat-removing channels. The non-conductive spacer may be located within a lumen of the inductor coil. The non-conductive spacer may include a heat sink, a heating absorbing material, a heating wicking material, a heating removing system, or heat-removing channels.

A component for inserting into a lead includes a non-conductive housing having a proximal connection interface to connect a first conductor thereto and a distal connection interface to connect a second conductor thereto; a first inner conductive layer; a second inner conductive layer; a dielectric material between the first inner conductive layer and the second inner conductive layer to form a capacitor; and an inductor coil connected to the second inner conductive layer.

The non-conductive housing may be substantially cylindrical, and the first and second inner conductive layers may be substantially ring-shaped.. The inductor coil and the capacitor may have a resonance frequency substantially equal to an operating frequency of a magnetic resonance imaging scanner; a resonance frequency substantially equal to a harmonic of an operating frequency of a magnetic resonance imaging scanner; or a resonance frequency slightly detuned from an operating frequency of a magnetic resonance imaging scanner.

The component may include a non-conductive spacer. The non-conductive spacer may include pass-through holes for enabling wires to pass therethrough. The non-conductive spacer may be substantially ring-shaped. The component may include a heat sink, a heating absorbing material, a heating wicking material, a heating removing system, or heat-removing channels. The non-conductive spacer may be located within a lumen of the inductor coil. The non-conductive spacer may include a heat sink, a heating absorbing material, a heating wicking material, a heating removing system, or heat-removing channels.

A component for inserting into a lead includes a non-conductive housing having a proximal connection interface to connect a first conductor thereto and a distal connection interface to connect a second conductor thereto and a discrete component having a capacitor and an inductor.

The non-conductive housing may be substantially cylindrical, and the first and second inner conductive layers may be substantially ring-shaped.. The inductor coil and the capacitor may have a resonance frequency substantially equal to an operating frequency of a magnetic resonance imaging scanner; a resonance frequency substantially equal to a harmonic of an operating frequency of a magnetic resonance imaging scanner; or a resonance frequency slightly detuned from an operating frequency of a magnetic resonance imaging scanner.

The component may include a non-conductive spacer. The non-conductive spacer may include pass-through holes for enabling wires to pass therethrough. The non-conductive spacer may be substantially ring-shaped. The component may include a heat sink, a heating absorbing material, a heating wicking material, a heating removing system, or heat-removing channels. The non-conductive spacer may be located within a lumen of the inductor coil. The non-conductive spacer may include a heat sink, a heating absorbing material, a heating wicking material, a heating removing system, or heat-removing channels.

A medical lead includes a polyfurcated conductor having a distal end and a proximal end; a first component located a first location along the polyfurcated conductor; and a second component located a second location along the polyfurcated conductor. The first component includes a non-conductive housing, a proximal connection interface to connect a first section conductor of the polyfurcated conductor thereto, a distal connection interface to connect a second section conductor of the polyfurcated conductor thereto, a first inner conductive layer, a second inner conductive layer, a dielectric material between the first inner conductive layer and the second inner conductive layer to form a capacitor, and an inductor coil connected to the second inner conductive layer. The second component includes a non-conductive housing, a proximal connection interface to connect a third section conductor of the polyfurcated conductor thereto, a distal connection interface to connect a fourth section conductor of the polyfurcated conductor thereto, a first inner conductive layer, a second inner conductive layer, a dielectric material between the first inner conductive layer and the second inner conductive layer to form a capacitor, and an inductor coil connected to the second inner conductive layer.

Each non-conductive housing may be substantially cylindrical. The inductor coil and the capacitor of the first component may have a resonance frequency equal to a first predetermined frequency and the inductor coil and the capacitor of the second component may have a resonance frequency equal to a second predetermined frequency, the first predetermined frequency being unequal to the second predetermined frequency. The inductor coil and the capacitor of the first component may have a resonance frequency substantially equal to an operating frequency of a magnetic resonance imaging scanner and the inductor coil and the capacitor of the second component may have a resonance frequency substantially equal to a harmonic of an operating frequency of a magnetic resonance imaging scanner.

The inductor coil and the capacitor of the first component may have a resonance frequency slightly detuned from an operating frequency of a magnetic resonance imaging scanner and the inductor coil and the capacitor of the second component may have a resonance frequency slightly detuned from a harmonic of an operating frequency of a magnetic resonance imaging scanner. The inductor coil and the capacitor of the first component may have a resonance frequency substantially equal to 64MHz and the inductor coil and the capacitor of the second component may have a resonance frequency substantially equal to 128 MHz. The inductor coil and the capacitor of the first component may have a resonance frequency slightly detuned from 64MHz and the inductor coil and the capacitor of the second component may have a resonance frequency slightly detuned from 128MHz.

The first component may be located at a distal end of the polyfurcated conductor. The first component may be located at a distal end of the polyfurcated conductor or located at a distal end of the polyfurcated conductor. Each of the first and second components may include a non-conductive spacer. The non-conductive spacers may include pass-through holes for enabling wires to pass therethrough. The non-conductive spacers may be substantially ring-shaped.

Each of the first and second components may include a heat sink, a heating absorbing material, a heating wicking material, a heating removing system, or heat-removing channels.

A medical lead includes a polyfurcated conductor having a distal end and a proximal end; a first component located a first location along the polyfurcated conductor; and a second component located a second location along the polyfurcated conductor. The first component includes a non-conductive housing, a proximal connection interface to connect a first section conductor of the polyfurcated conductor thereto, a distal connection interface to connect a second section conductor of the polyfurcated conductor thereto, and a discrete component having a capacitor and an inductor. The second component includes a non-conductive housing, a proximal connection interface to connect a third section conductor of the polyfurcated conductor thereto, a distal connection interface to connect a fourth section conductor of the polyfurcated conductor thereto, and a discrete component having a capacitor and an inductor.

Each non-conductive housing may be substantially cylindrical. The inductor coil and the capacitor of the first component may have a resonance frequency equal to a first predetermined frequency and the inductor coil and the capacitor of the second component may have a resonance frequency equal to a second predetermined frequency, the first predetermined frequency being unequal to the second predetermined frequency. The inductor coil and the capacitor of the first component may have a resonance frequency substantially equal to an operating frequency of a magnetic resonance imaging scanner and the inductor coil and the capacitor of the second component may have a resonance frequency substantially equal to a harmonic of an operating frequency of a magnetic resonance imaging scanner.

The inductor coil and the capacitor of the first component may have a resonance frequency slightly detuned from an operating frequency of a magnetic resonance imaging scanner and the inductor coil and the capacitor of the second component may have a resonance frequency slightly detuned from a harmonic of an operating frequency of a magnetic resonance imaging scanner. The inductor coil and the capacitor of the first component may have a resonance frequency substantially equal to 64MHz and the inductor coil and the capacitor of the second component may have a resonance frequency substantially equal to 128 MHz. The inductor coil and the capacitor of the first component may have a resonance frequency slightly detuned from 64MHz and the inductor coil and the capacitor of the second component may have a resonance frequency slightly detuned from 128MHz.

The first component may be located at a distal end of the polyfurcated conductor. The first component may be located at a distal end of the polyfurcated conductor or located at a distal end of the polyfurcated conductor. Each of the first and second components may include a non-conductive spacer. The non-conductive spacers may include pass-through holes for enabling wires to pass therethrough. The non-conductive spacers may be substantially ring-shaped.

Each of the first and second components may include a heat sink, a heating absorbing material, a heating wicking material, a heating removing system, or heat-removing channels.

A medical lead includes a polyfurcated conductor having a distal end and a proximal end; a first component located a first location along the polyfurcated conductor; and a second component located a second location along the polyfurcated conductor. The first component includes a conductive housing, a proximal connection interface to connect a first section conductor of the polyfurcated conductor thereto, a distal connection interface to connect a second section conductor of the polyfurcated conductor thereto, a inner conductive layer, a dielectric material between the inner conductive layer and the conductive housing to form a capacitor, and an inductor coil connected to the inner conductive layer. The second component includes a conductive housing, a proximal connection interface to connect a third section conductor of the polyfurcated conductor thereto, a distal connection interface to connect a fourth section conductor of the polyfurcated conductor thereto, a inner conductive layer, a dielectric material between the inner conductive layer and the conductive housing to form a capacitor, and an inductor coil connected to the inner conductive layer.

Each conductive housing may be substantially cylindrical. The inductor coil and the capacitor of the first component may have a resonance frequency equal to a first predetermined frequency and the inductor coil and the capacitor of the second component may have a resonance frequency equal to a second predetermined frequency, the first predetermined frequency being unequal to the second predetermined frequency. The inductor coil and the capacitor of the first component may have a resonance frequency substantially equal to an operating frequency of a magnetic resonance imaging scanner and the inductor coil and the capacitor of the second component may have a resonance frequency substantially equal to a harmonic of an operating frequency of a magnetic resonance imaging scanner.

The inductor coil and the capacitor of the first component may have a resonance frequency slightly detuned from an operating frequency of a magnetic resonance imaging scanner and the inductor coil and the capacitor of the second component may have a resonance frequency slightly detuned from a harmonic of an operating frequency of a magnetic resonance imaging scanner. The inductor coil and the capacitor of the first component may have a resonance frequency substantially equal to 64MHz and the inductor coil and the capacitor of the second component may have a resonance frequency substantially equal to 128 MHz. The inductor coil and the capacitor of the first component may have a resonance frequency slightly detuned from 64MHz and the inductor coil and the capacitor of the second component may have a resonance frequency slightly detuned from 128MHz.

The first component may be located at a distal end of the polyfurcated conductor. The first component may be located at a distal end of the polyfurcated conductor or located at a distal end of the polyfurcated conductor. Each of the first and second components may include a non-conductive spacer. The non-conductive spacers may include pass-through holes for enabling wires to pass therethrough. The non-conductive spacers may be substantially ring-shaped.

Each of the first and second components may include a heat sink, a heating absorbing material, a heating wicking material, a heating removing system, or heat-removing channels.

Each of the inductor coil and the capacitor circuits discussed above may include a diode connected across the inductor coil, a diode connected across the capacitor, or back-to-back diodes connected across the inductor coil.

A medical lead having a distal end and a proximal end includes a first section and a second section. The first section has a first coiling pitch and insulating dielectric material spacers, the first coiling pitch and insulating dielectric material spacers having a self-resonance at a first predetermined frequency. The second section has a second coiling pitch and insulating dielectric material spacers, the second coiling pitch and insulating dielectric material spacers having a self-resonance at a second predetermined frequency, the first predetermined frequency being unequal to the second predetermined frequency.

The first section may have a resonance frequency substantially equal to an operating frequency of a magnetic resonance imaging scanner and the second section may have a resonance frequency substantially equal to a harmonic of an operating frequency of a magnetic resonance imaging scanner. The first section may have a resonance frequency slightly detuned from an operating frequency of a magnetic resonance imaging scanner and the second section may have a resonance frequency slightly detuned from a harmonic of an operating frequency of a magnetic resonance imaging scanner. The first section may have a resonance frequency substantially equal to 64MHz and the second section may have a resonance frequency substantially equal to 128 MHz. The first section may have a resonance frequency slightly detuned from 64MHz and the second section may have a resonance frequency slightly detuned from 128MHz. The first section may be located at a distal end of the medical lead.

A medical apparatus includes a medical assist device to process signals to relating biological functions; a first lead operatively connected to the medical assist device, the first lead having a distal end and a proximal end; a second lead operatively connected to the medical assist device, the second lead having a distal end and a proximal end; a first electrode operatively connected to the distal end of the first lead; a second electrode operatively connected to the distal end of the second lead; a filter circuit operatively connected near the distal end of the first lead and the distal end of the second lead; and a compensation circuit, operatively connected to the first lead, to provide a compensation voltage to enable the filter to effectively block changing magnetic fields induced current in the second lead from passing through the second electrode of the distal end of the second lead.

The filter may be a capacitor. The compensation circuit may include a sensing circuit to sense voltages induced in the first lead, the voltages being induced by changing magnetic fields and a voltage compensation circuit, operatively connected to the sensing circuit and responsive thereto, to provide the compensation voltage. The compensation circuit may include a power supply or a battery.

The compensation circuit may include a sensing circuit to detect the changing magnetic fields such that the compensation circuit synchronizes application of the compensation voltage with the sensed changing magnetic fields. The compensation circuit may be located the proximal end of the first lead or within the medical assist device. The compensation circuit may include a coil that generates a voltage due to a changing magnetic resonance imaging electromagnetic field, wherein the coil may be curved in three different spatial directions. The compensation circuit may include orientation means for changing a spatial orientation of the coil to modify the strength of the voltage induced by the changing magnetic resonance imaging electromagnetic field.

The compensation circuit may include a plurality of coils, each coil generating a voltage due to a changing magnetic resonance imaging electromagnetic field such that a combination of voltages due to a changing magnetic resonance imaging electromagnetic field are produced, wherein the coils may be curved in three different spatial directions. The compensation circuit may include orientation means for changing a spatial orientation of the coils to modify the strength of the voltage due to the changing magnetic resonance imaging electromagnetic field.

The compensation circuit may include three orthogonally planar coils, each coil generating a voltage due to a changing magnetic resonance imaging electromagnetic field such that a combination of voltages due to a changing magnetic resonance imaging electromagnetic field are produced. The compensation circuit may include a plurality of coils, each coil generating a voltage due to a changing magnetic resonance imaging electromagnetic field; a sensor to measure a strength of voltages induced by the changing magnetic resonance imaging electromagnetic field; and a switching device, operatively connected to the sensor and plurality of coils, to operatively connect a number of the plurality of coils in response to the measured strength of voltages induced by the changing magnetic resonance imaging electromagnetic field such that a combination of voltages due to a changing magnetic resonance imaging electromagnetic field are produced. The coils may be curved in three different spatial directions. The compensation circuit may include orientation means for changing a spatial orientation of the coils to modify the strength of the voltage induced by the changing magnetic resonance imaging electromagnetic field.

The compensation circuit may include three orthogonally planar coils, each coil generating a voltage due to a changing magnetic resonance imaging electromagnetic field; a sensor to measure a strength of voltages induced by the changing magnetic resonance imaging electromagnetic field; and a switching device, operatively connected to the sensor and the coils, to operatively connect a number of the coils in response to the measured strength of voltages induced by the changing magnetic resonance imaging electromagnetic field such that a combination of voltages due to a changing magnetic resonance imaging electromagnetic field are produced.

The compensation circuit may include a plurality of coils, each coil generating a voltage due to a changing magnetic resonance imaging electromagnetic field; a transceiver to receive a signal indicating a number of coils to be connected; and a switching device, operatively connected to the transceiver and plurality of coils, to operatively connect a number of the plurality of coils in response to the received signal indicating the number of coils to be connected such that a combination of voltages due to a changing magnetic resonance imaging electromagnetic field.

The compensation circuit may include three orthogonally planar coils, each coil generating a voltage due to a changing magnetic resonance imaging electromagnetic field; a transceiver to receive a signal indicating a number of coils to be connected; and a switching device, operatively connected to the transceiver and the coils, to operatively connect a number of the coils in response to the received signal indicating the number of coils to be connected such that a combination of voltages due to a changing magnetic resonance imaging electromagnetic field.

A medical apparatus includes a medical assist device to process signals to relating biological functions; a first lead operatively connected to the medical assist device, the first lead having a distal end and a proximal end; a second lead operatively connected to the medical assist device, the second lead having a distal end and a proximal end; a first electrode operatively connected to the distal end of the first lead; a second electrode operatively connected to the distal end of the second lead; a first filter circuit operatively connected near the distal end of the first lead; a second filter circuit operatively connected near the distal end of the second lead; and a compensation circuit, operatively connected to the first filter and the second filter, to provide a compensation voltage to enable the first and second filters to effectively block changing magnetic fields induced current in the first and second leads from passing through the first and second electrodes of the distal ends of the first and second leads.

The first and second filters may be capacitors. The compensation circuit may include a sensing circuit to sense voltages induced in the first lead, the voltages being induced by changing magnetic fields and a voltage compensation circuit, operatively connected to the sensing circuit and responsive thereto, to provide the compensation voltage. The compensation circuit may include a power supply or a battery.

The compensation circuit may include a sensing circuit to detect the changing magnetic fields such that the compensation circuit synchronizes application of the compensation voltage with the sensed changing magnetic fields. The compensation circuit may be located the proximal end of the first lead or within the medical assist device. The compensation circuit may include a coil that generates a voltage due to a changing magnetic resonance imaging electromagnetic field, wherein the coil may be curved in three different spatial directions. The compensation circuit may include orientation means for changing a spatial orientation of the coil to modify the strength of the voltage induced by the changing magnetic resonance imaging electromagnetic field.

The compensation circuit may include a plurality of coils, each coil generating a voltage due to a changing magnetic resonance imaging electromagnetic field such that a combination of voltages due to a changing magnetic resonance imaging electromagnetic field are produced, wherein the coils may be curved in three different spatial directions. The compensation circuit may include orientation means for changing a spatial orientation of the coils to modify the strength of the voltage due to the changing magnetic resonance imaging electromagnetic field.

The compensation circuit may include three orthogonally planar coils, each coil generating a voltage due to a changing magnetic resonance imaging electromagnetic field such that a combination of voltages due to a changing magnetic resonance imaging electromagnetic field are produced. The compensation circuit may include a plurality of coils, each coil generating a voltage due to a changing magnetic resonance imaging electromagnetic field; a sensor to measure a strength of voltages induced by the changing magnetic resonance imaging electromagnetic field; and a switching device, operatively connected to the sensor and plurality of coils, to operatively connect a number of the plurality of coils in response to the measured strength of voltages induced by the changing magnetic resonance imaging electromagnetic field such that a combination of voltages due to a changing magnetic resonance imaging electromagnetic field are produced. The coils may be curved in three different spatial directions. The compensation circuit may include orientation means for changing a spatial orientation of the coils to modify the strength of the voltage induced by the changing magnetic resonance imaging electromagnetic field.

The compensation circuit may include three orthogonally planar coils, each coil generating a voltage due to a changing magnetic resonance imaging electromagnetic field; a sensor to measure a strength of voltages induced by the changing magnetic resonance imaging electromagnetic field; and a switching device, operatively connected to the sensor and the coils, to operatively connect a number of the coils in response to the measured strength of voltages induced by the changing magnetic resonance imaging electromagnetic field such that a combination of voltages due to a changing magnetic resonance imaging electromagnetic field are produced.

The compensation circuit may include a plurality of coils, each coil generating a voltage due to a changing magnetic resonance imaging electromagnetic field; a transceiver to receive a signal indicating a number of coils to be connected; and a switching device, operatively connected to the transceiver and plurality of coils, to operatively connect a number of the plurality of coils in response to the received signal indicating the number of coils to be connected such that a combination of voltages due to a changing magnetic resonance imaging electromagnetic field.

The compensation circuit may include three orthogonally planar coils, each coil generating a voltage due to a changing magnetic resonance imaging electromagnetic field; a transceiver to receive a signal indicating a number of coils to be connected; and a switching device, operatively connected to the transceiver and the coils, to operatively connect a number of the coils in response to the received signal indicating the number of coils to be connected such that a combination of voltages due to a changing magnetic resonance imaging electromagnetic field.

A medical apparatus includes a medical assist device to process signals to relating biological functions; a first lead operatively connected to the medical assist device, the first lead having a distal end and a proximal end; a second lead operatively connected to the medical assist device, the second lead having a distal end and a proximal end; a first electrode operatively connected to the distal end of the first lead; a second electrode operatively connected to the distal end of the second lead; a first filter circuit operatively connected near the distal end of the first lead; a second filter circuit operatively connected near the distal end of the second lead; a third filter circuit operatively connected near the proximal end of the first lead; a fourth filter circuit operatively connected near the proximal end of the second lead; and a compensation circuit, operatively connected to the first filter and the second filter, to provide a compensation voltage to enable the first and second filters to effectively block changing magnetic fields induced current in the first and second leads from passing through the first and second electrodes of the distal ends of the first and second leads.

The compensation circuit may be operatively connected to the third filter and the fourth filter, to provide a compensation voltage to enable the third and fourth filters to effectively block changing magnetic fields induced current in the first and second leads from passing into the medical assist device. The first, second, third, and fourth filters may be capacitors.

The compensation circuits may include a sensing circuit to sense voltages induced in the first lead, the voltages being induced by changing magnetic fields and a voltage compensation circuit, operatively connected to the sensing circuit and responsive thereto, to provide the compensation voltage. The compensation circuits may include a power supply or a battery.

The compensation circuits may include a sensing circuit to detect the changing magnetic fields such that the compensation circuit synchronizes application of the compensation voltage with the sensed changing magnetic fields. The compensation circuits may be located the proximal end of the first lead or within the medical assist device. The compensation circuits may include a coil that generates a voltage due to a changing magnetic resonance imaging electromagnetic field, wherein the coil may be curved in three different spatial directions. The compensation circuits may include orientation means for changing a spatial orientation of the coil to modify the strength of the voltage induced by the changing magnetic resonance imaging electromagnetic field.

The compensation circuits may include a plurality of coils, each coil generating a voltage due to a changing magnetic resonance imaging electromagnetic field such that a combination of voltages due to a changing magnetic resonance imaging electromagnetic field are produced, wherein the coils may be curved in three different spatial directions. The compensation circuits may include orientation means for changing a spatial orientation of the coils to modify the strength of the voltage due to the changing magnetic resonance imaging electromagnetic field.

The compensation circuits may include three orthogonally planar coils, each coil generating a voltage due to a changing magnetic resonance imaging electromagnetic field such that a combination of voltages due to a changing magnetic resonance imaging electromagnetic field are produced. The compensation circuits may include a plurality of coils, each coil generating a voltage due to a changing magnetic resonance imaging electromagnetic field; a sensor to measure a strength of voltages induced by the changing magnetic resonance imaging electromagnetic field; and a switching device, operatively connected to the sensor and plurality of coils, to operatively connect a number of the plurality of coils in response to the measured strength of voltages induced by the changing magnetic resonance imaging electromagnetic field such that a combination of voltages due to a changing magnetic resonance imaging electromagnetic field are produced. The coils may be curved in three different spatial directions. The compensation circuit may include orientation means for changing a spatial orientation of the coils to modify the strength of the voltage induced by the changing magnetic resonance imaging electromagnetic field.

The compensation circuits may include three orthogonally planar coils, each coil generating a voltage due to a changing magnetic resonance imaging electromagnetic field; a sensor to measure a strength of voltages induced by the changing magnetic resonance imaging electromagnetic field; and a switching device, operatively connected to the sensor and the coils, to operatively connect a number of the coils in response to the measured strength of voltages induced by the changing magnetic resonance imaging electromagnetic field such that a combination of voltages due to a changing magnetic resonance imaging electromagnetic field are produced.

The compensation circuits may include a plurality of coils, each coil generating a voltage due to a changing magnetic resonance imaging electromagnetic field; a transceiver to receive a signal indicating a number of coils to be connected; and a switching device, operatively connected to the transceiver and plurality of coils, to operatively connect a number of the plurality of coils in response to the received signal indicating the number of coils to be connected such that a combination of voltages due to a changing magnetic resonance imaging electromagnetic field.

The compensation circuits may include three orthogonally planar coils, each coil generating a voltage due to a changing magnetic resonance imaging electromagnetic field; a transceiver to receive a signal indicating a number of coils to be connected; and a switching device, operatively connected to the transceiver and the coils, to operatively connect a number of the coils in response to the received signal indicating the number of coils to be connected such that a combination of voltages due to a changing magnetic resonance imaging electromagnetic field.

A medical apparatus includes a medical assist device to process signals to relating biological functions; a first lead operatively connected to the medical assist device, the first lead having a distal end and a proximal end; a second lead operatively connected to the medical assist device, the second lead having a distal end and a proximal end; a first electrode operatively connected to the distal end of the first lead; a second electrode operatively connected to the distal end of the second lead; a first filter circuit operatively connected near the proximal end of the first lead; a second filter circuit operatively connected near the proximal end of the second lead; and a compensation circuit, operatively connected to the first filter and the second filter, to provide a compensation voltage to enable the first and second filters to effectively block changing magnetic fields induced current in the first and second leads from passing into the medical assist device.

The first and second filters may be capacitors. The compensation circuit may include a sensing circuit to sense voltages induced in the first lead, the voltages being induced by changing magnetic fields and a voltage compensation circuit, operatively connected to the sensing circuit and responsive thereto, to provide the compensation voltage. The compensation circuit may include a power supply or a battery.

The compensation circuit may include a sensing circuit to detect the changing magnetic fields such that the compensation circuit synchronizes application of the compensation voltage with the sensed changing magnetic fields. The compensation circuit may be located the proximal end of the first lead or within the medical assist device. The compensation circuit may include a coil that generates a voltage due to a changing magnetic resonance imaging electromagnetic field, wherein the coil may be curved in three different spatial directions. The compensation circuit may include orientation means for changing a spatial orientation of the coil to modify the strength of the voltage induced by the changing magnetic resonance imaging electromagnetic field.

The compensation circuit may include a plurality of coils, each coil generating a voltage due to a changing magnetic resonance imaging electromagnetic field such that a combination of voltages due to a changing magnetic resonance imaging electromagnetic field are produced, wherein the coils may be curved in three different spatial directions. The compensation circuit may include orientation means for changing a spatial orientation of the coils to modify the strength of the voltage due to the changing magnetic resonance imaging electromagnetic field.

The compensation circuit may include three orthogonally planar coils, each coil generating a voltage due to a changing magnetic resonance imaging electromagnetic field such that a combination of voltages due to a changing magnetic resonance imaging electromagnetic field are produced. The compensation circuit may include a plurality of coils, each coil generating a voltage due to a changing magnetic resonance imaging electromagnetic field; a sensor to measure a strength of voltages induced by the changing magnetic resonance imaging electromagnetic field; and a switching device, operatively connected to the sensor and plurality of coils, to operatively connect a number of the plurality of coils in response to the measured strength of voltages induced by the changing magnetic resonance imaging electromagnetic field such that a combination of voltages due to a changing magnetic resonance imaging electromagnetic field are produced. The coils may be curved in three different spatial directions. The compensation circuit may include orientation means for changing a spatial orientation of the coils to modify the strength of the voltage induced by the changing magnetic resonance imaging electromagnetic field.

The compensation circuit may include three orthogonally planar coils, each coil generating a voltage due to a changing magnetic resonance imaging electromagnetic field; a sensor to measure a strength of voltages induced by the changing magnetic resonance imaging electromagnetic field; and a switching device, operatively connected to the sensor and the coils, to operatively connect a number of the coils in response to the measured strength of voltages induced by the changing magnetic resonance imaging electromagnetic field such that a combination of voltages due to a changing magnetic resonance imaging electromagnetic field are produced.

The compensation circuit may include a plurality of coils, each coil generating a voltage due to a changing magnetic resonance imaging electromagnetic field; a transceiver to receive a signal indicating a number of coils to be connected; and a switching device, operatively connected to the transceiver and plurality of coils, to operatively connect a number of the plurality of coils in response to the received signal indicating the number of coils to be connected such that a combination of voltages due to a changing magnetic resonance imaging electromagnetic field.

The compensation circuit may include three orthogonally planar coils, each coil generating a voltage due to a changing magnetic resonance imaging electromagnetic field; a transceiver to receive a signal indicating a number of coils to be connected; and a switching device, operatively connected to the transceiver and the coils, to operatively connect a number of the coils in response to the received signal indicating the number of coils to be connected such that a combination of voltages due to a changing magnetic resonance imaging electromagnetic field.

A medical lead includes a conductor having a first section of coils and a second section of coils; the first section of coils having coils coiled along an axis of a length of the medical lead; the second section of coils having coils coiled perpendicular to the coils of the first section.

The conductor may have a third section of coils, the third section of coils having coils coiled perpendicular to the coils of the first section and perpendicular to the coils of the second section.

The first section of coils may have a predetermined number of coils per unit length, the second section of coils may have a predetermined number of coils per unit length, and the predetermined number of coils per unit length of the first section of coils may be different than the predetermined number of coils per unit length of the second section of coils.

The third section of coils may have a predetermined number of coils per unit length, the predetermined number of coils per unit length of the first section of coils may be different than the predetermined number of coils per unit length of the third section of coils, and the predetermined number of coils per unit length of the third section of coils may be different than the predetermined number of coils per unit length of the second section of coils.

A medical lead includes a conductor having a plurality of first sections of coils and a plurality of second sections of coils, each first section of coils having coils coiled along an axis of a length of the medical lead, each second section of coils having coils coiled perpendicular to the coils of the first section.

The conductor may have a plurality of third sections of coils, each third section of coils having coils coiled perpendicular to the coils of the first section and perpendicular to the coils of the second section.

Each first section of coils may have a predetermined number of coils per unit length, each second section of coils may have a predetermined number of coils per unit length, and the predetermined number of coils per unit length of the first sections of coils may be different than the predetermined number of coils per unit length of the second sections of coils.

Each third section of coils may have a predetermined number of coils per unit length, the predetermined number of coils per unit length of the first sections of coils may be different than the predetermined number of coils per unit length of the third sections of coils, and the predetermined number of coils per unit length of the third sections of coils may be different than the predetermined number of coils per unit length of the second sections of coils.

A stent structure includes a stent. The stent includes a plurality of rings to form a lumen, and a plurality of struts, a strut providing a connection between two adjacent rings. The stent structure includes a conductive trace and a dielectric material. The conductive trace has a first end and a second end. The conductive trace is formed on a portion of a first ring, on a portion of a second ring, and on struts between the first ring and the second ring to form an inductive conductive trace loop. The first end of the conductive trace overlaps the second end of the conductive trace such that the dielectric material is located between the first end of the conductive trace and the second end of the conductive trace to form a capacitor.

The first ring may be an end ring of the stent and the second ring may be another end ring of the stent. The conductive trace formed on the portion of the first ring may be located in a same hemisphere of the stent as the conductive trace formed on the portion of the second ring. The conductive trace formed on the portion of the first ring may be located in a same hemisphere of the stent as the conductive trace formed on the portion of the second ring. The conductive trace formed on the portion of the first ring may be located in a different hemisphere of the stent from the conductive trace formed on the portion of the second ring. The conductive trace formed on the portion of the first ring may be located in a different hemisphere of the stent from the conductive trace formed on the portion of the second ring. The rings may be zigzag shaped.

The stent structure may include a second conductive trace. The second conductive trace has a first end and a second end. The second conductive trace is formed on a portion of a third ring, on a portion of a fourth ring, and on struts between the third ring and the fourth ring to form a second inductive conductive trace loop. The first end of the conductive trace overlaps the second end of the conductive trace such that the dielectric material is located between the first end of the second conductive trace and the second end of the second conductive trace to form a capacitor.

The third ring may be an end ring of the stent and the fourth ring may be another end ring of the stent. The conductive trace formed on the portion of the first ring may be located in a same hemisphere of the stent as the conductive trace formed on the portion of the second ring and the second conductive trace formed on the portion of the third ring may be located in a same hemisphere of the stent as the second conductive trace formed on the portion of the fourth ring. The conductive trace formed on the portion of the first ring may be located in a same hemisphere of the stent as the conductive trace formed on the portion of the second ring and the second conductive trace formed on the portion of the third ring may be located in a same hemisphere of the stent as the second conductive trace formed on the portion of the fourth ring.

The conductive trace formed on the portion of the first ring may be located in a different hemisphere of the stent from the conductive trace formed on the portion of the second ring and the second conductive trace formed on the portion of the third ring may be located in a different hemisphere of the stent from the second conductive trace formed on the portion of the fourth ring. The conductive trace formed on the portion of the first ring may be located in a different hemisphere of the stent from the conductive trace formed on the portion of the second ring and the second conductive trace formed on the portion of the third ring may be located in a different hemisphere of the stent from the second conductive trace formed on the portion of the fourth ring. The rings are zigzag shaped.

The first ring and the third ring may be the same and the second ring and the fourth ring may be the same. The second inductive conductive trace loop may be located in a same hemisphere of the stent as the inductive conductive trace loop. The second inductive conductive trace loop may be located in a different hemisphere of the stent from the inductive conductive trace loop. A portion of the second inductive conductive trace loop may overlap a portion of the inductive conductive trace loop. The second inductive conductive trace loop may be orthogonal to the inductive conductive trace loop.

The conductive trace formed on the portion of the first ring may be located in a different hemisphere of the stent from the conductive trace formed on the portion of the second ring. The second conductive trace formed on the portion of the third ring may be located in a different hemisphere of the stent from the second conductive trace formed on the portion of the fourth ring. The second conductive trace formed on the portion of the third ring may be located in a same hemisphere of the stent as the conductive trace formed on the portion of the second ring. The second conductive trace formed on the portion of the fourth ring may be located in a same hemisphere of the stent as the conductive trace formed on the portion of the first ring.

The conductive trace formed on the portion of the first ring may be located in a different hemisphere of the stent from the conductive trace formed on the portion of the second ring. The second conductive trace formed on the portion of the third ring may be located in a different hemisphere of the stent from the second conductive trace formed on the portion of the fourth ring. The second conductive trace formed on the portion of the third ring may be located in a same hemisphere of the stent as the conductive trace formed on the portion of the first ring. The second conductive trace formed on the portion of the fourth ring may be located in a same hemisphere of the stent as the conductive trace formed on the portion of the second ring.

The second inductive conductive trace loop may be located within the inductive conductive trace loop. The second inductive conductive trace loop may be capacitively coupled to the inductive conductive trace loop. The conductive trace may be a resistive element. The resistance of the conductive trace may be controlled by a material of the conductive trace. The resistance of the conductive trace may be controlled by a thickness of a portion of the conductive trace deposited on the stent. The resistance of the conductive trace may be controlled by a width of at least a portion of the conductive trace deposited on the stent.

The inductor and capacitor may have a resonant frequency substantially equal to a radio-frequency of a magnetic resonance imaging scanner; a resonant frequency substantially equal to 64 MHz; a resonant frequency substantially equal to 128 MHz; a resonant frequency slightly detuned from a radio-frequency of a magnetic resonance imaging scanner; a resonant frequency slightly detuned from 64 MHz; or a resonant frequency slightly detuned from 128 MHz.

The inductor and capacitor of the conductive trace may have a resonant frequency substantially equal to a radio-frequency of a magnetic resonance imaging scanner and the inductor and capacitor of the second conductive trace may have a resonant frequency substantially equal to a harmonic of the radio-frequency of the magnetic resonance imaging scanner.

The inductor and capacitor of the conductive trace may have a resonant frequency substantially equal to 64 MHz and capacitor of the second conductive trace may have a resonant frequency substantially equal to a harmonic of 64 MHz.

The inductor and capacitor of the conductive trace may have a resonant frequency substantially equal to 128 MHz and capacitor of the second conductive trace may have a resonant frequency substantially equal to a harmonic of 128 MHz.

The inductor and capacitor of the conductive trace may have a resonant frequency slightly detuned from a radio-frequency of a magnetic resonance imaging scanner and the inductor and capacitor of the second conductive trace may have a resonant frequency slightly detuned from a harmonic of the radio-frequency of the magnetic resonance imaging scanner.

The inductor and capacitor of the conductive trace may have a resonant frequency slightly detuned from 64 MHz and capacitor of the second conductive trace may have a resonant frequency slightly detuned from a harmonic of 64 MHz.

The inductor and capacitor of the conductive trace may have a resonant frequency slightly detuned from 128 MHz and capacitor of the second conductive trace may have a resonant frequency slightly detuned from a harmonic of 128 MHz.

The inductor and capacitor of the conductive trace may form an image marker in a magnetic resonance image or a tracking marker for real-time magnetic resonance imaging tracking of the stent structure.

A medical device includes a conductive trace formed on the medical device and a dielectric material. The conductive trace has a first end and a second end. The conductive trace is formed on a portion of the medical device to form an inductive conductive trace loop. The first end of the conductive trace overlaps the second end of the conductive trace such that the dielectric material is located between the first end of the conductive trace and the second end of the conductive trace to form a capacitor.

The medical device may include a second conductive trace. The second conductive trace has a first end and a second end. The second conductive trace is formed on a portion of a third ring, on a portion of a fourth ring, and on struts between the third ring and the fourth ring to form a second inductive conductive trace loop. The first end of the conductive trace overlaps the second end of the conductive trace such that the dielectric material is located between the first end of the second conductive trace and the second end of the second conductive trace to form a capacitor.

The second inductive conductive trace loop may be orthogonal to the inductive conductive trace loop. The second inductive conductive trace loop may be located within the inductive conductive trace loop. The second inductive conductive trace loop may be capacitively coupled to the inductive conductive trace loop.

. The conductive trace may be a resistive element. The resistance of the conductive trace may be controlled by a material of the conductive trace. The resistance of the conductive trace may be controlled by a thickness of a portion of the conductive trace deposited on the medical device. The resistance of the conductive trace may be controlled by a width of at least a portion of the conductive trace deposited on the medical device.

The inductor and capacitor may have a resonant frequency substantially equal to a radio-frequency of a magnetic resonance imaging scanner; a resonant frequency substantially equal to 64 MHz; a resonant frequency substantially equal to 128 MHz; a resonant frequency slightly detuned from a radio-frequency of a magnetic resonance imaging scanner; a resonant frequency slightly detuned from 64 MHz; or a resonant frequency slightly detuned from 128 MHz.

The inductor and capacitor of the conductive trace may have a resonant frequency substantially equal to a radio-frequency of a magnetic resonance imaging scanner and the inductor and capacitor of the second conductive trace may have a resonant frequency substantially equal to a harmonic of the radio-frequency of the magnetic resonance imaging scanner.

The inductor and capacitor of the conductive trace may have a resonant frequency substantially equal to 64 MHz and capacitor of the second conductive trace may have a resonant frequency substantially equal to a harmonic of 64 MHz.

The inductor and capacitor of the conductive trace may have a resonant frequency substantially equal to 128 MHz and capacitor of the second conductive trace may have a resonant frequency substantially equal to a harmonic of 128 MHz.

The inductor and capacitor of the conductive trace may have a resonant frequency slightly detuned from a radio-frequency of a magnetic resonance imaging scanner and the inductor and capacitor of the second conductive trace may have a resonant frequency slightly detuned from a harmonic of the radio-frequency of the magnetic resonance imaging scanner.

The inductor and capacitor of the conductive trace may have a resonant frequency slightly detuned from 64 MHz and capacitor of the second conductive trace may have a resonant frequency slightly detuned from a harmonic of 64 MHz.

The inductor and capacitor of the conductive trace may have a resonant frequency slightly detuned from 128 MHz and capacitor of the second conductive trace may have a resonant frequency slightly detuned from a harmonic of 128 MHz.

The inductor and capacitor of the conductive trace may form an image marker in a magnetic resonance image or a tracking marker for real-time magnetic resonance imaging tracking of the medical device.

While various examples and embodiments of the present invention have been shown and described, it will be appreciated by those skilled in the art that the spirit and scope of the present invention are not limited to the specific description and drawings herein, but extend to various modifications and changes thereof.

**Claims**

1. A medical apparatus, comprising:

   a medical assist device to process signals to relating biological functions;
   a first lead operatively connected to said medical assist device, said first lead having a distal end and a proximal end;
   a second lead operatively connected to said medical assist device, said second lead having a distal end and a proximal end;
   a first electrode operatively connected to said distal end of said first lead;
   a second electrode operatively connected to said distal end of said second lead;
   a filter circuit operatively connected near said distal end of said first lead and said distal end of said second lead; and
   a compensation circuit, operatively connected to said first lead, to provide a compensation voltage to enable said filter to effectively block changing magnetic fields induced current in said second lead from passing through said second electrode of said distal end of said second lead.

2. The medical apparatus as claimed in claim 1, wherein said filter is a capacitor.

3. The medical apparatus as claimed in claim 1, wherein said compensation circuit comprises:

   a sensing circuit to sense voltages induced in said first lead, the voltages being induced by changing magnetic fields; and
   a voltage compensation circuit, operatively connected to said sensing circuit and responsive thereto, to provide said compensation voltage.

4. The medical apparatus as claimed in claim 3 wherein said compensation circuit comprises a power supply.

5. The medical apparatus as claimed in claim 3, wherein said compensation circuit comprises a battery.

6. The medical apparatus as claimed in claim 1, wherein said compensation circuit comprises:

   a sensing circuit to detect the changing magnetic fields;
   said compensation circuit, operatively connected to said sensing circuit and responsive thereto, to synchronize application of said compensation voltage with the sensed changing magnetic fields.

7. The medical apparatus as claimed in claim 3, wherein said compensation circuit comprises:

   a second sensing circuit to detect the changing magnetic fields;
   said compensation circuit, operatively connected to said second sensing circuit and responsive thereto, to synchronize application of said compensation voltage with the sensed changing magnetic fields.

8.  The medical apparatus as claimed in claim 1, wherein said compensation circuit is located said proximal end of said first lead, or wherein said compensation circuit is located within said medical assist device.

9.  The medical apparatus as claimed in claim 1, wherein said compensation circuit comprises:

    a coil that generates a voltage due to a changing magnetic resonance imaging electromagnetic fields.

10. The medical apparatus as claimed in claim 9, wherein said coil is curved in three different spatial directions.

11. The medical apparatus as claimed in claim 9, wherein said compensation circuit further comprises:

    orientation means for changing a spatial orientation of said coil to modify the strength of the voltage induced by the changing magnetic resonance imaging electromagnetic field.

12. The medical apparatus as claimed in claim 1, wherein said compensation circuit comprises:

    a plurality of coils, each coil generating a voltage due to a changing magnetic resonance imaging electromagnetic field such that a combination of voltages due to a changing magnetic resonance imaging electromagnetic field are produced.

13. The medical apparatus as claimed in claim 12, wherein said coils are curved in three spatial directions.

14. The medical apparatus as claimed in claim 12, wherein said compensation circuit further comprises:

    orientation means for changing a spatial orientation of said coils to modify the strength of the voltage due to the changing magnetic resonance imaging electromagnetic field.

15. The medical apparatus as claimed in claim 12, wherein said compensation circuit comprises:

    three orthogonally planar coils, each coil generating a voltage due to a changing magnetic resonance imaging electromagnetic field such that a combination of voltages due to a changing magnetic resonance imaging electromagnetic field are produced.

**FIG. 1**

FIG. 2
PRIOR ART

EP 2 067 501 A2

FIG. 3
PRIOR ART

EP 2 067 501 A2

FIG. 4

EP 2 067 501 A2

FIG. 5

EP 2 067 501 A2

FIG. 6

EP 2 067 501 A2

FIG. 7

FIG. 8

EP 2 067 501 A2

FIG. 9

EP 2 067 501 A2

FIG. 10

*FIG. 11*

EP 2 067 501 A2

FIG. 12

FIG. 13

FIG. 14

FIG. 15

FIG. 16

FIG. 17

**FIG. 18**

EP 2 067 501 A2

FIG. 19

FIG. 20

EP 2 067 501 A2

FIG. 21

EP 2 067 501 A2

FIG. 22

**FIG. 23**

**FIG. 24**

EP 2 067 501 A2

*FIG. 25*

FIG. 26

EP 2 067 501 A2

*FIG. 27*

FIG. 28

EP 2 067 501 A2

FIGURE 29

EP 2 067 501 A2

FIGURE 30

FIGURE 31

## Closed Wound Leads

Legend:
- Closed #1
- Closed #2
- C. #1 W.S.W.

FIGURE 32

FIGURE 33

**FIGURE 34**

**FIGURE 35**

Figure 36

Figure 37

FIGURE 38

30000

31000

32000

40000

41000

42000

EP 2 067 501 A2

FIGURE 39

30000

31000

32000

50000

41000

42000

40000

EP 2 067 501 A2

FIGURE 40

EP 2 067 501 A2

# FIGURE 41

EP 2 067 501 A2

9100

9116    9118

9110

9120

9122

9114

9112

FIGURE 42

9200

9210

9212

9214

9216

9218

9220

FIGURE 43

EP 2 067 501 A2

FIGURE 44

9400

9410 9412 9432

9430 9414

9416

9420

9422

FIGURE 45

EP 2 067 501 A2

FIGURE 46

FIGURE 47

FIGURE 48

EP 2 067 501 A2

R    C

Distal Tip ———[ —/\/\/— ——|| — ]——— To Proximal End of Lead

L

FIGURE 49

Lead Conductive Path

——| RLC Circuit #1 |—| RLC Circuit #2 |————

FIGURE 50

d1

—| RLC Circuit #1 |—| RLC Circuit #2 |— d2 —| RLC Circuit #3 |———— d3 ————| RLC Circuit #4 |—— d4 —| RLC Circuit #5 |————

d0

FIGURE 51

Distal Tip

Proximal End of Lead

Diode

## FIGURE 52

R    C

Distal Tip

To Proximal End of Lead

L

Diodes

## FIGURE 53

Diodes

R    C

Distal Tip

To Proximal End of Lead

L

## FIGURE 54

Lead

Distal End

Coiling Adjusted
For High Impedance
At Frequency #1

Coiling Adjusted
For High Impedance
At Frequency #2

Proximal End

FIGURE 55

FIGURE 56

Figure 57

Figure 58

Figure 59

600190

600180

600172

600184

TRANSCEIVER

POWER
SOURCE

PLU

600174

600182

PULSE
GENER.

OTHER
ELECTRONICS

2nd POWER
SOURCE
MODULE

600176

600178

600186

Figure 60

610200

610204

610172

GRADIENT
DETECTION

610184

610202

POWER
SOURCE

PLU

BY-PASS
SWITCH

610174

610182

PULSE
GENER.

OTHER
ELECTRONICS

610176

610178

Figure 61

Figure 62

Figure 63

Figure 64

Figure 65

Figure 66

Figure 67

Figure 68

690450

690452

690460

690456

690454

690458

690468

690464

690466

690462

690470

Figure 69

700480

700486

700482

700484

700488

700494

700492

700490

Figure 70

710500

710508

710502 710512 710504 710510 710514 710506

Figure 71

720520

720522 720524 720528 720528

Figure 72

730600

730625    C₃    N₁    730650

PROBE
OUTPUT
(90 OHMS)

TU

TG    C₂    N₂    Rₚ    L

C₁

Figure 73

740250    740000    740500

C₂

C₁    Cₛ    L

Cₜ

ℓ

Figure 74

Figure 75

Figure 76

Figure 77

Figure 78

Figure 79

Figure 80

Figure 81

Figure 82

Figure 83

Figure 84

842000

842060 842200 842040 842020

842180

842080

842220

842100

$V_3$

$V_4$

853000

853300

853060 853200 853040 853020

853180

853080

853220

853100

$V_5$

Figure 85

$V_6$

864000

864060

864040    864300 864020

864200

864180

864080

864120

864100

Figure 86

875000

875040 875220 875110    87530   875020

875060

87200

875180

875080

875120    875240

875100

Figure 87

880200

880100    880200    880400    880600

Figure 88

890200

890100    890200    890400    890600

Figure 89

900100

900106    900104

900104

900102

Figure 90

900100

910210 910206

910200

910204

910212 910208 910202

Figure 91

920300

920312 Figure 92 920310

930300

930312 930310 Figure 93

940300 940334 940336
940314

940336 940330 940332 940310

Figure 94

950300 950344 950318
950340
950342
950346
950310 950320

Figure 95

960400

960406          960408
                    960410

960402       960404

Figure 96

970500

970504

970508

970502

970506

Figure 97

Figure 98

Figure 99

Figure 100

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5968083 A

- US 6188926 B